(19) Europäisches Patentamt
European Patent Office
Office européen des brevets

(11) EP 1 118 858 A2

(12) # EUROPEAN PATENT APPLICATION

(43) Date of publication:
**25.07.2001 Bulletin 2001/30**

(21) Application number: **01300020.3**

(22) Date of filing: **03.01.2001**

(51) Int Cl.$^7$: **G01N 33/50**, G01N 33/566, G01N 33/68, G01N 33/58, A61P 31/18

(84) Designated Contracting States:
**AT BE CH CY DE DK ES FI FR GB GR IE IT LI LU MC NL PT SE TR**
Designated Extension States:
**AL LT LV MK RO SI**

(30) Priority: **12.01.2000 GB 0000659**
**12.01.2000 GB 0000663**
**12.01.2000 GB 0000661**

(71) Applicants:
- **Pfizer Limited**
**Sandwich Kent CT13 9NJ (GB)**
Designated Contracting States:
**AT BE CH CY DE DK ES FI FR GB GR IE IT LI LU MC NL PT SE TR**
- **PFIZER INC.**
**New York, N.Y. 10017 (US)**
Designated Contracting States:
**BE CH DE DK ES FI FR GR IE IT LI LU MC NL PT SE TR CY**

(72) Inventors:
- **Dobbs, Susan**
**Sandwich, Kent CT13 9NJ (GB)**
- **Perros, Manoussos**
**Sandwich, Kent CT13 9NJ (GB)**
- **Rickett, Graham Anthony**
**Sandwich, Kent CT13 9NJ (GB)**

(74) Representative: **Hayles, James Richard**
**Pfizer Limited,**
**Patents Department,**
**Ramsgate Road**
**Sandwich Kent CT13 9NJ (GB)**

(54) **Assay method**

(57) An assay method for determining whether an agent is capable of modulating the interaction of CCR5 with gp120 is disclosed. The method comprises incubating the agent with CCR5 and gp120 to form a first reaction mixture; and determining whether said agent modulates the interaction of CCR5 with gp120; wherein said gp120 is associated with CD4. In particular, the interaction is a low affinity binding.

EP 1 118 858 A2

## Description

### FIELD OF INVENTION

The present invention relates to a method. In particular, the present invention relates to an assay method.

More in particular, the present invention relates to an assay method for identifying whether a candidate agent can modulate the binding of gp120 to CCR5.

### BACKGROUND ART

It is desirable to identify agents that can modulate the interaction of CCR5 and gp120. By way of background information, gp120 is the envelope protein of HIV that is required for viral entry into the target cells and CCR5 is the cellular receptor to the β-chemokines RANTES, Mip-1-α and Mip-1-β. CCR5 has been identified as being an important receptor in HIV infection.

WO-A-98/00535 discloses a method for detecting gp120 binding on the surface of cells bearing a co-receptor. The method uses directly labelled protein. This method requires high affinity interactions (low nanomolar Kd). However, the affinity of gp120 proteins from different HIV strains varies. Hence, it is believed that this method may not be capable or even suitable to identify agents that can modulate the interaction of biologically relevant molecules.

WO-A-96/418884 discloses a method for screening for agents that may inhibit HIV-1 infection. That method utilises an antibody that, in itself, acts as an agent that would perturb CCR5 and gp120 binding. In addition the protein used in the specific examples cannot bind CCR5. Hence, the specific target receptors are not CCR5.

WO-A-97/37005 discloses an assay method for detecting and preventing HIV infection. The assay described detects fusion of cells expressing gp160 with cells expressing CD4 and CCR5. It is believed that this assay has inherent problems as containment and reagent provision issues would restrict the use of the method for high-throughput screening, particularly on a large scale.

US-A-5928881 discloses an assay that enables identification of CCR5 inhibitors. The assay requires directly labelled chemokine RANTES. We believe that this assay would not always be predictive of binding of gp120.

The present invention seeks to provide an effective assay method, readily adaptable to high throughput screening format, for identifying agents that would modulate medium to low affinity interactions of CCR5 with gp120.

### SUMMARY ASPECTS OF THE PRESENT INVENTION

The present invention is based on the finding that *in vivo* the interaction of CCR5 with gp120 may be fairly weak. In this regard, our work with recombinant proteins suggests that the dissociation constant (Kd) of the interaction can be in the $10^{-7}$M to $10^{-6}$M range. This is contrast to the $10^{-9}$M range described by others in model systems and in, for example, the patent applications cited above (e.g. WO-A-98/00535 and references within).

The assay method of the present invention utilises this finding. It enables detection of such weak interaction.

For some applications, the assay method of the present invention may be described as being a competitive binding study between a candidate agent and the gp120/CCR5 combination.

### DETAILED ASPECTS OF THE PRESENT INVENTION

In one aspect, the present invention relates to an assay method for determining whether an agent is capable of modulating the interaction of CCR5 with gp120; the method comprising: incubating the agent with CCR5 and gp120 to form a first reaction mixture; and determining whether said agent modulates the interaction of CCR5 with gp120; wherein said gp120 is associated with CD4; and wherein said interaction is a low affinity binding.

In another aspect, the present invention relates to an agent identified by the method according to the present invention, wherein said agent is capable of modulating the interaction of CCR5 with gp120.

In a further aspect, the present invention relates to a process comprising the steps of: (a) performing the assay according to the present invention; (b) identifying one or more agents that are capable of modulating the interaction of CCR5 with gp120; and (c) preparing a quantity of those one or more identified agents.

In a further aspect, the present invention relates to a method of affecting the *in vivo* interaction of CCR5 with gp120 with an agent; wherein the agent is capable of modulating the interaction of CCR5 with gp120 in an *in vitro* assay method; wherein the *in vitro* assay method is the assay method according to the present invention.

In a further aspect, the present invention relates to the use of an agent in the preparation of a pharmaceutical composition for the treatment of a disease or condition associated with the interaction of CCR5 with gp120, wherein the agent is the agent according to the present invention and/or wherein the agent is capable of modulating the interaction of CCR5 with gp120 when assayed *in vitro* by the assay method according to the present invention.

In a further aspect, the present invention relates to a method of treating a subject with an agent, wherein the agent is the agent of the present invention and/or wherein the agent is capable of modulating the interaction of CCR5 with gp120 when assayed *in vitro* by the assay method according to the present invention.

For ease of reference, these and further aspects of the present invention are now discussed under appropriate section headings. However, the teachings under each section are not necessarily limited to each particular section.

## PREFERABLE ASPECTS

Preferably the method includes the step of adding a ligand to said first reaction mixture to form a second reaction mixture; wherein said ligand is capable of indicating whether said agent has modulated said interaction.

Preferably said ligand has a detectable label.

Preferably said detectable label is a atom or a group capable of emitting fluorescent light.

Preferably said fluorescent atom is $Eu^{3+}$.

Preferably detection consists of enhancing natural fluorescence of the $Eu^{3+}$ atom by addition of an enhancer solution known to the art.

Preferably said ligand comprises at least a first antibody.

Preferably said first antibody is capable of binding to gp120; and wherein said binding is high affinity binding.

Preferably said first antibody is associated with a detectable label.

Preferably said ligand may comprise a second antibody.

Preferably said second antibody is capable of binding to said first antibody.

Preferably said second antibody is an anti-IgG antibody.

Preferably said detectable label is associated with said second antibody.

Preferably, the agent is capable of adversely modulating the weak binding interaction of CCR5 and gp120.

Examples of adverse modulation include the separation of, the prevention of the binding of, the cleavage of any one or more of, CCR5 and gp120 - and/or changing their folding configuration so that one or more of gp120 and CCR5 is inoperative.

Further examples of adverse modulation include the separation of, the prevention of the binding of, the cleavage of any one or more of, gp120 and CD4 - and/or changing their folding configuration so that one or more of gp120 and CD4 is inoperative.

## ADVANTAGES

The present invention has a number of advantages. These advantages will be apparent in the following description.

By way of example, the present invention is advantageous since it provides a commercially useful assay to identify suitable agents that could be used *in vivo* to treat conditions associated with CCR5/gp120 binding.

By way of further example, the present invention is advantageous since the method detects interaction of gp120 with cells that express CCR5 alone.

By way of example, the present invention is advantageous since the method is amenable to high throughput screening (HTS).

## AMINO ACID SEQUENCE

As used herein, the term "amino acid sequence" is synonymous with the term "polypeptide" and/or the term "protein". In some instances, the term "amino acid sequence" is synonymous with the term "peptide". In some instances, the term "amino acid sequence" is synonymous with the term "protein".

The amino acid sequence may be prepared isolated from a suitable source, or it may be made synthetically or it may be prepared by use of recombinant DNA techniques.

In one aspect, the present invention provides an amino acid sequence that is capable of acting as a target in an assay for the identification of one or more agents and/or derivatives thereof capable of affecting gp120/CCR5 binding.

## NUCLEOTIDE SEQUENCE

As used herein, the term "nucleotide sequence" is synonymous with the term "polynucleotide".

The nucleotide sequence may be DNA or RNA of genomic or synthetic or of recombinant origin. The nucleotide sequence may be double-stranded or single-stranded whether representing the sense or antisense strand or combinations thereof.

For some applications, preferably, the nucleotide sequence is DNA.

For some applications, preferably, the nucleotide sequence is prepared by use of recombinant DNA techniques (e.g. recombinant DNA).

For some applications, preferably, the nucleotide sequence is cDNA.

For some applications, preferably, the nucleotide sequence may be the same as the naturally occurring form for this aspect.

In one aspect, the present invention provides a nucleotide sequence encoding a substance capable of acting as a target in an assay (such as a yeast two hybrid assay) for the identification of one or more agents and/or derivatives thereof capable of affecting the substance in order to modulate CCR5/gp120 binding.

VARIANTS/HOMOLOGUES/DERIVATIVES

In addition to the specific amino acid sequences and nucleotide sequences mentioned herein, the present invention also encompasses the use of variants, homologue and derivatives thereof. Here, the term "homology" can be equated with "identity".

In the present context, an homologous sequence is taken to include an amino acid sequence which may be at least 75, 85 or 90% identical, preferably at least 95 or 98% identical. In particular, homology should typically be considered with respect to those regions of the sequence (such as amino acids at positions 51, 56 and 57) known to be essential for an activity. Although homology can also be considered in terms of similarity (i.e. amino acid residues having similar chemical properties/functions), in the context of the present invention it is preferred to express homology in terms of sequence identity.

Homology comparisons can be conducted by eye, or more usually, with the aid of readily available sequence comparison programs. These commercially available computer programs can calculate % homology between two or more sequences.

% homology may be calculated over contiguous sequences, i.e. one sequence is aligned with the other sequence and each amino acid in one sequence is directly compared with the corresponding amino acid in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion will cause the following amino acid residues to be put out of alignment, thus potentially resulting in a large reduction in % homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids, a sequence alignment with as few gaps as possible - reflecting higher relatedness between the two compared sequences - will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example when using the GCG Wisconsin Bestfit package (see below) the default gap penalty for amino acid sequences is -12 for a gap and -4 for each extension.

Calculation of maximum % homology therefore firstly requires the production of an optimal alignment, taking into consideration gap penalties. A suitable computer program for carrying out such an alignment is the GCG Wisconsin Bestfit package (University of Wisconsin, U.S.A.; Devereux et al., 1984, Nucleic Acids Research 12:387). Examples of other software than can perform sequence comparisons include, but are not limited to, the BLAST package (see Ausubel *et al*., 1999 ibid - Chapter 18), FASTA (Atschul *et al*., 1990, J. Mol. Biol., 403-410) and the GENEWORKS suite of comparison tools. Both BLAST and FASTA are available for offline and online searching (see Ausubel *et al*., 1999 ibid, pages 7-58 to 7-60). However, for some applications, it is preferred to use the GCG Bestfit program. A new tool, called BLAST 2 Sequences is also available for comparing protein and nucleotide sequence (see FEMS Microbiol Lett 1999 174(2): 247-50; FEMS Microbiol Lett 1999 177(1): 187-8 and tatiana@ncbi.nlm.nih.gov).

Although the final % homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. GCG Wisconsin programs generally use either the public default values or a custom symbol comparison table if supplied (see user manual for further details). It is preferred to use the public default values for the GCG package, or in the case of other software,

the default matrix, such as BLOSUM62.

Once the software has produced an optimal alignment, it is possible to calculate % homology, preferably % sequence identity. The software typically does this as part of the sequence comparison and generates a numerical result.

The sequences may also have deletions, insertions or substitutions of amino acid residues which produce a silent change and result in a functionally equivalent substance. Deliberate amino acid substitutions may be made on the basis of similarity in polarity, charge, solubility, hydrophobicity, hydrophilicity, and/or the amphipathic nature of the residues as long as the secondary binding activity of the substance is retained. For example, negatively charged amino acids include aspartic acid and glutamic acid; positively charged amino acids include lysine and arginine; and amino acids with uncharged polar head groups having similar hydrophilicity values include leucine, isoleucine, valine, glycine, alanine, asparagine, glutamine, serine, threonine, phenylalanine, and tyrosine.

Conservative substitutions may be made, for example according to the Table below. Amino acids in the same block in the second column and preferably in the same line in the third column may be substituted for each other:

| | | |
|---|---|---|
| ALIPHATIC | Non-polar | G A P |
| | | I L V |
| | Polar - uncharged | C S T M |
| | | N Q |
| | Polar - charged | D E |
| | | K R |
| AROMATIC | | H F W Y |

The present invention also encompasses homologous substitution (substitution and replacement are both used herein to mean the interchange of an existing amino acid residue, with an alternative residue) may occur i.e. like-for-like substitution such as basic for basic, acidic for acidic, polar for polar etc. Non-homologous substitution may also occur i.e. from one class of residue to another or alternatively involving the inclusion of unnatural amino acids such as ornithine (hereinafter referred to as Z), diaminobutyric acid ornithine (hereinafter referred to as B), norleucine ornithine (hereinafter referred to as O), pyriylalanine, thienylalanine, naphthylalanine and phenylglycine.

Replacements may also be made by unnatural amino acids include; alpha* and alpha-disubstituted* amino acids, N-alkyl amino acids*, lactic acid*, halide derivatives of natural amino acids such as trifluorotyrosine*, p-Cl-phenylalanine*, p-Br-phenylalanine*, p-I-phenylalanine*, L-allyl-glycine*, ß-alanine*, L-α-amino butyric acid*, L-γ-amino butyric acid*, L-α-amino isobutyric acid*, L-ε-amino caproic acid#, 7-amino heptanoic acid*, L-methionine sulfone#*, L-norleucine*, L-norvaline*, p-nitro-L-phenylalanine*, L-hydroxyproline#, L-thioproline*, methyl derivatives of phenylalanine (Phe) such as 4-methyl-Phe*, pentamethyl-Phe*, L-Phe (4-amino)#, L-Tyr (methyl)*, L-Phe (4-isopropyl)*, L-Tic (1,2,3,4-tetrahydroisoquinoline-3-carboxyl acid)*, L-diaminopropionic acid# and L-Phe (4-benzyl)*. The notation * has been utilised for the purpose of the discussion above (relating to homologous or non-homologous substitution), to indicate the hydrophobic nature of the derivative whereas # has been utilised to indicate the hydrophilic nature of the derivative, #* indicates amphipathic characteristics.

Variant amino acid sequences may include suitable spacer groups that may be inserted between any two amino acid residues of the sequence including alkyl groups such as methyl, ethyl or propyl groups in addition to amino acid spacers such as glycine or β-alanine residues. A further form of variation, involves the presence of one or more amino acid residues in peptoid form, will be well understood by those skilled in the art. For the avoidance of doubt, "the peptoid form" is used to refer to variant amino acid residues wherein the α-carbon substituent group is on the residue's nitrogen atom rather than the α-carbon. Processes for preparing peptides in the peptoid form are known in the art, for example Simon RJ et al., PNAS (1992) 89(20), 9367-9371 and Horwell DC, Trends Biotechnol. (1995) 13 (4), 132-134.

The nucleotide sequences for use in the present invention may include within them synthetic or modified nucleotides. A number of different types of modification to oligonucleotides are known in the art. These include methylphosphonate and phosphorothioate backbones and/or the addition of acridine or polylysine chains at the 3' and/or 5' ends of the molecule. For the purposes of the present invention, it is to be understood that the nucleotide sequences described herein may be modified by any method available in the art. Such modifications may be carried out in to enhance the *in vivo* activity or life span of nucleotide sequences of the present invention.

The present invention also encompasses the use of nucleotide sequences that are complementary to the sequences presented herein, or any derivative, fragment or derivative thereof. If the sequence is complementary to a fragment thereof then that sequence can be used a probe to identify similar coding sequences in other organisms etc.

EXPRESSION VECTORS

The nucleotide sequence for use as the target or for expressing the target can be incorporated into a recombinant replicable vector. The vector may be used to replicate and express the nucleotide sequence in and/or from a compatible host cell. Expression may be controlled using control sequences which include promoters/enhancers and other expression regulation signals. Prokaryotic promoters and promoters functional in eukaryotic cells may be used. Tissue specific or stimuli specific promoters may be used. Chimeric promoters may also be used comprising sequence elements from two or more different promoters described above.

The protein produced by a host recombinant cell by expression of the nucleotide sequence may be secreted or may be contained intracellularly depending on the sequence and/or the vector used. The coding sequences can be designed with signal sequences which direct secretion of the substance coding sequences through a particular prokaryotic or eukaryotic cell membrane.

FUSION PROTEINS

The target amino acid sequence may be produced as a fusion protein, for example to aid in extraction and purification. Examples of fusion protein partners include glutathione-S-transferase (GST), 6xHis, GAL4 (DNA binding and/or transcriptional activation domains) and (-galactosidase. It may also be convenient to include a proteolytic cleavage site between the fusion protein partner and the protein sequence of interest to allow removal of fusion protein sequences. Preferably the fusion protein will not hinder the activity of the target.

The fusion protein may comprise an antigen or an antigenic determinant fused to the substance of the present invention. In this embodiment, the fusion protein may be a non-naturally occurring fusion protein comprising a substance which may act as an adjuvant in the sense of providing a generalised stimulation of the immune system. The antigen or antigenic determinant may be attached to either the amino or carboxy terminus of the substance.

In another embodiment of the invention, the amino acid sequence may be ligated to a heterologous sequence to encode a fusion protein. For example, for screening of peptide libraries for agents capable of affecting the substance activity, it may be useful to encode a chimeric substance expressing a heterologous epitope that is recognized by a commercially available antibody.

CCR5

An essential component of the assay is CCR5.

CCR5 is a chemokine receptor that typically is present on the surfaces of certain cells within the human body.

The term "chemokine", is a contraction of "chemotactic cytokines". The chemokines comprise a large family of proteins which have in common important structural features and which have the ability to attract leukocytes. As leukocyte chemotactic factors, chemokines play an indispensable role in the attraction of leukocytes to various tissues of the body, a process which is essential for both inflammation and the body's response to infection. Because chemokines and their receptors are central to the pathophysiology of inflammatory and infectious diseases, agents which are active in modulating, preferably antagonizing, the activity of chemokines and their receptors, are useful in the therapeutic treatment of such inflammatory and infectious diseases.

The chemokine receptor CCR5 is of particular importance in the context of treating inflammatory and infectious diseases. CCR5 is a receptor for chemokines, especially for the macrophage inflammatory proteins (MIP) designated MIP-1$\alpha$ and MIP-1$\beta$, and for a protein which is regulated upon activation and is normal T-cell expressed and secreted (RANTES).

Background teachings on CCR5 may be found in WO-A-97/32019.

The nucleotide sequence encoding same and the amino acid sequence for same are presented in the attached sequence listings.

Background teachings on CCR5 have also been presented by Victor A. McKusick *et al* on http://www.ncbi.nlm.nih.gov/Omim. The following information concerning CCR5 has been extracted from that source.

"Samson et al. (1996) cloned a human C-C chemokine receptor gene from a human genomic DNA library based on its similarity to a murine C-C chemokine receptor clone (MOP020). The human gene, which they designated ChemR13, encodes a 352-amino acid protein (designated CCCKR5 by them) with a calculated molecular mass of 40,600 Da and a potential N-linked glycosylation site. With a set of overlapping lambda clones, they showed that the gene is 17.5 kb from the CMKBR2 gene. The 2 coding regions share 75% DNA and amino acid sequence identity.

Samson et al. (1996) functionally expressed the gene in a stably transfected CHO-K1 cell line. In transfected cells, macrophage inflammatory protein (MIP)-1-$\alpha$ appeared to be the most potent agonist for CCCKR5, with MIP-1-$\beta$ and RANTES also active at physiologic concentrations. Samson et al. (1996) detected transcript from the gene in a promyeloblastic cell line, which suggested a potential role for the chemokine receptor in granulocyte lineage proliferation and differentiation.

By radiation hybrid mapping, Liu et al. (1996) localized the CCR5 gene (designated CKR5 by them) to chromosome 3p21.

The C-C chemokine receptor CMKBR5 was identified as a coreceptor for the human immunodeficiency virus-1 (HIV-1) by Deng et al. (1996) and Dragic et al. (1996). CMKBR5 and fusin (162643) facilitate the fusion of HIV-1 with the plasma membrane of CD4(+) cells (CD4; 186940). Deng et al. (1996) found that CMKBR5, and not fusin, promotes entry of the macrophage-tropic viruses believed to be the key pathogenic strains in vivo. Dragic et al. (1996) showed that MIP-1-$\alpha$, MIP-1-$\beta$, and RANTES each inhibit infection of CD4(+) cells by primary, nonsyncytium-inducing (NSI) HIV-1 strains at the virus entry stage and also block env-mediated cell-cell fusion. Both groups showed that expression of the CCCKR5 protein renders nonpermissive CD4(+) cells susceptible to infection by HIV-1 strains. Alkhatib et al. (1996) reported similar observations and detected mRNA for the receptor only in cell types susceptible to macrophage-tropic isolates of HIV-1. See also Choe et al. (1996), who implicated both CCR5 and CCR3 in the ability of HIV-1 to infect cells expressing those receptors.

Some individuals remain uninfected by HIV-1 despite repeated exposure to the virus. Both Liu et al. (1996) and Samson et al. (1996) identified a molecular basis for such HIV-1 resistance. Samson et al. (1996) postulated that variants of the CMKBR5 gene may be responsible for relative or absolute resistance to HIV-1 infection. In an HIV-1-infected patient with slow disease progression, Samson et al. (1996) identified a heterozygous 32-bp deletion in the CMKBR5 gene that results in a frameshift and premature termination of translation of the transcript. Liu et al. (1996) identified the same homozygous 32-bp deletion with CMKBR5 in 2 individuals who, though multiply exposed to HIV-1 infection, remained uninfected. Liu et al. (1996) found that the deletion comprises nucleotides 794 to 825 of the cDNA sequence and results in a reading frameshift after amino acid 174, inclusion of 7 novel amino acids, and truncation at codon 182. They showed that the severely truncated protein could not be detected at the surface of cells that normally express the protein. Samson et al. (1996) stated that the mutant protein lacks the last 3 of 7 putative transmembrane regions of the receptor as well as regions involved in G protein coupling and signal transduction. Through in vitro fusion assays, both Liu et al. (1996) and Samson et al. (1996) determined that the truncated receptor did not allow fusion of CD4(+) cells with cells expressing env protein from either macrophage-tropic or dual-tropic viruses. Samson et al. (1996) found that coexpression of the deletion mutant with wildtype CCR5 reduced the fusion efficiency of 2 different viral envelope proteins in 3 independent experiments.

Dean et al. (1996) reported results of their CKR5 studies in 1,955 individuals included in 6 well-characterized AIDS cohort studies. They identified 17 individuals who were homozygous for the CKR5 32-bp deletion allele (601373.0001). Deletion homozygotes occurred exclusively among the 612 members of the HIV-1-exposed, antibody-negative group and not at all in 1,343 HIV-1 infected individuals. The frequency of the CKR5 deletion heterozygotes was significantly elevated in groups of individuals who had survived HIV-1 infection for more than 10 years. In some risk groups the frequency of CKR5 deletion heterozygotes was twice as frequent as in groups with rapid progressors to AIDS. Survival analysis clearly showed that the disease progression was slower in CKR5 deletion heterozygotes than in individuals homozygous for the normal CKR5 allele. Dean et al. (1996) postulated that the CKR5 32-bp deletion may act as 'a recessive restriction gene against HIV-1 infection' and may exert a dominant phenotype of delayed progression to AIDS among infected individuals. Dean et al. (1996) reported that in addition to the CKR5 32-bp deletion allele, they found unique single-strand conformation polymorphisms (SSCPs) in other patients, some of whom were long-term nonprogressors. They speculated that at least some of these alleles disrupt CKR5 function and inhibit the spread of HIV-1 or the progression to AIDS. Dean et al. (1996) recommended that the entire coding region of CKR5 be screened in nonprogressors and in rapid progressors to identify other CKR5 variants.

To determine the role of the 32-bp deletion in CKR5 in HIV-1 transmission and disease progression, Huang et al. (1996) analyzed the CKR5 genotype of 1,252 homosexual men enrolled in the Chicago component of the Multicenter AIDS Cohort Study. No infected participant was found to be homozygous for the 32-bp deletion allele, whereas 3.6% of at-risk but uninfected Caucasian participants were homozygous, showing the highly protective role of this genotype against sexual acquisition of HIV-1. No evidence was found that suggested heterozygotes were protected against HIV-1 infection, but a limited protective role against disease progression was noted.

Zimmerman et al. (1997) reported results of a large study that analyzed the frequency of the 32-bp deletion allele of CMKBR5 in populations from North America, Asia, and Africa. Ansari-Lari et al. (1997) also published data on the population frequencies of various mutations in CCR5. The study indicated that the mutations are relatively specific to different ethnicities; apart from the 32-bp deletion allele in the American Caucasian population, and 2 alleles in Chinese and Japanese populations, the CCR5 locus did not show a high degree of genetic variation. The authors stated that, while additional population screening at this locus might identify other sequence variants, their frequencies are likely to be less than 0.01. The frequency of the 32-bp deletion allele in American Caucasians was approximately 0.16, a value somewhat higher than that previously reported for this group.

Smith et al. (1997) analyzed 2-locus genotypes and found that the 32-bp deletion at the CCR5 locus and the 64I allele at the CCR2 locus are in strong, perhaps complete, linkage disequilibrium with each other. This means that

CCR5-del32 invariably occurs on a chromosome with allele CCR2-64V, whereas CCR2-64I occurs on a chromosome that has the wildtype (undeleted) allele at the CCR5 locus. Thus, they could estimate the independent effects of the CCR2 and CCR5 polymorphisms. An estimated 38 to 45% of AIDS patients who had rapid progression of less than 3 years from HIV-1 exposure to onset of AIDS symptoms could be attributed to their wildtype status at one or the other of these loci, whereas the survival of 28 to 29% of long-term survivors, who avoided AIDS for 16 years or more, could be explained by a mutant genotype for CCR2 or CCR5.

Biti et al. (1997) reported an HIV-infected, asymptomatic individual of European descent who was found to be homozygous for the 32-bp deletion. The presence of homozygosity was supported by genotyping his sole surviving parent (a heterozygote) and his sibs (a CCR5-del32 homozygous brother, a heterozygous brother, and a CCR5 wildtype homozygous sister). The patient presented in 1992 with a seroconversion-like illness of 1-month duration, at which time he was diagnosed HIV-1 seropositive by Western blot. At the time of their report, his CD4+ T-cell count was 460, and a plasma RNA viral load test showed 19,000 copies per milliliter. Biti et al. (1997) noted that the tropism of the infecting HIV-1 strain was still under investigation.

Using a panel of monoclonal antibodies specific for human CCR5, Rottman et al. (1997) showed by immunohistochemistry and flow cytometry that CCR5 is expressed by bone marrow-derived cells known to be targets for HIV-1 infection, including a subpopulation of lymphocytes and monocytes/macrophages in blood, primary and secondary lymphoid organs, and noninflamed tissues. In the central nervous system, CCR5 was expressed on neurons, astrocytes, and microglia. In other tissues, CCR5 was expressed on epithelium, endothelium, vascular smooth muscle, and fibroblasts. Chronically inflamed tissues contained an increased number of CCR5-positive mononuclear cells, and the number of immunoreactive cells was directly associated with a histopathologic correlate of inflammatory severity. The results suggested that CCR5-positive cells are recruited to inflammatory sites and, as such, may facilitate transmission of macrophagetropic strains of HIV-1.

Cohen et al. (1997) studied a cohort of 33 HIV-1 nonprogressors and compared 21 patients who were homozygous wildtype at the CCR5 locus with 12 who were heterozygous for the 32-bp deletion mutation in CCR5. There were no differences in CD4+ or CD8+ T-cell counts, or in plasma or lymph node viral loads. The authors concluded that CCR5 is not the sole determinant of long-term nonprogression in some HIV-1 infected individuals. Although no differences were detected at an average of 11 years postinfection, the authors suggest that CCR5 may still play a role in nonprogression by limiting viral replication during acute infection.

Although CD4 was identified initially as the cellular receptor for HIV, several lines of evidence indicated that expression of CD4 alone was insufficient to confer susceptibility to infection by the virus. Specifically, HIV did not infect mouse cells transfected with a human CD4 expression vector or mice transgenic for the expression of human CD4. Furthermore, although HIV binding and internalization can be mediated by CD4 acting together with one of several members of the chemokine receptor superfamily, CCR5 appears to be the critical coreceptor used by HIV in the initial stages of infection. However, because mouse CCR5 differs significantly from human CCR5, it cannot function as a coreceptor for HIV, and thus, expression of human CD4 alone is insufficient to permit entry of HIV into mouse cells. Browning et al. (1997) found that mice transgenic for both CD4 and CCR5 are susceptible to HIV infection.

Zagury et al. (1998) found that there were factors other than the CCR5 polymorphism accounting for the fact that exposure to HIV-1 does not usually lead to infection. Although this fact could be because of insufficient virus titer, there is abundant evidence that some individuals resist infection even when directly exposed to a high titer of HIV. This protection is related to homozygous mutations in CCR5, the receptor for the β-chemokines, and earlier studies had shown that the same chemokines markedly suppressed the nonsyncytial inducing variants of HIV-1, the chief virus type transmitted from person to person. However, CCR5 mutations are not likely to be the unique mechanism of protection because HIV-1 variants can use other chemokine receptors as their coreceptor and, indeed, infection has been demonstrated within the presence of such mutations. Zagury et al. (1998) found transient natural resistance over time of most of 128 hemophiliacs who were inoculated repeatedly with HIV-1-contaminated factor VIII concentrate from plasma during 1980 to 1985, before the development of the HIV blood test. Furthermore, and remarkably, 14 subjects remained unaffected to the time of the report, and in these subjects homozygous CCR5 mutations were found in none, but in most of them there was overproduction of β-chemokines. In vitro experiments confirmed the potent anti-HIV suppressive effect of these chemokines. The chemokines studied were generically referred to as MMR, an abbreviation for MIP-1-α, MIP-1-β, and RANTES.

Martin et al. (1998) showed by genetic association analysis of 5 cohorts of people with AIDS that infected individuals homozygous for a multisite haplotype of the CCR5 regulatory region containing the promoter allele, CCR5P1, progress to AIDS more rapidly than those with other CCR5 promoter genotypes, particularly in the early years after infection. Composite genetic epidemiologic analyses of the genotypes bearing CCR5P1, CCR5-delta-32, CCR2-64I (see 601267), and SDF1-3-prime A (see 600835) affirmed distinct regulatory influences for each gene on AIDS progression. An estimated 10 to 17% of patients who developed AIDS within 3.5 years of HIV-1 infection did so because they were homozygous for CCR5P1/P1, and 7 to 13% of all people carry this susceptible genotype. The cumulative and interactive influence of these AIDS restriction genes illustrates the multigenic nature of host factors limiting AIDS

disease progression. CCR5 and CCR2 are tightly linked on 3p22-p21, separated by 20 kb. Common allelic variants in both genes are associated with slower progression to AIDS after infection. The protective influences of CCR5-delta-32 and CCR2-64I are independent in AIDS cohorts, and the 2 mutations have never been found on the same chromosome haplotype. The physical proximity of CCR2 and CCR5, the equivalent functional efficiency of alternative CCR2 allelic products as chemokine or HIV-1 coreceptors, and the rarity of HIV-1 strains that use the CCR2 receptor led to the speculation that CCR2-64I may be hitchhiking (or tracking by linkage disequilibrium) with an undiscovered CCR5 variant, perhaps in the cis-regulatory region, that is directly responsible for the CCR2-64I protective effect. Martin et al. (1998) found that among 2,603 individuals enrolled in 5 AIDS cohorts, CCR2-64I was always found on a CCR5P1-bearing haplotype and that CCR5-delta-32 was consistently found on a CCR5P1 haplotype as well. All CCR2-64I/64I homozygotes were always CCR5P1/P1 homozygotes (N = 43). Similarly, all CCR5-delta-32/delta-32 homozygotes were CCR5P1/P1 homozygotes (N = 18). Finally, none of 657 individuals who lacked the CCR5P1 allele had either the CCR5-delta-32 or the CCR2-64I allele. Thus the entire CCR2-CCR5 complex can be considered as a 6-allele genotype system, based on the composite CCR2 and CCR5 haplotype.

In a denaturing high-pressure liquid chromatography (DHPLC) screen of AIDS patients, Martin et al. (1998) detected 4 common allelic variants (CCR5P1-P4); 6 rare alleles (CCR5P5-P10) were discovered as heterozygotes upon subsequent single-strand conformation polymorphism (SSCP) screening of 5 AIDS cohorts. Sequence analysis of the CCR5 promoter region of individuals homozygous for the CCR5P1-P4 variants and heterozygotes of the 6 rare variants revealed 10 polymorphic nucleotide positions that described 10 CCR5 promoter haplotype alleles, referred to as promoter alleles. McDermott et al. (1998) reported a G/T variant, corresponding to position 303 of the promoter region, that showed an epidemiologic association with rapid progression to AIDS.

Farzan et al. (1999) showed that the chemokine receptor CCR5, a principal HIV-1 coreceptor, is posttranslationally modified by O-linked glycosylation and by sulfation of its N-terminal tyrosines. Sulfated tyrosines contributed to the binding of CCR5 to MIP-1-$\alpha$, MIP-1-$\beta$, and HIV-1 gp120/CD4 complexes, and to the ability of HIV-1 to enter cells expressing CCR5 and CD4. Farzan et al. (1999) concluded that tyrosine sulfation may contribute to the natural function of many 7-transmembrane-segment receptors and may be a modification common to primate immunodeficiency virus coreceptors.

Carrington et al. (1999) reviewed the growing number of genetic variants within the coding and 5-prime regulatory region of CCR5 that had been identified, several of which have functional consequences for HIV-1 pathogenesis. The findings provided logic for the development of therapeutic strategies that target the interaction of HIV-1 envelope and CCR5 in HIV-1 associated disease."

gp120

An essential component of the assay is gp120. Information on gp120 has been presented above. Further teachings on gp120 may be found in the art, such as in some of the patent applications cited above.
The nucleotide sequence encoding gp120 and the amino acid sequence for same are presented in the attached sequence listings.

CD4

In the assay of the present invention the gp120 is typically associated with CD4. A suitable CD4 is a commercialy available soluble version of CD4 (baculovirus expressed sCD4). In the preparation of the first reaction mixture, the gp120 and the CD4 may be added sequentially, simultaneously or together.
Background teachings on CD4 are mentioned above.
Background teachings on CD4 may also be found in WO-A-89/03222. The nucleotide sequence encoding same and the amino acid sequence of same is presented in the attached sequence listings.
In addition, further background teachings on CD4 have been presented by Victor A. McKusick *et al* on http://www.ncbi.nim.nih.gov/Omim. The following further information concerning CD4 has been extracted from that source.

"Circulating mature T lymphocytes constitute a heterogeneous cell population with 2 major phenotypes, one expressing the CD4 marker on its surface (generally associated with helper/inducer function), and the other expressing the CD8 antigen (usually associated with cytotoxic/suppressor activity). Amadori et al. (1995) noted that evaluation of the CD4/CD8 ratio is routine in AIDS patients for the assessment of immune function and added that not only HIV infection but also other acute viral diseases, such as infections by cytomegalovirus, Epstein-Barr virus, and influenza virus, are usually associated with an inversion of the CD4/CD8 ratio. A low ratio is also a hallmark of intense, chronic immune responses, such as allograft rejection and graft-versus-host disease. CD4/CD8 ratios of 1.5 to 2.5 are usually considered normal. The occasional finding of a CD4/DC8 ratio less than 1 in otherwise normal, healthy individuals is usually disregarded.

Because of a personal interest on the part of one member of the group (A.A.) who had shown an invariable ratio

less than 1 over many years, and because the ratio in mice was shown by Kraal et al. (1983) to be under genetic control, Amadori et al. (1995) studied the genetic pattern of inheritance of the ratio in a population of 468 healthy blood donors. Distribution of the ratio in males and females was significantly different and was significantly affected by age. In 46 randomly selected families, the parental CD4/CD8 ratios significantly influenced the ratio in offspring. Complex segregation analysis of the data rejected the non-genetic hypothesis; among the genetic models tested, a major recessive gene with a polygenic component and random environmental effects was the most parsimonious model. Overall, Amadori et al. (1995) found that 57% of the variation in the CD4/CD8 ratio could be attributed to genetic factors, as opposed to noninherited (stochastic or environmental) factors. In mice, the CD4/CD8 ratio appears to be under the genetic control of a single dominant gene (Kraal et al., 1983). Chakravarti (1995) pointed to several important implications of the simple observation in this study. First, norms for the ratio must be defined separately for different ages, genders, and perhaps even populations. The possibility of a major gene determining the ratio implies that family history of low ratios may need to be considered for accurate prognosis of HIV or other infection. The identification of genes underlying this phenotype will lead to a better understanding of the mechanisms that commit immature thymocytes to the helper or cytotoxic lineages, and the site in the developmental chain in which they function.

Lastly, identification of the genes will also help answer questions regarding genetic factors that control infection and immunity.

To define the mode of inheritance of the CD4/CD8 ratio, Clementi et al. (1999) examined the absolute number of CD4 and CD8 cells in a large unselected control population and in members of 70 nuclear families. Pedigrees of nuclear families were analyzed by complex segregation analysis. Data were adjusted before this analysis to remove the effects of relevant covariates. The nongenetic transmission and the multifactorial model could be easily rejected for both CD4 and CD8 cells. The best fitting models were a major autosomal recessive gene with a residual multifactorial effect controlling the high number of CD4 and a major autosomal recessive gene with a residual multifactorial effect controlling the high number of CD8 cells. The authors pointed out that the knowledge of the CD4+ cell number and the proportion between CD4+ and CD8+ T cells could be a useful parameter in predicting human immunodeficiency virus infection outcome."

LOW AFFINITY BINDING

As used in relation to the present invention, preferably the term "low affinity binding" means a Kd value of at least about 200 nM. More preferably the term "low affinity binding" means a Kd value of more than about 200 nM.

LIGAND

In a preferred aspect, the assay method of the present invention utilises a ligand. This ligand is capable of indicating whether said agent has modulated said interaction.

The ligand may be any suitable ligand. The ligand may be an organic chemical compound. The ligand may be a protein.

In a preferred aspect, the ligand carries a detectable label. This detectable label may become detectable once modulation has occurred - such as through a change in spectrometric properties of the ligand.

This label may be detectable by, for example, spectrometric means - such as spectrophotometric means. Preferably, however, the detectable label is a fluorescent label. Here, any suitable fluorescent can be used.

In a preferred aspect, the ligand comprises at least one antibody, preferably at least two antibodies. In this respect, the at least one antibody is termed a "first antibody". This first antibody may be able to bind to any of the agent to gp120. In a preferred aspect, the first antibody is able to bind to gp120 with high affinity and specificity, and in a mode that does not disturb the interaction of gp120 with either CD4 or CCR5.

If the ligand comprises at least two antibodies, then the at least one other antibody is called a "second antibody". The second antibody may be able to bind the first antibody. In a preferred aspect, the second antibody is able to bind to the first antibody with high affinity and specificity, and in a way that does not disturb the interaction of the first antibody with its ligand.

Preferably, the ligand comprises the first antibody and the second antibody. In this embodiment, in the preparation of the second reaction mixture the first antibody and the second antibody may be added sequentially, simultaneously or together.

Preferably the second antibody carries the detectable label. If there is no second antibody, the first antibody typically carries the detectable label.

ANTIBODY

As indicated, preferably the ligand for use in the assay method of the present invention comprises one or two

antibodies.

The "antibody" as used herein includes but is not limited to, polyclonal, monoclonal, chimeric, single chain, Fab fragments and fragments produced by a Fab expression library. Such fragments include fragments of whole antibodies which retain their binding activity for a target substance, Fv, F(ab') and F(ab')2 fragments, as well as single chain antibodies (scFv), fusion proteins and other synthetic proteins which comprise the antigen-binding site of the antibody. Furthermore, the antibodies and fragments thereof may be humanised antibodies, for example as described in US-A-239400. Neutralizing antibodies, i.e., those which inhibit biological activity of the substance polypeptides, are especially preferred for diagnostics and therapeutics.

Antibodies may be produced by standard techniques, such as by immunisation with the substance of the invention or by using a phage display library.

If polyclonal antibodies are desired, a selected mammal (e.g., mouse, rabbit, goat, horse, etc.) is immunised with an immunogenic polypeptide bearing a epitope(s) obtainable from an identified agent and/or substance of the present invention. Depending on the host species, various adjuvants may be used to increase immunological response. Such adjuvants include, but are not limited to, Freund's, mineral gels such as aluminium hydroxide, and surface active substances such as lysolecithin, pluronic polyols, polyanions, peptides, oil emulsions, keyhole limpet hemocyanin, and dinitrophenol. BCG (Bacilli Calmette-Guerin) and Corynebacterium parvum are potentially useful human adjuvants which may be employed if purified the substance polypeptide is administered to immunologically compromised individuals for the purpose of stimulating systemic defence.

Serum from the immunised animal is collected and treated according to known procedures. If serum containing polyclonal antibodies to an epitope obtainable from an identifed agent and/or substance of the present invention contains antibodies to other antigens, the polyclonal antibodies can be purified by immunoaffinity chromatography. Techniques for producing and processing polyclonal antisera are known in the art. In order that such antibodies may be made, the invention also provides polypeptides of the invention or fragments thereof haptenised to another polypeptide for use as immunogens in animals or humans.

Monoclonal antibodies directed against particular epitopes can also be readily produced by one skilled in the art. The general methodology for making monoclonal antibodies by hybridomas is well known. Immortal antibody-producing cell lines can be created by cell fusion, and also by other techniques such as direct transformation of B lymphocytes with oncogenic DNA, or transfection with Epstein-Barr virus. Panels of monoclonal antibodies produced against orbit epitopes can be screened for various properties; i.e., for isotype and epitope affinity.

Monoclonal antibodies may be prepared using any technique which provides for the production of antibody molecules by continuous cell lines in culture. These include, but are not limited to, the hybridoma technique originally described by Koehler and Milstein (1975 Nature 256:495-497), the human B-cell hybridoma technique (Kosbor et al (1983) Immunol Today 4:72; Cote et al (1983) Proc Natl Acad Sci 80:2026-2030) and the EBV-hybridoma technique (Cole et al (1985) Monoclonal Antibodies and Cancer Therapy, Alan R Liss Inc, pp 77-96). In addition, techniques developed for the production of "chimeric antibodies", the splicing of mouse antibody genes to human antibody genes to obtain a molecule with appropriate antigen specificity and biological activity can be used (Morrison et al (1984) Proc Natl Acad Sci 81:6851-6855; Neuberger et al (1984) Nature 312:604-608; Takeda et al (1985) Nature 314:452-454). Alternatively, techniques described for the production of single chain antibodies (US Patent No. 4,946,779) can be adapted to produce the substance specific single chain antibodies.

Antibodies may also be produced by inducing in vivo production in the lymphocyte population or by screening recombinant immunoglobulin libraries or panels of highly specific binding reagents as disclosed in Orlandi et al (1989, Proc Natl Acad Sci 86: 3833-3837), and Winter G and Milstein C (1991; Nature 349:293-299).

Antibody fragments which contain specific binding sites for the substance may also be generated. For example, such fragments include, but are not limited to, the F(ab')2 fragments which can be produced by pepsin digestion of the antibody molecule and the Fab fragments which can be generated by reducing the disulfide bridges of the F(ab')2 fragments. Alternatively, Fab expression libraries may be constructed to allow rapid and easy identification of monoclonal Fab fragments with the desired specificity (Huse WD et al (1989) Science 256:1275-128 1).

## ASSAY

Any one or more of appropriate targets - such as an amino acid sequence and/or nucleotide sequence - may be used for identifying an agent capable of modulating the interaction of gp120 with CCR5 in any of a variety of drug screening techniques. The target employed in such a test may be free in solution, affixed to a solid support, borne on a cell surface, or located intracellularly. The abolition of target activity or the formation of binding complexes between the target and the agent being tested may be measured.

The assay of the present invention may be a screen, whereby a number of agents are tested. In one aspect, the assay method of the present invention is a high through put screen.

Techniques for drug screening may be based on the method described in Geysen, European Patent Application 84/03564, published on September 13, 1984. In summary, large numbers of different small peptide test compounds are synthesized on a solid substrate, such as plastic pins or some other surface. The peptide test compounds are reacted with a suitable target or fragment thereof and washed. Bound entities are then detected - such as by appropriately adapting methods well known in the art. A purified target can also be coated directly onto plates for use in a drug screening techniques. Alternatively, non-neutralising antibodies can be used to capture the peptide and immobilise it on a solid support.

This invention also contemplates the use of competitive drug screening assays in which neutralising antibodies capable of binding a target specifically compete with a test compound for binding to a target.

Another technique for screening provides for high throughput screening (HTS) of agents having suitable binding affinity to the substances and is based upon the method described in detail in WO-A-84/03564.

It is expected that the assay methods of the present invention will be suitable for both small and large-scale screening of test compounds as well as in quantitative assays.

In one preferred aspect, the present invention relates to a method of identifying agents that selectively modulate the interaction between gp120 and CCR5.

In a preferred aspect, the assay of the present invention utilises cells that display CCR5 on their surface. These cells may be isolated from a subject possessing such cells. However, preferably, the cells are prepared by transfecting cells so that upon transfect those cells display on their surface CCR5. Teachings for preparing such transfected cells may be found in US-A-5939320.

REPORTERS

A wide variety of reporters may be used in the assay methods (as well as screens) of the present invention with preferred reporters providing conveniently detectable signals (eg. by spectroscopy). By way of example, a reporter gene may encode an enzyme which catalyses a reaction which alters light absorption properties.

Other protocols include enzyme-linked immunosorbent assay (ELISA), radioimmunoassay (RIA) and fluorescent activated cell sorting (FACS). A two-site, monoclonal-based immunoassay utilising monoclonal antibodies reactive to two non-interfering epitopes may even be used. These and other assays are described, among other places, in Hampton R et al (1990, Serological Methods, A Laboratory Manual, APS Press, St Paul MN) and Maddox DE et al (1983, J Exp Med 15 8:121 1).

Examples of reporter molecules include but are not limited to (galactosidase, invertase, green fluorescent protein, luciferase, chloramphenicol, acetyltransferase, (glucuronidase, exo-glucanase and glucoamylase. Alternatively, radiolabelled or fluorescent tag-labelled nucleotides can be incorporated into nascent transcripts which are then identified when bound to oligonucleotide probes.

By way of further examples, a number of companies such as Pharmacia Biotech (Piscataway, NJ), Promega (Madison, WI), and US Biochemical Corp (Cleveland, OH) supply commercial kits and protocols for assay procedures. Suitable reporter molecules or labels include those radionuclides, enzymes, fluorescent, chemiluminescent, or chromogenic agents as well as substrates, cofactors, inhibitors, magnetic particles and the like. Patents teaching the use of such labels include US-A-3817837; US-A-3850752; US-A-3939350; US-A-3996345; US-A-4277437; US-A-4275149 and US-A-4366241.

HOST CELLS

Polynucleotides for use in the present invention - such as for use as targets or for expressing targets - may be introduced into host cells.

The term "host cell" - in relation to the present invention includes any cell that could comprise the target for the agent of the present invention.

Here, polynucleotides may be introduced into prokaryotic cells or eukaryotic cells, for example yeast, insect or mammalian cells.

Polynucleotides of the invention may introduced into suitable host cells using a variety of techniques known in the art, such as transfection, transformation and electroporation. Where polynucleotides of the invention are to be administered to animals, several techniques are known in the art, for example infection with recombinant viral vectors such as retroviruses, herpes simplex viruses and adenoviruses, direct injection of nucleic acids and biolistic transformation.

Thus, a further embodiment of the present invention provides host cells transformed or transfected with a polynucleotide that is or expresses the target of the present invention. Preferably said polynucleotide is carried in a vector for the replication and expression of polynucleotides that are to be the target or are to express the target. The cells will be chosen to be compatible with the said vector and may for example be prokaryotic (for example bacterial), fungal, yeast or plant cells.

The gram negative bacterium *E. coli* is widely used as a host for heterologous gene expression. However, large amounts of heterologous protein tend to accumulate inside the cell. Subsequent purification of the desired protein from the bulk of E.coli intracellular proteins can sometimes be difficult.

In contrast to *E.coli*, bacteria from the genus *Bacillus* are very suitable as heterologous hosts because of their capability to secrete proteins into the culture medium. Other bacteria suitable as hosts are those from the genera Streptomyces and Pseudomonas.

Depending on the nature of the polynucleotide encoding the polypeptide of the present invention, and/or the desirability for further processing of the expressed protein, eukaryotic hosts such as yeasts or other fungi may be preferred. In general, yeast cells are preferred over fungal cells because they are easier to manipulate. However, some proteins are either poorly secreted from the yeast cell, or in some cases are not processed properly (e.g. hyperglycosylation in yeast). In these instances, a different fungal host organism should be selected.

Examples of suitable expression hosts within the scope of the present invention are fungi such as Aspergillus species (such as those described in EP-A-0184438 and EP-A-0284603) and Trichoderma species; bacteria such as Bacillus species (such as those described in EP-A-0134048 and EP-A-0253455), Streptomyces species and Pseudomonas species; and yeasts such as Kluyveromyces species (such as those described in EP-A-0096430 and EP-A-0301670) and Saccharomyces species. By way of example, typical expression hosts may be selected from Aspergillus niger, Aspergillus niger var. tubigenis, Aspergillus niger var. awamori, Aspergillus aculeatis, Aspergillus nidulans, Aspergillus orvzae, Trichoderma reesei, Bacillus subtilis, Bacillus licheniformis, Bacillus amyloliquefaciens, Kluyveromyces lactis and Saccharomyces cerevisiae.

Polypeptides that are extensively modified may require correct processing to complete their function. In those instances, mammalian cell expression systems (such as HEK-293, CHO, HeLA) are required, and the polypeptides are expressed either intracellularly, on the cell membranes, or secreted in the culture media if preceded by an appropriate leader sequence.

The use of suitable host cells - such as yeast, fungal, plant and mammalian host cells - may provide for post-translational modifications (e.g. myristoylation, glycosylation, truncation, lapidation and tyrosine, serine or threonine phosphorylation) as may be needed to confer optimal biological activity on recombinant expression products of the present invention.

ORGANISM

The term "organism" in relation to the present invention includes any organism that could comprise the target according to the present invention and/or products obtained therefrom. Examples of organisms may include a fungus, yeast or a plant.

The term "transgenic organism" in relation to the present invention includes any organism that comprises the target according to the present invention and/or products obtained.

TRANSFORMATION OF HOST CELLS/HOST ORGANISMS

As indicated earlier, the host organism can be a prokaryotic or a eukaryotic organism. Examples of suitable prokaryotic hosts include *E. coli* and *Bacillus subtilis.* Teachings on the transformation of prokaryotic hosts is well documented in the art, for example see Sambrook *et al* (Molecular Cloning: A Laboratory Manual, 2nd edition, 1989, Cold Spring Harbor Laboratory Press) and Ausubel et al., Current Protocols in Molecular Biology (1995), John Wiley & Sons, Inc.

If a prokaryotic host is used then the nucleotide sequence may need to be suitably modified before transformation - such as by removal of introns.

In another embodiment the transgenic organism can be a yeast. In this regard, yeast have also been widely used as a vehicle for heterologous gene expression. The species *Saccharomyces cerevisiae* has a long history of industrial use, including its use for heterologous gene expression. Expression of heterologous genes in *Saccharomyces cerevisiae* has been reviewed by Goodey et al (1987, Yeast Biotechnology, D R Berry et al, eds, pp 401-429, Allen and Unwin, London) and by King et al (1989, Molecular and Cell Biology of Yeasts, E F Walton and G T Yarronton, eds, pp 107-133, Blackie, Glasgow).

For several reasons *Saccharomyces cerevisiae* is well suited for heterologous gene expression. First, it is non-pathogenic to humans and it is incapable of producing certain endotoxins. Second, it has a long history of safe use following centuries of commercial exploitation for various purposes. This has led to wide public acceptability. Third, the extensive commercial use and research devoted to the organism has resulted in a wealth of knowledge about the genetics and physiology as well as large-scale fermentation characteristics of *Saccharomyces cerevisiae.*

A review of the principles of heterologous gene expression in *Saccharomyces cerevisiae* and secretion of gene products is given by E Hinchcliffe E Kenny (1993, "Yeast as a vehicle for the expression of heterologous genes",

Yeasts, Vol 5, Anthony H Rose and J Stuart Harrison, eds, 2nd edition, Academic Press Ltd.).

Several types of yeast vectors are available, including integrative vectors, which require recombination with the host genome for their maintenance, and autonomously replicating plasmid vectors.

In order to prepare the transgenic Saccharomyces, expression constructs are prepared by inserting the nucleotide sequence of the present invention into a construct designed for expression in yeast. Several types of constructs used for heterologous expression have been developed. The constructs contain a promoter active in yeast fused to the nucleotide sequence of the present invention, usually a promoter of yeast origin, such as the GAL1 promoter, is used. Usually a signal sequence of yeast origin, such as the sequence encoding the SUC2 signal peptide, is used. A terminator active in yeast ends the expression system.

For the transformation of yeast several transformation protocols have been developed. For example, a transgenic Saccharomyces according to the present invention can be prepared by following the teachings of Hinnen et al (1978, Proceedings of the National Academy of Sciences of the USA 75, 1929); Beggs, J D (1978, Nature, London, 275, 104); and Ito, H et al (1983, J Bacteriology 153, 163-168).

The transformed yeast cells are selected using various selective markers. Among the markers used for transformation are a number of auxotrophic markers such as LEU2, HIS4 and TRP1, and dominant antibiotic resistance markers such as aminoglycoside antibiotic markers, eg G418.

Another host organism is a plant. The basic principle in the construction of genetically modified plants is to insert genetic information in the plant genome so as to obtain a stable maintenance of the inserted genetic material. Several techniques exist for inserting the genetic information, the two main principles being direct introduction of the genetic information and introduction of the genetic information by use of a vector system. A review of the general techniques may be found in articles by Potrykus (Annu Rev Plant Physiol Plant Mol Biol [1991] 42:205-225) and Christou (Agro-Food-Industry Hi-Tech March/April 1994 17-27). Further teachings on plant transformation may be found in EP-A-0449375.

Further hosts suitable for the nucleotide sequence of the present invention include higher eukaryotic cells, such as insect cells or vertebrate cells, particularly mammalian cells, including human cells, or nucleated cells from other multicellular organisms. In recent years propagation of vertebrate cells in culture (tissue culture) has become a routine procedure. Examples of useful mammalian host cell lines are epithelial or fibroblastic cell lines such as Chinese hamster ovary (CHO) cells, NIH 3T3 cells, HeLa cells or 293T cells.

The nucleotide sequence of the present invention may be stably incorporated into host cells or may be transiently expressed using methods known in the art. By way of example, stably transfected mammalian cells may be prepared by transfecting cells with an expression vector having a selectable marker gene, and growing the transfected cells under conditions selective for cells expressing the marker gene. To prepare transient transfectants, mammalian cells are transfected with a reporter gene to monitor transfection efficiency.

To produce such stably or transiently transfected cells, the cells should be transfected with a sufficient amount of the nucleotide sequence of the present invention. The precise amounts of the nucleotide sequence of the present invention may be empirically determined and optimised for a particular cell and assay.

Thus, the present invention also provides a method of transforming a host cell with a nucleotide sequence that is to be the target or is to express the target. Host cells transformed with the nucleotide sequence may be cultured under conditions suitable for the expression of the encoded protein. The protein produced by a recombinant cell may be displayed on the surface of the cell. If desired, and as will be understood by those of skill in the art, expression vectors containing coding sequences can be designed with signal sequences which direct secretion of the coding sequences through a particular prokaryotic or eukaryotic cell membrane. Other recombinant constructions may join the coding sequence to nucleotide sequence encoding a polypeptide domain which will facilitate purification of soluble proteins (Kroll DJ et al (1993) DNA Cell Biol 12:441-53).

<u>AGENT</u>

The agent may be an organic compound or other chemical. The agent can be an amino acid sequence or a chemical derivative thereof, or a combination thereof. The agent may even be a nucleotide sequence - which may be a sense sequence or an anti-sense sequence. The agent may even be an antibody.

Typically, the agents will be organic compounds. Typically the organic compounds will comprise two or more hydrocarbyl groups. Here, the term "hydrocarbyl group" means a group comprising at least C and H and may optionally comprise one or more other suitable substituents. Examples of such substituents may include halo-, alkoxy-, nitro-, an alkyl group, a cyclic group etc. In addition to the possibility of the substituents being a cyclic group, a combination of substituents may form a cyclic group. If the hydrocarbyl group comprises more than one C then those carbons need not necessarily be linked to each other. For example, at least two of the carbons may be linked *via* a suitable element or group. Thus, the hydrocarbyl group may contain hetero atoms. Suitable hetero atoms will be apparent to those skilled in the art and include, for instance, sulphur, nitrogen and oxygen. For some applications, preferably the agent

comprises at least one cyclic group,. The cyclic group may be a polycyclic group, such as a non-fused polycyclic group. For some applications, the agent comprises at least the one of said cyclic groups linked to another hydrocarbyl group.

Examples of agents may be the compounds disclosed in WO-A-00/38680 (claiming priority from *inter alia* GB patent application No. 9921375.3 filed 10 September 1999) - the contents of which are incorporated herein by reference.

The compounds of WO-A-00/38680 may be of the Formula (I);

$$[R_{egion}\ \alpha] - [R_{egion}\ \beta] - [R_{egion}\ \gamma] - [R_{egion}\ \delta] \qquad (I)$$

wherein **[$R_{egion}$ α]** is selected from the group consisting of:

- A. Aryl heterocyclyl substituent components comprising:
  - 1. hetero-phenylmethylene moieties of partial Formula (1.0.0):

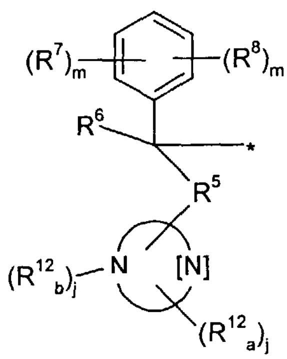

(1.0.0)

--- wherein: the symbol "*" indicates the point of attachment of the moiety of partial Formula (1.0.0) to $R_{egion}$ β, as hereinafter defined;

--- $R^5$ is a member selected from the group consisting of a direct bond; -O-; -C(=O)-; $-NR^4-$; and $-S(=O)_p-$; where:

---- $R^4$ is hydrogen or $(C_1\text{-}C_2)$alkyl;

--- $R^6$ is a member selected from the group consisting of hydrogen; $(C_1\text{-}C_2)$alkyl; $(C_1\text{-}C_2)$alkoxy; -CN; -OH; and $-C(=O)NH_2$;

--- j is an integer selected from 0, 1, and 2;

--- m is an integer selected from 0, 1, and 2;

--- $R^7$ and $R^8$ are each a member selected from the group consisting of -F; -Cl; $-CO_2R^4$; -OH; -CN; $-CONR^4_aR^4_b$; $-NR^4_aR^4_b$-; $-NR^4_aC(=O)R^4_b$; $-NR^4_aC(=O)OR^4_b$; $-NR^4_aS(=O)_pR^4_b$; $-S(=O)_pNR^4_aR^4_b$; $(C_1\text{-}C_4)$ alkyl, and $(C_1\text{-}C_4)$alkoxy wherein said alkyl and alkoxy are each substituted with 0 to 3 substituents independently selected from F and Cl;
$(C_1\text{-}C_2)$alkoxycarbonyl; $(C_1\text{-}C_2)$alkylcarbonyl; and $(C_1\text{-}C_2)$alkylcarbonyloxy; where:

---- p is an integer selected from 0, 1, and 2;
---- $R^4_a$ and $R^4_b$ are each independently selected from hydrogen and $(C_1\text{-}C_2)$alkyl;

--- the moiety represented by partial Formula (1.0.1):

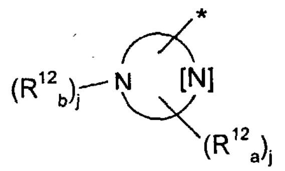

(1.0.1)

in partial Formula (1.0.0) represents a monocyclic heterocyclic group, or a bicyclic benzo-fused ring system containing said heterocyclic group wherein said heterocyclic group contains a total of 5- or 6- members of which one or two of said members is nitrogen, the presence of the optional second nitrogen atom being represented by: "[N]"; wherein said heterocyclic group or ring system are selected from the group consisting of pyrrolyl; pyrazolyl; imidazolyl; pyridinyl; pyrazinyl; pyrimidinyl; pyridazinyl; piperazinyl; indolyl; indazolinyl; benzimidazolyl; quinolinyl; *iso*-quinolinyl; and quinazolinyl; wherein:

---- $R^{12}_a$ is a member selected from the group consisting of hydrogen; F; Cl; $-CO_2R^4$; oxo; -OH; CN; $NH_2$; $NH(C_1-C_2)$alkyl; $N(C_1-C_2)_2$dialkyl; $-CF_3$; $(C_1-C_4)$alkyl; $(C_2-C_4)$alkenyl; $(C_1-C_4)$alkoxy; $(C_3-C_7)$cycloalkyl; and phenyl; wherein said alkyl, alkenyl, alkoxy, cycloalkyl and phenyl are substituted with 0 to 2 substituents $R^9$ where:

----- $R^9$ is a member independently selected from the group consisting of F; Cl; $-CO_2R^4$; -OH; cyano; $-CONR^4_aR^4_b$; $-NR^4_aR^4_b$-; $-NR^4_aC(=O)R^4_b$; $-NR^4_aC(=O)OR^4_b$; $-NR^4_aS(=O)_pR^4_b$; $-S(=O)_pNR^4_aR^4_b$; $(C_1-C_4)$alkyl including dimethyl, and $(C_1-C_4)$alkoxy wherein said alkyl and alkoxy are each independently substituted with 0 to 3 substituents independently selected from F and Cl; $(C_1-C_2)$alkoxycarbonyl; $(C_1-C_2)$alkylcarbonyl; and $(C_1-C_2)$alkylcarbonyloxy; and

---- $R^{12}_b$ is absent or is a member selected from the group consisting of hydrogen; $(C_1-C_4)$alkyl;$(C_2-C_4)$alkenyl; $(C_1-C_2)$alkoxy; $(C_3-C_7)$cycloalkyl; and phenyl; wherein said alkyl, alkenyl, alkoxy, cycloalkyl and phenyl are substituted with 0 to 2 substituents $R^9$ wherein $R^9$ has the same meaning as above, except that it is selected independently selected therefrom; and

2. hetero-phenylmethylene moieties of partial Formula (1.1.0):

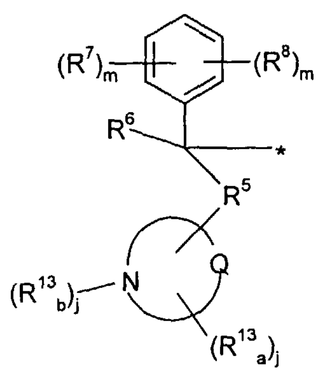

(1.1.0)

--- wherein: the symbol " * "; $R^5$; $R^6$; $R^7$; $R^8$; j and m are as defined further above, except that all of the above-recited substituents are selected independently of their selection above;

--- the moiety represented by partial Formula (1.1.1):

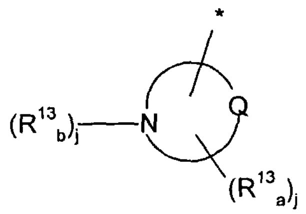

(1.1.1)

in partial Formula (1.1.0) represents:

- ---- a. a monocyclic heterocyclic group containing a total of 5 or 6 members of which one said member is nitrogen and Q is selected from O and S where said S may optionally be in the sulfonate form, -S(=O)$_2$; wherein said heterocyclic group is selected from the group consisting of oxazolyl; oxazolidinyl; *iso*xazolyl; thiazolyl; thiazolidinyl; *iso*-thiazolyl; morpholinyl; and thiomorpholinyl; or
- ---- b. a monocyclic heterocyclic group containing a total of 5- or 6- member s of which two said members are nitrogen and a third or fourth said member is independently selected from N, O, and S where said S may optionally be in the sulfonate form, -S(=O)$_2$; wherein said heterocyclic group is selected from the group consisting of triazolyl; triazinyl; tetrazolyl; oxadiazolyl; thiadiazolyl; and
- ---- $R^{13}_a$ is selected from the group consisting of hydrogen; F; Cl; -CO$_2$R$^4$; oxo; -OH; CN; NH$_2$; NH(C$_1$-C$_2$)alkyl; N(C$_1$-C$_2$)$_2$dialkyl; -CF$_3$; (C$_1$-C$_4$)alkyl; (C$_2$-C$_4$)alkenyl; (C$_1$-C$_2$)alkoxy; (C$_3$-C$_7$)cycloalkyl; and phenyl; wherein said alkyl, alkenyl, alkoxy, cycloalkyl and phenyl are substituted with 0 to 2 substituents $R^{11}$ where:

  - ----- $R^{11}$ is a member selected from the group consisting of F; Cl; -CO$_2$R$^4$; -OH; -CN; -CONR$^4_a$R$^4_b$; -NR$^4_a$R$^4_b$; -NR$^4_a$C(=O)R$^4_b$; -NR$^4_a$C(=O)OR$^4_b$; -NR$^4_a$S(=O)$_p$R$^4_b$; -S(=O)$_p$NR$^4_a$R$^4_b$; (C$_1$-C$_4$)alkyl including dimethyl, and (C$_1$-C$_4$)alkoxy wherein said alkyl and alkoxy are each independently substituted with 0 to 3 substituents independently selected from F and Cl; (C$_1$-C$_2$)alkoxycarbonyl; (C$_1$-C$_2$)alkylcarbonyl; and (C$_1$-C$_2$)alkylcarbonyloxy; and

- ---- $R^{13}_b$ is a member selected from the group consisting of hydrogen; (C$_1$-C$_4$)alkyl; (C$_2$-C$_4$)alkenyl; (C$_1$-C$_2$)alkoxy; (C$_3$-C$_7$)cycloalkyl; C(=O)(C$_1$-C$_4$)alkyl; S(=O)$_2$(C$_1$-C$_4$)alkyl; and phenyl; wherein said alkyl, alkenyl, alkoxy, cycloalkyl and phenyl are substituted with 0 to 2 substituents $R^{11}$ wherein $R^{11}$ has the same meaning as in above, except that it is selected independently;

- B. a (substituted)-amido-aryl or -heterocyclyl moiety selected from the group consisting of

-- 1. alkyl-, alkenyl-, and alkynyl-substituted-amido-aryl moieties of partial Formula (2.0.0):

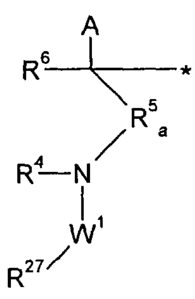

(2.0.0)

--- wherein: the symbol "*"; R$^4$ and R$^6$; are as defined above, except that all of the above-recited substituents are selected independently of their selection above;

--- A is a member selected from the group consisting of:

---- 1. the moiety of partial Formula (2.0.3)

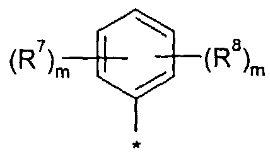

(2.0.3)

----- wherein: the symbol $R^7$; $R^8$ and m are as defined above, except that all of the above-recited substituents are selected independently of their selection above; and the symbol: "*" indicates the point of attachment of the moiety A to the, remaining portions of partial Formula (2.0.0);

---- 2. the moiety of partial Formula (2.0.4)

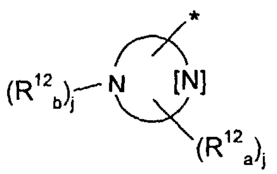

(2.0.4)

which represents a monocyclic heterocyclic group, selected from the group consisting of pyrrolyl; pyrazolyl; imidazolyl; pyridinyl; pyrazinyl; pyrimidinyl; wherein: the symbol $R^{12}{}_a$ and $R^{12}{}_b$ are as defined above, except that all of the above-recited substituents are selected independently of their selection above; and the symbol: "*" indicates the point of attachment of the moiety A to the other, remaining portions of partial Formula (2.0.0);

---- 3. the moiety of partial Formula (2.0.5)

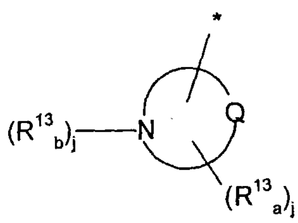

(2.0.5)

which represents

----- a. a monocyclic heteroaromatic group containing a total of 5- members of which one said member is nitrogen and Q is selected from O and S where said S may optionally be in the sulfonate form, $-S(=O)_2$; selected from the group consisting of oxazolyl; *iso*xazolyl; thiazolyl; and *iso*-thiazolyl; or

----- b. a monocyclic heterocyclic group containing a total of 5- or 6- members of which two said members are nitrogen and a third or fourth said member is independently selected from N, O, and S where said S may optionally be in the sulfonate form, $-S(=O)_2$; selected from the group consisting of triazolyl; triazinyl; tetrazolyl; oxadiazolyl; and thiadiazolyl; and

------wherein: the $R^{13}{}_a$, $R^{13}{}_b$ and j are as defined above, except that all of the above-recited substituents are selected independently of their selection above; and the symbol: "*" indicates the point of attachment of the moiety A to the other, remaining portions of partial Formula (2.0.2);

--- $R^5{}_a$ is a member selected from the group consisting of a direct bond; -C(=O)-; and -S(=O)$_2$-;

--- $W^1$ is (1.) a direct bond; (2.) in the case where $R^5{}_a$ is -C(=O)- or -S(=O)$_2$, $W^1$ is a direct bond or -($C_1$-$C_3$) alkylene- wherein any single carbon atom thereof is substituted by 0 to 2 substituents $R^{23}$ where $R^{23}$ is a member selected from the group consisting of-F; -Cl; -$CO_2R^4$; -OH; -CN; ($C_1$-$C_4$)alkoxy; ($C_3$-$C_7$)cycloalkyl; and phenyl; wherein said alkoxy, cycloalkyl, and phenyl are substituted with 0 to 2 substituents $R^{11}$, wherein said $R^{11}$ is as defined above, except that all of the above-recited substituents are selected independently of their selection above; or (3.) is a member independently selected from the group consisting of the moieties of partial Formulas (2.0.6) through (2.0.16), inclusive:

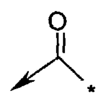 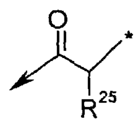 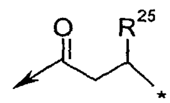

(2.0.6) (2.0.7) (2.0.8)

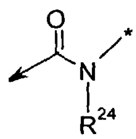 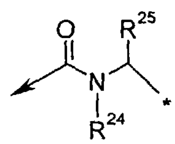 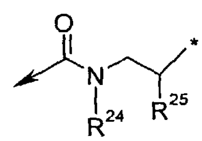

(2.0.9) (2.0.10) (2.0.11)

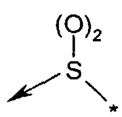 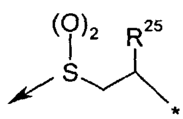 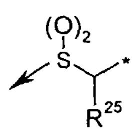 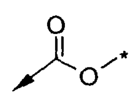 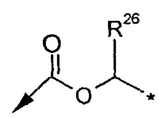

(2.0.12)

(2.0.13) (2.0.14) (2.0.15) (2.0.16)

---- wherein: the symbol: "→" indicates the point of attachment of the moiety $W^1$ to the nitrogen atom in partial Formula (2.0.0), and the symbol: "*" indicates the point of attachment of the moiety $W^1$ to the other, remaining portions of partial Formula (2.0.0); and $R^4$ is as defined further above, but selected on an independent basis;

--------$R^{24}$ is selected from the group consisting of hydrogen and ($C_1$-$C_4$)alkyl; and

---- $R^{25}$ and $R^{26}$are each selected from the group consisting of -OH; ($C_{1}$-$C_2$)alkyl substituted by 0 to 3 substituents selected from F; and OH; and ($C_{1}$-$C_2$)alkoxy; and

--- $R^{27}$ is selected from the group consisting of ($C_{1}$-$C_6$)alkyl; ($C_{2}$-$C_6$)alkenyl; and ($C_{2}$-$C_6$)alkynyl; wherein said alkyl, alkenyl, and alkynyl groups comprising $R^{27}$ are substituted with 0 to 3 substituents $R^{28}$ where:

---- $R^{28}$ is selected from the group consisting of phenyl; F or Cl; oxo; hydroxy; ($C_{1}$-$C_2$)alkyl; ($C_{1}$-$C_3$)alkoxy; -C(=O)$OR^{29}$; -C(=O)($C_1$-$C_4$)alkyl; -S(=O)$_2$($C_1$-$C_4$)alkyl; -C(=O)$NR^{29}R^{30}$; -$NR^{29}R^{30}$; -$NR^{29}$C(=O)$R^{30}$; -$NR^{29}$C(=O)$OR^{30}$; -$NR^{29}$S(=O)$_p$$R^{30}$; and -S(=O)$_2$$NR^{29}R^{30}$, where:

----- $R^{29}$ and $R^{30}$ are each a member independently selected from the group consisting of hydrogen and ($C_{1}$-$C_4$)alkyl substituted by 0 to 3 substituents selected from the group consisting of F and Cl;

-- 2. cycloalkyl-substituted-amido-aryl moieties of partial Formula (2.1.0):

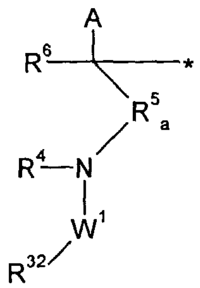

(2.1.0)

--- wherein: A; $W^1$; the symbol "*"; $R^4$; $R^5{}_a$; and $R^6$ have the same meaning as set out above, except that all of the above-recited substituents are selected independently of their selection above; and

--- $R^{32}$ is a member selected from the group consisting of $-(CH_2)_n-(C_3-C_7)$cycloalkyl, where n is an integer selected from 0, 1, and 2; in the event n is 0, then the α-carbon atom of said $(C_3-C_7)$cycloalkyl is substituted by 0 or 1 $(C_1-C_4)$alkyl or phenyl, where said alkyl or phenyl are substituted by 0, 1, or 2 of $CH_3$, $OCH_3$, OH or $NH_2$; and in the event that n is 1 or 2, the resulting methylene or ethylene is substituted by 0 or 1 of F; $NH_2$; $N(CH_3)_2$; OH; $OCH_3$; $(C_1-C_4)$alkyl; or phenyl; where said alkyl and phenyl are substituted by 0, 1, or 2 of $CH_3$, $OCH_3$, OH, and $NH_2$; and further wherein said $(C_3-C_7)$cycloalkyl is substituted by 0 to 3 substituents $R^{28}$ where $R^{28}$ is as defined further above, but selected independently

-- 3. aryl and heterocyclic-substituted-amido-aryl moieties of partial Formula (2.2.0):

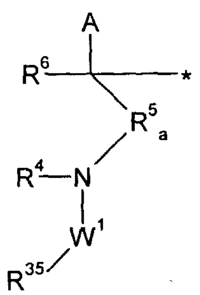

(2.2.0)

--- wherein: A; $W^1$; the symbol: "*"; $R^4$; $R^5{}_a$; and $R^6$ have the same meaning as set out above, except that all of the above-recited substituents are selected independently of their selection above; and

--- $R^{35}$ is selected from the group consisting of phenyl; furyl; tetrahydrofuranyl; tetrahydropyranyl; oxetanyl; thienyl; pyrrolyl; pyrrolidinyl; oxazolyl; isoxazolyl; thiazolyl; isothiazolyl; imidazolyl; pyrazolyl; oxadiazolyl; thiadiazolyl; triazolyl; pyridyl; pyrazinyl; pyridazinyl; piperazinyl; pyrimidinyl; pyranyl; azetidinyl; morpholinyl; parathiazinyl; indolyl; indolinyl; benzo[*b*]furanyl; 2;3-dihydrobenzofuranyl; benzothienyl; 1H-indazolyl; benzimidazolyl; benzoxazolyl; benzisoxazolyl; benzthiazolyl; quinolinyl; isoquinolinyl; phthalazinyl; quinazolinyl; and quinoxalinyl; wherein (1.) said group $R^{35}$ may be substituted upon any one or more carbon atoms thereof by 0 to 3 substituents $R^{28}$ where $R^{28}$ is as defined above, except that it is selected independently; (2.) said group $R^{35}$ is substituted with respect to any one or more nitrogen atoms thereof that is not a point of attachment of said aryl or heterocyclic moiety, by 0 to 3 substituents $R^{13}{}_b$ where $R^{13}{}_b$ is as defined above, except that it is selected independently; and (3.) said group $R^{35}$ with respect to any sulfur atom thereof that is not a point of attachment of said heterocyclic moiety, is substituted by 0 or 2 oxygen atoms;

**[$R_{egion}$ β]** is an alkyl bridging element of partial Formula (3.0.0):

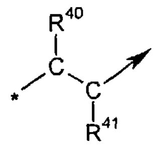

(3.0.0)

wherein:

- "*" is a symbol which represents the point of attachment of the moiety of partial Formula (3.0.0) to $R_{egion}$ α;
- "→ " is a symbol which represents the point of attachment of the moiety of partial Formula (3.0.0) to $R_{egion}$ γ;
- $R^{40}$ and $R^{41}$ are independently selected from the group consisting of hydrogen; ($C_1$-$C_2$) alkyl including dimethyl; hydroxy; and ($C_1$-$C_3$) alkoxy;

**[$R_{egion}$ γ]** is an aza-bicyclic moiety of partial Formula (4.2.0):

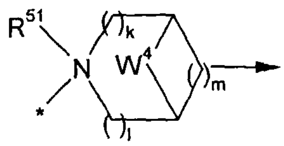

(4.2.0)

- wherein
- "*" is a symbol which represents the point of attachment of the moiety of partial Formula (4.2.0) to $R_{egion}$ β;
- "→" is a symbol representing a covalent bond from any of the carbon atoms of the moiety of partial Formula (4.2.0) to $R_{egion}$ δ;
- $W^4$ is a direct bond; or is a member independently selected from the group consisting of partial Formulas (4.2.1) through (4.2.6 ):

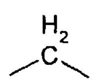
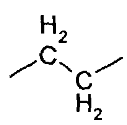
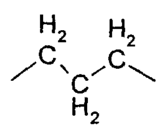

(4.2.1) (4.2.2) (4.2.3)

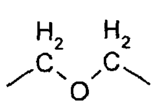
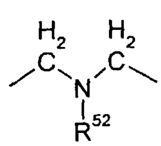
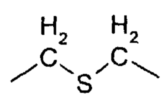

(4.2.4) (4.2.5) (4.2.6)

- where:
- $R^{52}$ is a member selected from the group consisting of hydrogen; phenyl; ($C_1$-$C_4$)alkyl substituted by 0 or 1 substituent independently selected from ($C_1$-$C_2$)alkoxy and $-CO_2R^4$; ($C_3$-$C_6$)cycloalkyl; $-CO_2R^4$; →O; C(=O)($C_1$-$C_3$)alkyl; -C(=O)$NR^4_aR^4_b$; -S(=O)($C_1$-$C_4$)alkyl; and ($C_1$-$C_2$)alkylcarbonyl; where $R^4$, $R^4_a$, and $R^4_b$; are as defined above, but selected on an independent basis;

- $R^{51}$ is absent or is a member selected from the group consisting of ($C_1$-$C_4$)alkyl substituted by 0 or 1 substituent

independently selected from $(C_1\text{-}C_2)$alkoxy and $-CO_2R^4$ where $R^4$ is as defined above; and →O; it being understood that in the case where substituent $R^{51}$ is present, the nitrogen atom is in quaternary form; and

-- k, l and m are each an integer selected from 0, 1, 2, and 3;

**[$R_{egion}$ δ]** is a member selected from the group consisting of:

- A. a two-nitrogen-atom-containing five-membered heterocyclic moiety of partial Formulas (5.0.0) through (5.0.10):

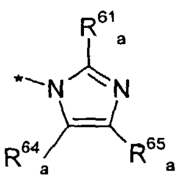

**(5.0.0)**

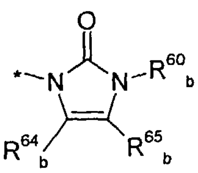

**(5.0.1)**

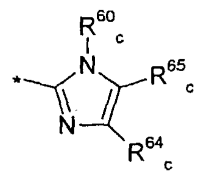

**(5.0.2)**

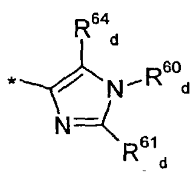

**(5.0.3)**

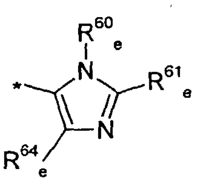

**(5.0.4)**

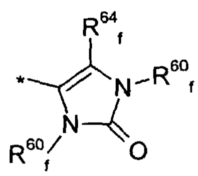

**(5.0.5)**

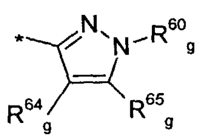

**(5.0.6)**

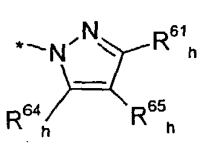

(5.0.7)

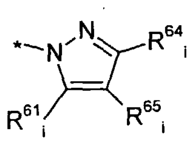

(5.0.8)

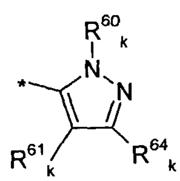

(5.0.9)

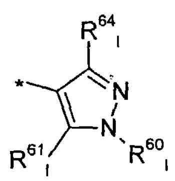

(5.0.10)

-- wherein: the symbol: "*" indicates the point of attachment of each of the moieties of partial Formulas (5.0.0) through (5.0.10), inclusive, to $R_{egion}$ γ;

-- $R^{60}_b$ through $R^{60}_g$, inclusive, $R^{60}_k$, and $R^{60}_l$ are each a member selected from the group consisting of hydrogen; $-CO_2R^4$; $-C(=O)NR^4_aR^4_b$; $-S(=O)_pNR^4_aR^4_b$; where: $R^4$; $R^4_a$; and $R^4_b$ are as defined above but selected on an independent basis; →O; $(C_1\text{-}C_2)$alkylcarbonyl; $-(C_1\text{-}C_4)$alkyl; $-(CH_2)_n\text{-}(C_3\text{-}C_7)$cycloalkyl; $-(C_2\text{-}C_3)$alkenyl; $-(CH_2)_n$-(phenyl); and $-(CH_2)_n-(HET_1)$, where n is an integer independently selected from 0, 1, and 2; wherein said $(C_1\text{-}C_4)$alkyl, alkenyl, cycloalkyl, phenyl, and heterocyclyl groups are independently substituted with 0 to 3 substituents $R^{66}$, where:

--- $HET_1$ is a heterocyclyl group selected from the group consisting of thienyl; oxazolyl; isoxazolyl; thiazolyl; isothiazolyl; pyrazolyl; oxadiazolyl; thiadiazolyl; triazolyl; pyridyl; pyrazinyl; pyridazinyl; pyrimidinyl; parathiazinyl; and morpholinyl; where:

--- $R^{66}$ is a member selected from the group consisting of -F; -Cl; -OH; cyano; $-C(=O)OR^{68}$; $-C(=O)NR^{68}R^{69}$; $-NR^{68}R^{69}$; $-NR^{68}C(=O)R^{69}$; $-NR^{68}C(=O)OR^{69}$; $-NR^{68}S(=O)_2R^{69}$; $-S(=O)_2NR^{68}R^{69}$; $(C_1\text{-}C_4)$alkyl including dimethyl, and $(C_1\text{-}C_4)$alkoxy wherein said alkyl and alkoxy are each independently substituted with 0 to 3 substituents independently selected from F and Cl; $(C_1\text{-}C_2)$alkoxycarbonyl; $(C_1\text{-}C_2)$alkylcarbonyl; and $(C_1\text{-}C_2)$alkylcarbonyloxy, where:

---- $R^{68}$ and $R^{69}$ are each a member selected from the group consisting of hydrogen; and $(C_1\text{-}C_2)$alkyl; and where said:

-- $R^{61}{}_a$; $R^{61}{}_d$; $R^{61}{}_e$; and $R^{61}{}_h$ through $R^{61}{}_l$ inclusive; $R^{64}{}_a$ through $R^{64}{}_l$ inclusive; $R^{65}{}_a$ through $R^{65}{}_c$ inclusive; and $R^{65}{}_g$ through $R^{65}{}_i$ inclusive are each a member selected from the group consisting of hydrogen; -OH; $-CF_3$; cyano; $-(C_1\text{-}C_3)$alkoxy; $-C(=O)OR^4$; $-C(=O)NR^4{}_aR^4{}_b$; $-NR^4{}_aR^4{}_b$; $-NR^4{}_aC(=O)R^4{}_b$;$-NR^4{}_aC(=O)OR^4{}_b$; $-NR^4{}_aS(=O)_pR^4{}_b$; $-S(=O)_pNR^4{}_aR^4{}_b$; where: $R^4$; $R^4{}_a$; and $R^4{}_b$ are as defined further above but selected on an independent basis; $-(C_1\text{-}C_4)$alkyl; $-(CH_2)_n\text{-}(C_3\text{-}C_7)$cycloalkyl; $-(C_2\text{-}C_3)$alkenyl; $-(CH_2)_n$-(phenyl); and $-(CH_2)_n$-($HET_1$), where n is an integer selected from 0, 1, and 2; wherein said $(C_1\text{-}C_4)$alkyl, alkenyl, cycloalkyl, phenyl, and heterocyclyl groups where heterocyclyl groups is as defined above, are independently substituted with 0 to 3 substituents $R^{66}$ where:

--- $R^{66}$ is as defined above, or:

-- $R^{64}{}_a$ through $R^{64}{}_c$ inclusive; $R^{64}{}_g$ through $R^{64}{}_i$ inclusive; $R^{65}{}_a$ through $R^{65}{}_c$ inclusive; and $R^{65}{}_g$ through $R^{65}{}_l$ inclusive may be taken together in their same subscript denominated pairs along with the remaining portions of the moieties of partial Formulas (5.0.0) through (5.0.2), and (5.0.6) through (5.0.8), to form a fused bicyclic ring system comprising a member independently selected from the group consisting of benzimidazolyl; purinyl, *i.e.*, imidazopyrimidinyl; and imidazopyridinyl; wherein said fused bicyclic ring system is independently substituted with 0 to 3 substituents $R^{66}$, where $R^{66}$ has the same meaning as set out further above, except that it is independently selected therefrom;

- B. a (substituted)-amide, carbamate, or urea moiety selected from the group consisting of:

-- 1. alkyl-, cycloalkyl-, and alkenyl-(substituted)-amide, carbamate, or urea moieties of partial Formula (5.1.0):

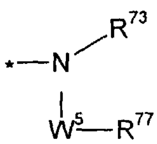

(5.1.0)

--- wherein: the symbol "*" is as defined above;
--- $R^{73}$ is a member selected from the group consisting of hydrogen and $(C_1\text{-}C_2)$alkyl;
--- $W^5$ is selected from the group consisting the moieties of partial Formulas (5.1.1) through (5.1.12):

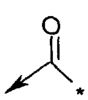 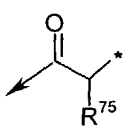 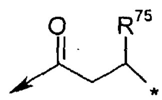 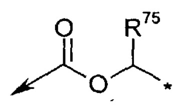

(5.1.1) (5.1.2) (5.1.3) (5.1.4)

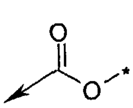 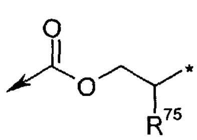 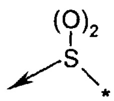 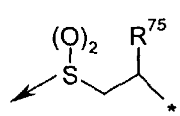

(5.1.5) (5.1.6) (5.1.7) (5.1.8)

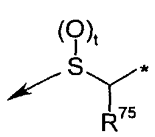 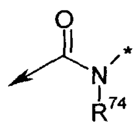 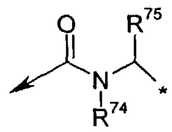 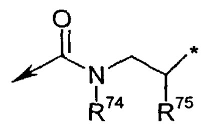

(5.1.9) (5.1.10) (5.1.11) (5.1.12)

---- wherein: the symbol: "→" indicates the point of attachment of the moiety $W^5$ represented by partial Formulas (5.1.1) through (5.1.12), inclusive, to the nitrogen atom in partial Formula (5.1.0), and the symbol: "*" indicates the point of attachment of the moiety $W^5$ to $R^{77}$ as defined further below;

---- $R^{74}$ and $R^{75}$ are each selected from the group consisting of hydrogen; $(C_{1-}C_2)$alkyl substituted by 0 or 1 substituent independently selected from OH; and $(C_{1-}C_2)$alkoxy; and

--- $R^{77}$ is a member selected from the group consisting of $(C_{1-}C_6)$alkyl; $(C_{2-}C_6)$alkenyl; and $-(CH_2)_n-(C_{3-}C_7)$ cycloalkyl, where n is an integer selected from 0, 1, and 2; and wherein said alkyl, alkenyl, and cycloalkyl groups comprising $R^{77}$ are substituted with 0 to 3 substituents $R^{78}$, where:

---- $R^{78}$ is a member selected from the group consisting of oxo; -OH; $-(C_{1-}C_2)$alkyl; $-(C_{1-}C_3)$alkoxy; $-CF_3$; $-C(=O)OR^{79}$; $-C(=O)NR^{79}R^{80}$; $-NR^{79}R^{80}$; $-NR^{79}C(=O)R^{80}$; $-NR^{79}C(=O)OR^{80}$; $-NR^{79}S(=O)_2R^{80}$; and $-S(=O)_2NR^{79}R^{80}$, where:

----- $R^{79}$ and $R^{80}$ are each a member independently selected from the group consisting of hydrogen; and $(C_{1-}C_4)$alkyl; and

-- 2. aryl and heterocyclyl-(substituted)-amide, carbamate, or urea moieties of partial Formula (5.2.0):

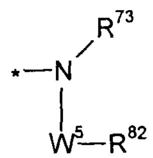

(5.2.0)

--- wherein: the symbol: "*"; $R^{73}$; and $W^5$ have the same meanings as under the definitions of partial Formula (5.1.0) above, except that they are independently selected therefrom; and under $W^5$ the symbols: "→" and " *" are as defined under partial Formula (5.1.0); and

--- $R^{82}$ is a member selected from the group consisting of phenyl; cinnolinyl; furyl; thienyl; pyrrolyl; oxazolyl; isoxazolyl; thiazolyl; isothiazolyl; imidazolyl; imidazolinyl; pyrazolyl; pyrazolinyl; oxadiazolyl; thiadiazolyl; triazolyl; pyridyl; pyrazinyl; pyridazinyl; pyrimidinyl; parathiazinyl; indolyl; isoindolyl; indolinyl; benzo[*b*] furanyl; 2;3-dihydrobenzofuranyl; benzo[*b*]thiophenyl; 1H-indazolyl; benzimidazolyl; benzthiazolyl; quinolinyl; isoquinolinyl; phthalazinyl; quinazolinyl; quinoxalinyl; wherein:

---- the aryl or heterocyclyl moiety is substituted by 0 to 3 substituents $R^{78}$ where $R^{78}$ is as defined above, but selected on an independent basis; or

- C. a (substituted)-heterocyclyl moiety independently selected from the group consisting of:

-- 1. a heterocyclyl moiety of partial Formula (5.3.0):

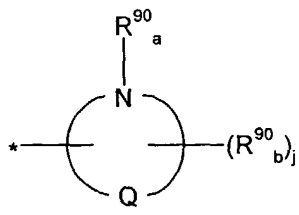

(5.3.0)

-- wherein: the symbol: " * " indicates the point of attachment of partial Formula (5.3.0) to Region $\gamma$; Q is N, O or S and

-- partial Formula (5.3.0) represents:

--- a. a monocyclic heterocyclic group containing a total of 5- members of which one said member is nitrogen and a second said member is selected from O and S where said S may optionally be in the sulfonate form, wherein said heterocyclic group is selected from the group consisting of oxazolyl; *iso*xazolyl; thiazolyl; and *iso*-thiazolyl; or

---- b. a monocyclic heterocyclic group containing a total of 5- members of which two said members are nitrogen and a third or fourth said member is independently selected from N, O, and S where said S may optionally be in the sulfonate form, $-S(=O)_2$; wherein said heterocyclic group is independently selected from the group consisting of triazolyl; tetrazolyl; oxadiazolyl; and thiadiazolyl; and

-- $R^{90}{}_a$ and $R^{90}{}_b$ are each a member independently selected from the group consisting of hydrogen, $-(C_{1-}C_2)$alkylcarbonyl; $-(C_{1-}C_4)$alkyl; $-(CH_2)_n-(C_{3-}C_7)$cycloalkyl; $-(C_{2-}C_3)$alkenyl; $-(CH_2)_{n-}$(phenyl); and $-(CH_2)_{n-}(HET_2)$, where n is an integer independently selected from 0, 1, and 2; wherein said $(C_{1-}C_4)$alkyl, alkenyl, cycloalkyl, phenyl, and $HET_2$ groups are independently substituted with 0 to 3 substituents $R^{91}$, where:

--- j has the same meaning as set forth above, but is selected on an independent basis therefrom;

--- $HET_2$ is a heterocyclyl group selected from the group consisting of thienyl; oxazolyl; isoxazolyl; thiazolyl; isothiazolyl; pyrazolyl; oxadiazolyl; thiadiazolyl; triazolyl; pyridyl; pyrazinyl; pyridazinyl; pyrimidinyl; parathiazinyl; and morpholinyl; where:

---- $R^{91}$ is selected from the group consisting of -F; -Cl; $-CO_2R^4$; -OH; -CN; $-CONR^{93}R^{94}$; $-NR^{93}R^{94}$; $C(=O)(C_1-C_4)$alkyl; $-NR^{93}C(=O)R^{94}$; $-NR^{93}C(=O)OR^{94}$; $-NR^{93}S(=O)R^{94}$; $-S(=O)NR^{93}R^{94}$; $(C_1-C_4)$alkyl including dimethyl, and $(C_{1-}C_4)$alkoxy wherein said alkyl and alkoxy are each independently substituted with 0 to 3 substituents independently selected from F and Cl; $(C_{1-}C_2)$alkoxycarbonyl; $(C_{1-}C_2)$alkylcarbonyl; and $(C_{1-}C_2)$alkylcarbonyloxy; wherein:

----- $R^{93}$and $R^{94}$ are each a member independently selected from the group consisting of hydrogen; and $(C_{1-}C_2)$alkyl; and

2. a heterocyclyl moiety of partial Formula (5.4.0):

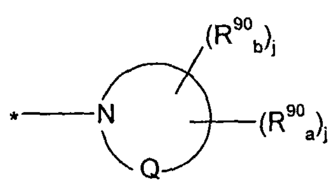

(5.4.0)

--- wherein: $R^{90a}$; $R^{90b}$; and j have the same meanings as set out above, but are selected independently.

Preferred compounds of WO-A-00/38680 are selected from the group consisting of

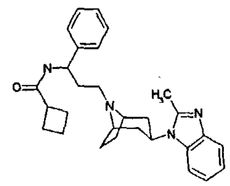

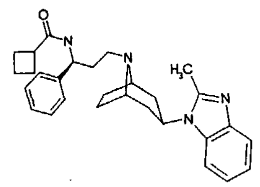

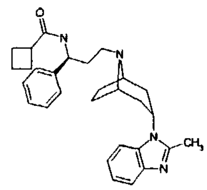

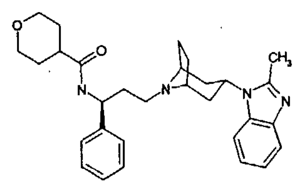

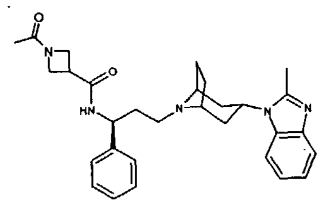

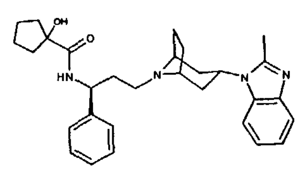

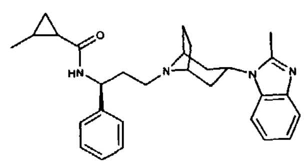

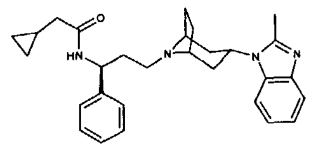

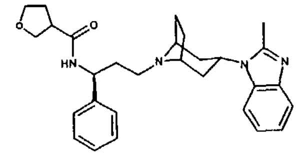

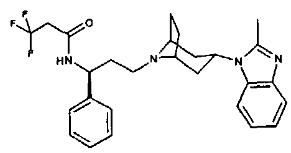

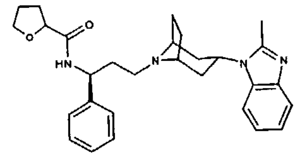

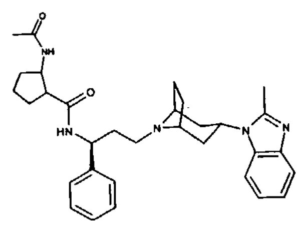

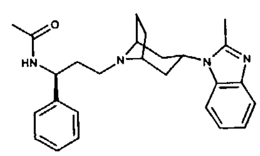

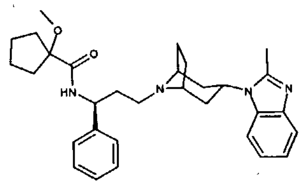

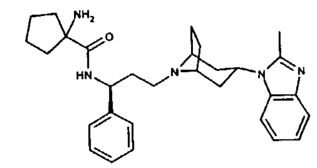

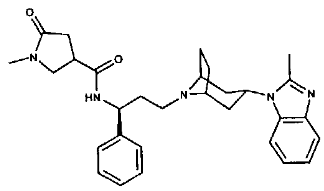

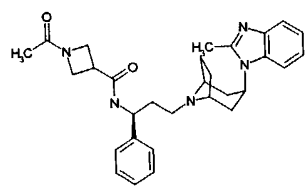

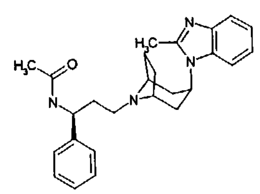

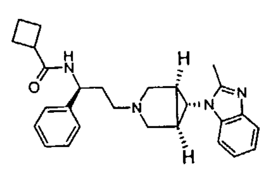

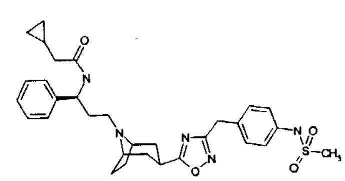

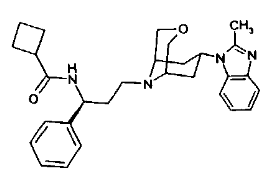

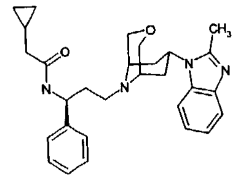

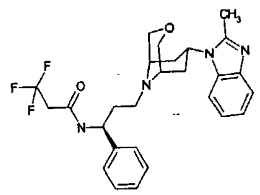

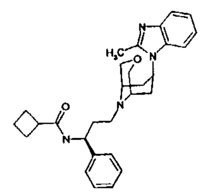

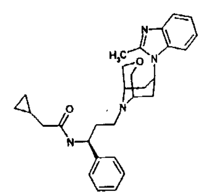

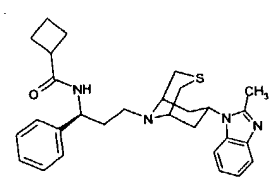

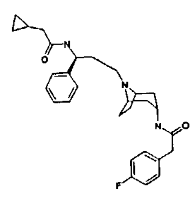

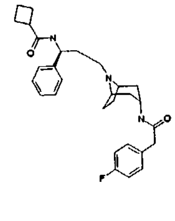

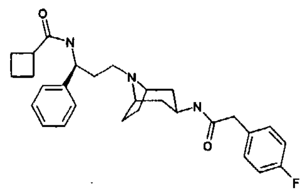

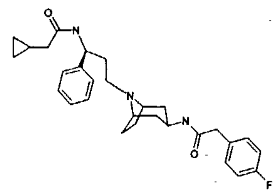

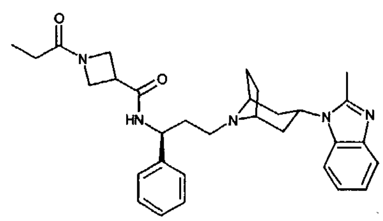

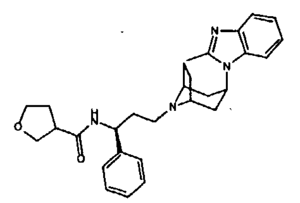

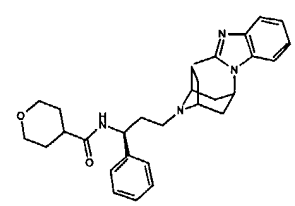

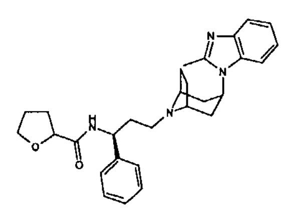

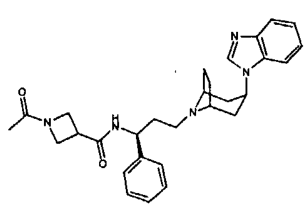

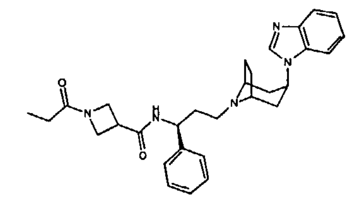

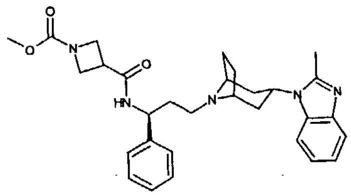

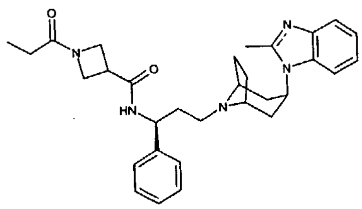

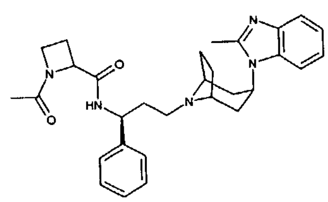

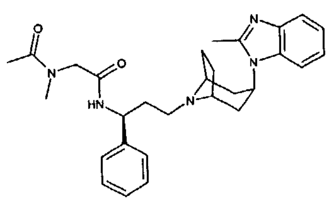

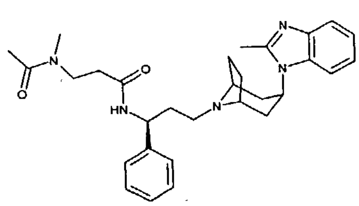

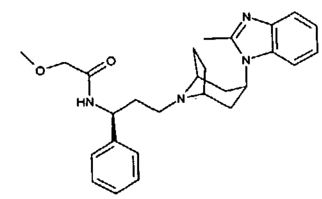

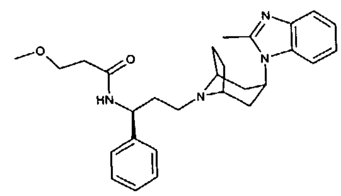

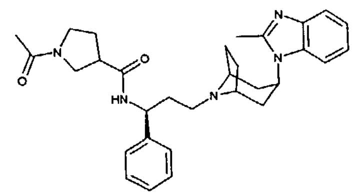

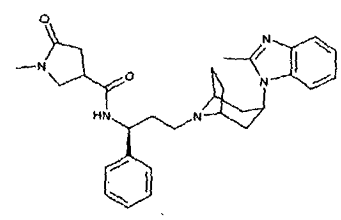

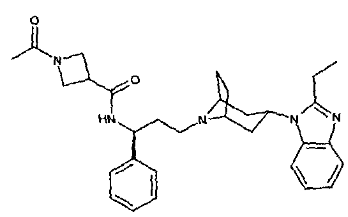

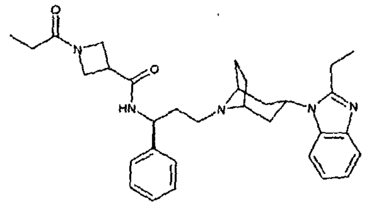

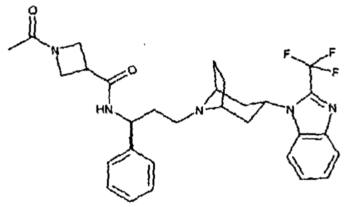

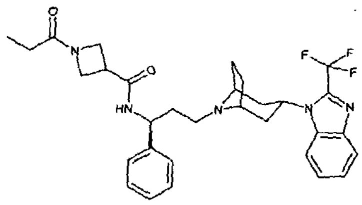

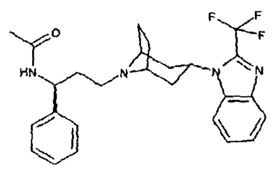

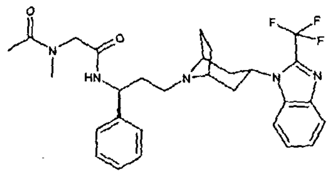

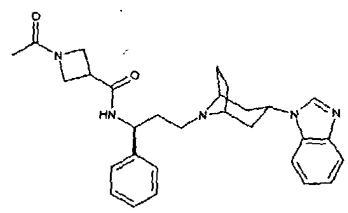

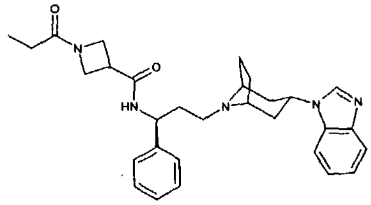

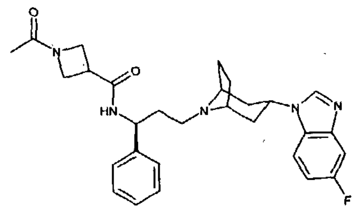

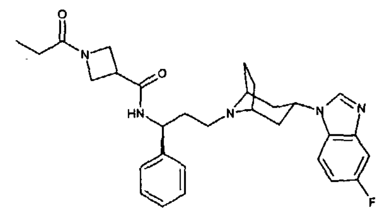

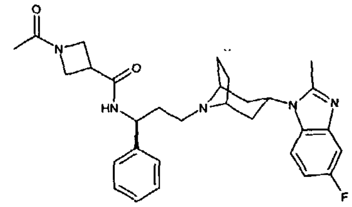

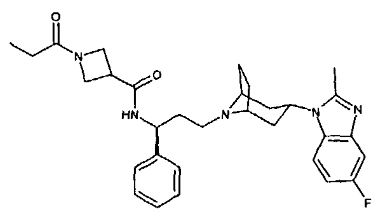

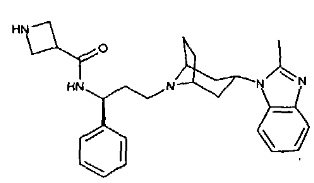

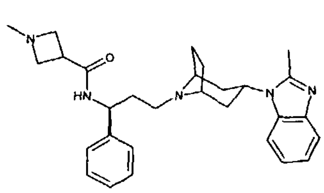

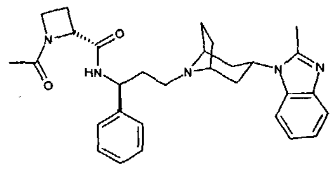

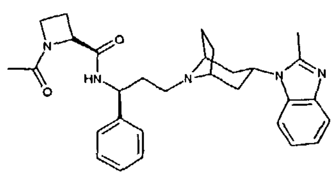

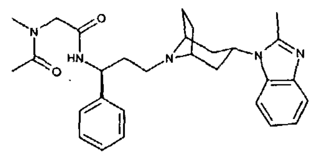

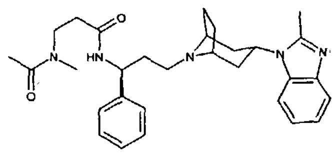

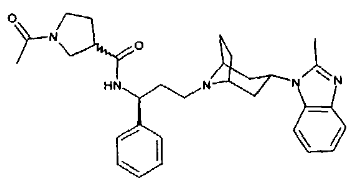

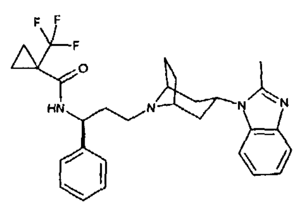

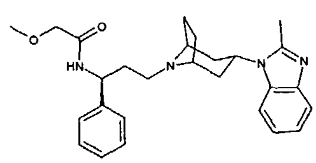

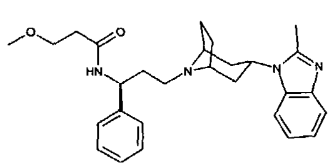

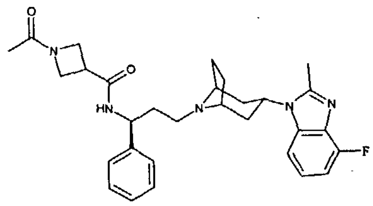

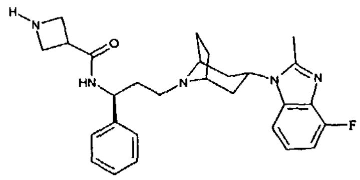

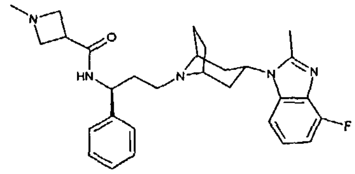

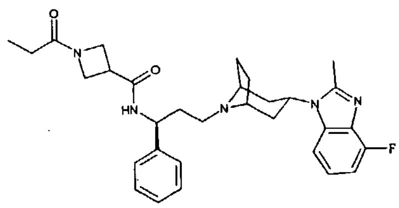

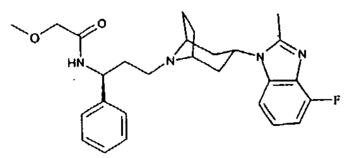

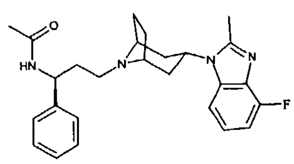

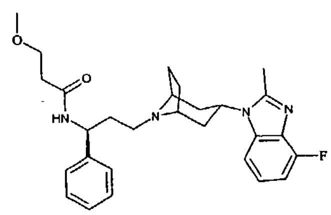

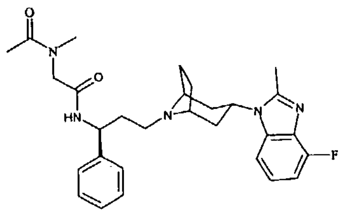

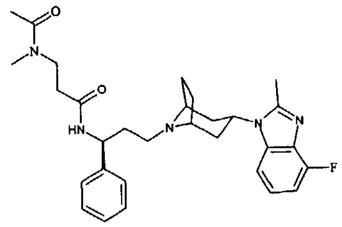

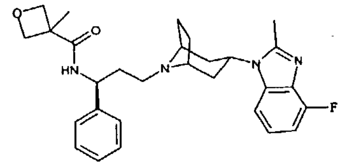

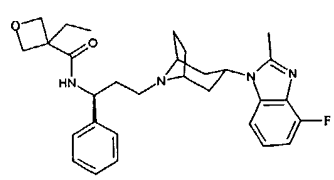

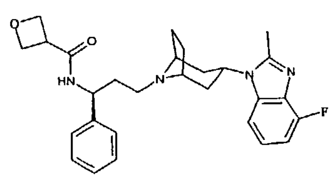

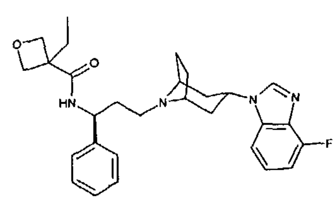

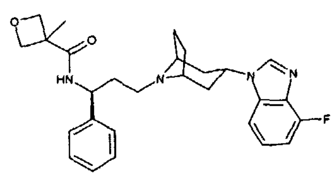

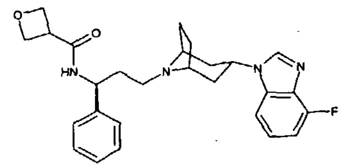

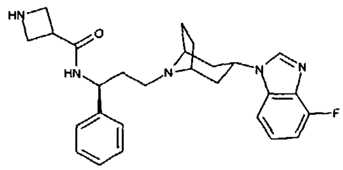

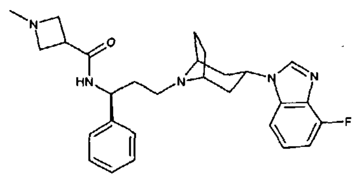

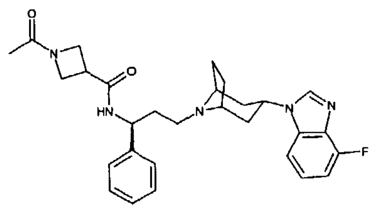

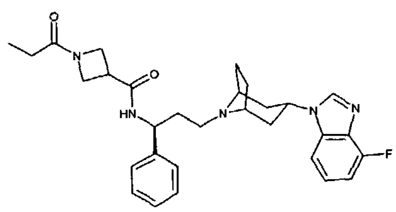

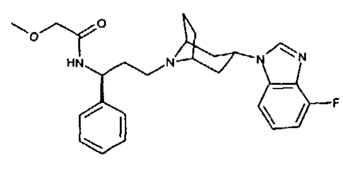

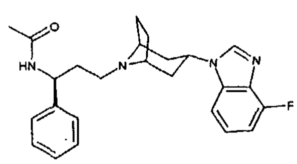

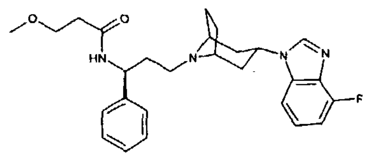

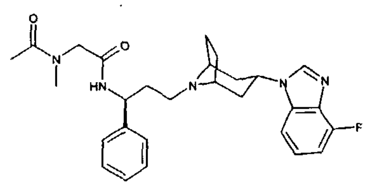

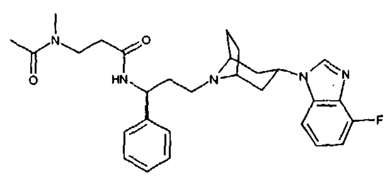

Preferred compounds of WO-A-00/38680 are selected from the group consisting of:

*N*-{3-[3-*exo*-(2-Methyl-1*H*-benzimidazol-1-yl)-8-azabicyclo[3.2.1]oct-8-yl]-1-phenylpropyl}cyclobutanecarboxamide

*N*-{(1*S*)-3-[3-*exo*-(2-Methyl-1*H*-benzimidazol-1-yl)-8-azabicyclo[3.2.1]oct-8-yl]-1-phenylpropyl}cyclobutanecarboxamide

*N*-{(1*S*)-3-[3-*endo*-(2-Methyl-1*H*-benzimidazol-1-yl)-8-azabicyclo[3.2.1]oct-8-yl]-1-phenylpropyl}cyclobutanecarboxamide

*N*-{(1*S*)-3-[3-*exo*-(2-Methyl-1*H*-benzimidazol-1-yl)-8-azabicyclo[3.2.1]oct-8-yl]-1-phenylpropyl}tetrahydro-2*H*-pyran-4-carboxamide

1-Acetyl-*N*-{(1*S*)-3-[3-*exo*-(2-methyl-1*H*-benzimidazol-1-yl)-8-azabicyclo[3.2.1] oct-8-yl]-1-phenyl propyl}-3-azetidine carboxamide

1-Hydroxy-*N*-{(1*S*)-3-[3-*exo*-(2-methyl-1*H*-benzimidazol-1-yl)-8-azabicyclo[3.2.1] oct-8-yl]-1-phenyl propyl}cyclo pentanecarboxamide

2-Methyl-*N*-{(1*S*)-3-[3-*exo*-(2-methyl-1*H*-benzimidazol-1-yl)-8-azabicyclo[3.2.1] oct-8-yl]-1-phenyl propyl}cyclopropanecarboxamide

2-Cyclopropyl-*N*-{(1*S*)-3-[3-*exo*-(2-methyl-1*H*-benzimidazol-1-yl)-8-azabicyclo [3.2.1]oct-8-yl]-1-phenylpropyl} acetamide

*N*-{(1*S*)-3-[3-*exo*-(2-methyl-1*H*-benzimidazol-1-yl)-8-azabicyclo [3.2.1]oct-8-yl]-1-phenylpropyl}tetrahydro-3-furancarboxamide

3,3,3-Trifluoro-*N*-{(1*S*)-3-[3-*exo*-(2-methyl-1*H*-benzimidazol-1-yl)-8-azabicyclo [3.2.1]oct-8-yl]-1-phenylpropyl} propanamide

*N*-{(1*S*)-3-[3-*exo*-(2-Methyl-1*H*-benzimidazol-1-yl)-8-azabicyclo [3.2.1]oct-8-yl]-1-phenylpropyl}tetrahydro-2-furancarboxamide

1-(Acetylamino)-*N*-{(1*S*)-3-[3-*exo*-(2-methyl-1*H*-benzimidazol-1-yl)-8-azabicyclo [3.2.1]oct-8-yl]-1-phenylpropyl} cyclopentanecarboxamide

*N*-{(1*S*)-3-[3-*exo*-(2-Methyl-1*H*-benzimidazol-1-yl)-8-azabicyclo[3.2.1]oct-8-yl]-1-phenylpropyl}acetamide

1-Methoxy-*N*-{(1*S*)-3-[3-*exo*-(2-methyl-1*H*-benzimidazol-1-yl)-8-azabicyclo[3.2.1]oct-8-yl]-1-phenylpropyl} cyclopentanecarboxamide

1-Amino-*N*-{(1*S*)-3-[3-*exo*-(2-methyl-1*H*-benzimidazol-1-yl)-8-azabicyclo[3.2.1]oct-8-yl]-1-phenylpropyl} cyclopentanecarboxamide

1-Methyl-*N*-{(1*S*)-3-[3-*exo*-(2-methyl-1*H*-benzimidazol-1-yl)-8-azabicyclo[3.2.1]oct-8-yl]-1-phenylpropyl}-2-oxo-4-pyrrolidinecarboxamide

1-Acetyl-N-{(1*S*)-3-[3-*endo*-(2-methyl-1*H*-benzimidazol-1-yl)-8-azabicyclo[3.2.1]oct-8-yl]-1-phenylpropyl}-3-azetidinecarboxamide

*N*-{(1*S*)-3-[3-*endo*-(2-Methyl-1*H*-benzimidazol-1-yl)-8-azabicyclo[3.2.1]oct-8-yl]-1-phenylpropyl}acetamide
*N*-{(1*S*)-3-[6-(2-Methyl-1*H*-benzimidazol-1-yl)-3-azabicyclo[3.1.0]hex-3-yl]-1-phenylpropyl}cyclobutanecarboxamide
2-Cyclopropyl-*N*-{(1*S*)-3-[3-*exo*-(3-{4-[(methylsulfonyl)amino]benzyl}-1,2,4-oxadiazol-5-yl)-8-azabicyclo[3.2.1]oct-8-yl]-1-phenylpropyl}acetamide
*N*-{(1*S*)-3-[7-*exo*-(2-Methyl-1*H*-benzimidazol-1-yl)-3-oxa-9-azabicyclo[3.3.1]non-9-yl]-1-phenylpropyl}cyclobutanecarboxamide
2-Cyclopropyl-*N*-{(1*S*)-3-[7-*exo*-(2-methyl-1*H*-benzimidazol-1-yl)-3-oxa-9-azabicyclo[3.3.1]non-9-yl]-1-phenylpropyl}acetamide
3,3,3-Trifluoro-*N*-{(1*S*)-3-[7-*exo*-(2-methyl-1*H*-benzimidazol-1-yl)-3-oxa-9-azabicyclo[3.3.1]non-9-yl]-1-phenylpropyl}propanamide
*N*-{(1*S*)-3-[7-*endo*-(2-Methyl-1*H*-benzimidazol-1-yl)-3-oxa-9-azabicyclo[3.3.1]non-9-yl]-1-phenylpropyl}cyclobutanecarboxamide
2-Cyclopropyl-*N*-{(1*S*)-3-[7-*endo*-(2-methyl-1*H*-benzimidazol-1-yl)-3-oxa-9-azabicyclo[3.3.1]non-9-yl]-1-phenylpropyl}acetamide
*N*-{(1*S*)-3-[7-*exo*-(2-Methyl-1*H*-benzimidazol-1-yl)-3-thia-9-azabicyclo[3.3.1]non-9-yl]-1-phenylpropyl}cyclobutanecarboxamide
2-Cyclopropyl-*N*-[(1*S*)-3-(3-*endo*-{[2-(4-fluorophenyl)acetyl]amino}-8-azabicyclo[3.2.1]oct-8-yl)-1-phenylpropyl]acetamide
*N*-[(1*S*)-3-(3-{[3-*endo*-(4-Fluorophenyl)propanoyl]amino}-8-azabicyclo[3.2.1]oct-8-yl)-1-phenylpropyl]cyclobutanecarboxamide
*N*-[(1*S*)-3-(3-{[3-*exo*-(4-Fluorophenyl)propanoyl]amino}-8-azabicyclo[3.2.1]oct-8-yl)-1-phenylpropyl]cyclobutanecarboxamide
2-Cyclopropyl-*N*-[(1*S*)-3-(3-*exo*-{[2-(4-fluorophenyl)acetyl]amino}-8-azabicyclo[3.2.1]oct-8-yl)-1-phenylpropyl]acetamide
*N*-{(1*S*)-3-[3-*exo*-(2-Methyl-1*H*-benzimidazol-1-yl)-8-azabicyclo[3.2.1]oct-8-y1]-1-phenylpropyl}-1-propionyl-3-azetidinecarboxamide
*N*-{(1*S*)-3-[3-*endo*-(2-Methyl-1*H*-benzimidazol-1-yl)-8-azabicyclo[3.2.1]oct-8-yl]-1-phenylpropyl}tetrahydro-3-furancarboxamide
*N*-{(1*S*)-3-[3-*endo*-(2-Methyl-1*H*-benzimidazol-1-yl)-8-azabicyclo[3.2.1]oct-8-yl]-1-phenylpropyl}tetrahydro-2*H*-pyran-4-carboxamide
*N*-{(1*S*)-3-[3-*endo*-(2-Methyl-1*H*-benzimidazol-1-yl)-8-azabicyclo[3.2.1]oct-8-yl]-1-phenylpropyl}tetrahydro-2-furancarboxamide
1-Acetyl-*N*-{(1*S*)-3-[3-*endo*-(1*H*-benzimidazol-1-yl)-8-azabicyclo[3.2.1]oct-8-yl]-1-phenylpropyl}-3-azetidinecarboxamide
*N*-{(1*S*)-3-[3-*endo*-(1*H*-Benzimidazol-1-yl)-8-azabicyclo[3.2.1]oct-8-yl]-1-phenylpropyl}-1-propionyl-3-azetidinecarboxamide
Methyl 3-[({(1*S*)-3-[3-*exo*-(2-methyl-1*H*-benzimidazol-1-yl)-8-azabicyclo[3.2.1]oct-8-yl]-1-phenylpropyl}amino)carbonyl]-1-azetidinecarboxylate

*N*-{(1*S*)-3-[3-*endo*-(2-Methyl-1*H*-benzimidazol-1-yl)-8-azabicyclo[3.2.1]oct-8-yl]-1-phenylpropyl}-1-propionyl-3-azetidinecarboxamide
1-Acetyl-*N*-{(1*S*)-3-[3-*endo*-(2-methyl-1*H*-benzimidazol-1-yl)-8-azabicyclo[3.2.1]oct-8-yl]-1-phenylpropyl}-2-azetidinecarboxamide
2-[Acetyl(methyl)amino]-*N*-{(1*S*)-3-[3-*endo*-(2-methyl-1*H*-benzimidazol-1-yl)-8-azabicyclo[3.2.1]oct-8-yl]-1-phenylpropyl}acetamide
3-[Acetyl(methyl)amino]-*N*-{(1*S*)-3-[3-*endo*-(2-methyl-1*H*-benzimidazol-1-yl)-8-azabicyclo[3.2.1]oct-8-yl]-1-phenylpropyl}propanamide
2-Methoxy-*N*-{(1*S*)-3-[3-*endo*-(2-methyl-1*H*-benzimidazol-1-yl)-8-azabicyclo[3.2.1]oct-8-yl]-1-phenylpropyl}acetamide
3-Methoxy-*N*-{(1*S*)-3-[3-*endo*-(2-methyl-1*H*-benzimidazol-1-yl)-8-azabicyclo[3.2.1]oct-8-yl]-1-phenylpropyl}propanamide
1-Acetyl-*N*-{(1*S*)-3-[3-*endo*-(2-methyl-1*H*-benzimidazol-1-yl)-8-azabicyclo[3.2.1]oct-8-yl]-1-phenylpropyl}-3-pyrrolidinecarboxamide
1-Methyl-*N*-{(1*S*)-3-[3-*endo*-(2-methyl-1*H*-benzimidazol-1-yl)-8-azabicyclo[3.2.1]oct-8-yl]-1-phenylpropyl}-2-oxo-4-pyrrolidinecarboxamide
1-Acetyl-*N*-{(1*S*)-3-[3-*exo*-(2-ethyl-1*H*-benzimidazol-1-yl)-8-azabicyclo[3.2.1]oct-8-yl]-1-phenylpropyl}-3-azetidinecarboxamide

*N*-{(1*S*)-3-[3-*exo*-(2-Ethyl-1*H*-benzimidazol-1-yl)-8-azabicyclo[3.2.1]oct-8-yl]-1-phenylpropyl}-1-propionyl-3-azetidinecarboxamide
1-Acetyl-*N*-((1*S*)-1-phenyl-3-{3-*exo*-[2-(trifluoromethyl)-1*H*-benzimidazol-1-yl]-8-azabicyclo[3.2.1]oct-8-yl}propyl)-3-azetidinecarboxamide
*N*-((1*S*)-1-Phenyl-3-{3-*exo*-[2-(trifluoromethyl)-1*H*-benzimidazol-1-yl]-8-azabicyclo[3.2.1]oct-8-yl}propyl)-1-propionyl-3-azetidinecarboxamide
*N*-((1*S*)-1-Phenyl-3-{3-*exo*-[2-(trifluoromethyl)-1*H*-benzimidazol-1-yl]-8-azabicyclo[3.2.1]oct-8-yl}propyl)acetamide
2-[Acetyl(methyl)amino]-*N*-((1*S*)-1-phenyl-3-{3-*exo*-[2-(trifluoromethyl)-1*H*-benzimidazol-1-yl]-8-azabicyclo[3.2.1]oct-8-yl}propyl)acetamide
1-Acetyl-*N*-{(1*S*)-3-[3-*exo*-(1*H*-benzimidazol-1-yl)-8-azabicyclo[3.2.1]oct-8-yl]-1-phenylpropyl}-3-azetidinecarboxamide
*N*-{(1*S*)-3-[3-*exo*-(1*H*-Benzimidazol-1-yl)-8-azabicyclo[3.2.1]oct-8-yl]-1-phenylpropyl}-1-propionyl-3-azetidinecarboxamide
1-acetyl-*N*-{(1*S*)-3-[3-*exo*-(5-fluoro-1*H*-benzimidazol-1-yl)-8-azabicyclo[3.2.1]oct-8-yl]-1-phenylpropyl}-3-azetidinecarboxamide
*N*-{(1*S*)-3-[3-*exo*-(5-Fluoro-1*H*-benzimidazol-1-yl)-8-azabicyclo[3.2.1]oct-8-yl]-1-phenylpropyl}-1-propionyl-3-azetidinecarboxamide
1-Acetyl-*N*-{(1*S*)-3-[3-*exo*-(5-fluoro-2-methyl-1*H*-benzimidazol-1-yl)-8-azabicyclo[3.2.1]oct-8-yl]-1-phenylpropyl}-3-azetidinecarboxamide
*N*-{(1*S*)-3-[3-*exo*-(5-Fluoro-2-methyl-1*H*-benzimidazol-1-yl)-8-azabicyclo[3.2.1]oct-8-yl]-1-phenylpropyl}-1-propionyl-3-azetidinecarboxamide
*N*-{(1*S*)-3-[3-*exo*-(2-methyl-1*H*-benzimidazol-1-yl)-8-azabicyclo[3.2.1]oct-8-yl]-1-phenylpropyl}-3-azetidinecarboxamide
1-methyl-*N*-{(1*S*)-3-[3-*exo*-(2-methyl-1*H*-benzimidazol-1-yl)-8-azabicyclo[3.2.1]oct-8-yl]-1-phenylpropyl}-3-azetidinecarboxamide
(2*S*)-1-acetyl-*N*-{(1*S*)-3-[3-*exo*-(2-methyl-1*H*-benzimidazol-1-yl)-8-azabicyclo[3.2.1]oct-8-yl]-1-phenylpropyl}-2-azetidinecarboxamide (2*R*)-1-acetyl-*N*-{(1*S*)-3-[3-*exo*-(2-methyl-1*H*-benzimidazol-1-yl)-8-azabicyclo[3.2.1]oct-8-yl]-1-phenylpropyl}-2-azetidinecarboxamide
2-[acetyl(methyl)amino]-*N*-{(1*S*)-3-[3-*exo*-(2-methyl-1*H*-benzimidazol-1-yl)-8-azabicyclo[3.2.1]oct-8-yl]-1-phenylpropyl}acetamide
3-[acetyl(methyl)amino]-*N*-{(1*S*)-3-[3-*exo*-(2-methyl-1*H*-benzimidazol-1-yl)-8-azabicyclo[3.2.1]oct-8-yl]-1-phenylpropyl}propanamide
1-acetyl-*N*-{(1*S*)-3-[3-*exo*-(2-methyl-1*H*-benzimidazol-1-yl)-8-azabicyclo[3.2.1]oct-8-yl]-1-phenylpropyl}-3-pyrrolidinecarboxamide
*N*-{(1*S*)-3-[3-*exo*-(2-methyl-1*H*-benzimidazol-1-yl)-8-azabicyclo[3.2.1]oct-8-yl]-1-phenylpropyl}-1-(trifluoromethyl)cyclopropanecarboxamide
2-methoxy-*N*-{(1*S*)-3-[3-*exo*-(2-methyl-1*H*-benzimidazol-1-yl)-8-azabicyclo[3.2.1]oct-8-yl]-1-phenylpropyl}acetamide
3-methoxy-*N*-{(1*S*)-3-[3-*exo*-(2-methyl-1*H*-benzimidazol-1-yl)-8-azabicyclo[3.2.1]oct-8-yl]-1-phenylpropyl}propanamide
1-Acetyl-*N*-{(1*S*)-3-[3-*exo*-(4-fluoro-2-methyl-1*H*-benzimidazol-1-yl)-8-azabicyclo[3.2.1]oct-8-yl]-1-phenylpropyl}-3-azetidinecarboxamide
*N*-{(1*S*)-3-[3-*exo*-(4-Fluoro-2-methyl-1*H*-benzimidazol-1-yl)-8-azabicyclo [3.2.1]oct-8-yl]-1-phenylpropyl}-3-azetidinecarboxamide
1-Methyl-*N*-{(1*S*)-3-[3-*exo*-(4-fluoro-2-methyl-1*H*-benzimidazol-1-yl)-8-azabicyclo[3.2.1]oct-8-yl)-1-phenylpropyl}-3-azetidinecarboxamide
*N*-{(1*S*)-3-[3-*exo*-(4-Fluoro-2-methyl-1*H*-benzimidazol-1-yl)-8-azabicyclo[3.2.1]oct-8-yl]-1-phenylpropyl}-1-propionyl-3-azetidinecarboxamide
2-Methoxy-*N*-{(1*S*)-3-[3-*exo*-(4-Fluoro-2-methyl-1*H*-benzimidazol-1-yl)-8-azabicyclo[3.2.1]oct-8-yl]-1-phenylpropyl} acetamide
*N*-{(1*S*)-3-[3-*exo*-(4-Fluoro-2-Methyl-1*H*-benzimidazol-1-yl)-8-azabicyclo[3.2.1)oct-8-yl]-1-phenylpropyl} acetamide
3-Methoxy-*N*-{(1*S*)-3-[3-*exo*-(4-fluoro-2-methyl-1*H*-benzimidazol-1-yl)-8-azabicyclo[3.2.1]oct-8-yl]-1-phenylpropyl}propanamide
2-[Acetyl(methyl)amino]-*N*-{(1*S*)-3-[3-*exo*-(4-fluoro-2-methyl-1*H*-benzimidazol-1-yl)-8-azabicyclo[3.2.1]oct-8-yl]-1-phenylpropyl}acetamide
3-[Acetyl(methyl)amino]-*N*-{(1*S*)-3-[3-*exo*-(4-fluoro-2-methyl-1*H*-benzimidazol-1-yl)-8-azabicyclo[3.2.1]oct-8-yl]-1-phenylpropyl}propanamide
*N*-{(1*S*)-3-[3-*exo*-(4-fluoro-2-methyl-1*H*-benzimidazol-1-yl)-8-azabicyclo[3.2.1]oct-8-yl]-1-phenylpropyl}-3-methyl-3-oxetanecarboxamide
3-Ethyl-*N*-{(1*S*)-3-[3-*exo*-(4-fluoro-2-methyl-1*H*-benzimidazol-1-yl)-8-azabicyclo[3.2.1]oct-8-yl]-1-phenylpropyl}-3-oxetanecarboxamide
*N*-{(1*S*)-3-[3-*exo*-(4-Fluoro-2-methyl-1*H*-benzimidazol-1-yl)-8-azabicyclo[3.2.1]oct-8-yl]-1-phenylpropyl}-3-oxetanecarboxamide
3-Ethyl-*N*-{(1*S*)-3-[3-*exo*-(4-fluoro-1*H*-benzimidazol-1-yl)-8-azabicyclo[3.2.1]oct-8-yl]-1-phenylpropyl}-3-oxetanecarboxamide
*N*-{(1*S*)-3-[3-*exo*-(4-Fluoro-1*H*-benzimidazol-1-yl)-8-azabicyclo[3.2.1]oct-8-yl]-1-phenylpropyl}-3-methyl-3-oxetanecarboxamide
*N*-{(1*S*)-3-[3-*exo*-(4-Fluoro-1*H*-benzimidazol-1-yl)-8-azabicyclo[3.2.1]oct-8-yl]-1-phenylpropyl}-3-oxetanecarboxamide
*N*-{(1*S*)-3-[3-*exo*-(4-Fluoro-1*H*-benzimidazol-1-yl)-8-azabicyclo[3.2.1]oct-8-yl]-1-phenylpropyl}-3-azetidinecarboxamide
*N*-{(1*S*)-3-[3-*exo*-(4-Fluoro-1*H*-benzimidazol-1-yl)-8-azabicyclo[3.2.1]oct-8-yl]-1-phenylpropyl}-1-methyl-3-azetidinecarboxamide
1-Acetyl-*N*-{(1*S*)-3-[3-*exo*-(4-fluoro-1*H*-benzimidazol-1-yl)-8-azabicyclo[3.2.1]oct-8-yl]-1-phenylpropyl}-3-azetidinecarboxamide
*N*-{(1*S*)-3-[3-*exo*-(4-Fluoro-1*H*-benzimidazol-1-yl)-8-azabicyclo[3.2.1]oct-8-yl]-1-phenylpropyl}-1-propionyl-3-azetidinecarboxamide
*N*-{(1*S*)-3-[3-*exo*-(4-Fluoro-1*H*-benzimidazol-1-yl)-8-azabicyclo[3.2.1]oct-8-yl]-1-phenylpropyl}-2-methoxyacetamide
*N*-{(1*S*)-3-[3-*exo*-(4-Fluoro-1*H*-benzimidazol-1-yl)-8-azabicyclo[3.2.1]oct-8-yl]-1-phenylpropyl}acetamide
*N*-{(1*S*)-3-[3-*exo*-(4-fluoro-1*H*-benzimidazol-1-yl)-8-azabicyclo[3.2.1]oct-8-yl]-1-phenylpropyl}-3-methoxypropanamide
2-[Acetyl(methyl)amino]-*N*-{(1*S*)-3-[3-*exo*-(4-fluoro-1*H*-benzimidazol-1-yl)-8-azabicyclo[3.2.1]oct-8-yl]-1-phenylpropyl}acetamide
3-[Acetyl(methyl)amino]-*N*-{(1*S*)-3-[3-*exo*-(4-fluoro-1*H*-benzimidazol-1-yl)-8-azabicyclo[3.2.1]oct-8-yl]-1-phenylpropyl}propanamide

Examples of agents may be the compounds disclosed in WO-A-00/39125 (claiming priority from *inter alia* GB patent application No. 9922009.7 filed 18 September 1999) - the contents of which are incorporated herein by reference.
The compounds of WO-A-00/39125 may be of the Formula (I):

$$[R_{egion}\ \alpha] - [R_{egion}\ \beta] - [R_{egion}\ \gamma] - [R_{egion}\ \delta] \qquad (I)$$

wherein:
**[$R_{egion}$ α]** is selected from the group consisting of:

- A. Aryl heterocyclyl substituent components comprising:

  -- 1. hetero-phenylmethylene moieties of partial Formula (1.0.0):

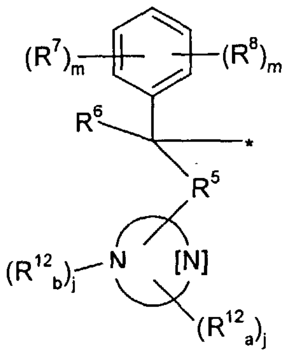

(1.0.0)

--- wherein: the symbol "*" indicates the point of attachment of the moiety of partial Formula (1.0.0) to $R_{egion}$ β, as hereinafter defined;

--- $R^5$ is a member selected from the group consisting of a direct bond; -O-; -C(=O)-; -$NR^4$-; and -$S(=O)_p$-; where:

---- $R^4$ is hydrogen or $(C_1\text{-}C_2)$alkyl;

--- $R^6$ is a member selected from the group consisting of hydrogen; $(C_1\text{-}C_2)$alkyl; $(C_1\text{-}C_2)$alkoxy; -CN; -OH; and -$C(=O)NH_2$;

--- j is an integer selected from 0, 1, and 2;

--- m is an integer selected from 0, 1, and 2;

--- $R^7$ and $R^8$ are each a member selected from the group consisting of -F; -Cl; -$CO_2R^4$; -OH; -CN; -$CONR^4_aR^4_b$; -$NR^4_aR^4_b$-; -$NR^4_aC(=O)R^4_b$; -$NR^4_aC(=O)OR^4_b$; -$NR^4_aS(=O)_pR^4_b$; -$S(=O)_pNR^4_aR^4_b$; $(C_1\text{-}C_4)$alkyl, and $(C_1\text{-}C_4)$alkoxy wherein said alkyl and alkoxy are each substituted with 0 to 3 substituents independently selected from F and Cl; $(C_1\text{-}C_2)$alkoxycarbonyl; $(C_1\text{-}C_2)$alkylcarbonyl; and $(C_1\text{-}C_2)$alkylcarbonyloxy; where:

---- p is an integer selected from 0, 1, and 2;
---- $R^4_a$ and $R^4_b$are each independently selected from hydrogen and $(C_1\text{-}C_2)$alkyl;

--- the moiety represented by partial Formula (1.0.1):

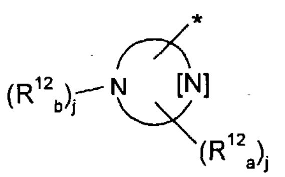

(1.0.1)

in partial Formula (1.0.0) represents a monocyclic heterocyclic group, or a bicyclic benzo-fused ring system containing said heterocyclic group wherein said heterocyclic group contains a total of 5- or 6- members of which one or two of said members is nitrogen, the presence of the optional second nitrogen atom being represented by: "[N]"; wherein said heterocyclic group or ring system are selected from the group consisting of pyrrolyl; pyrazolyl; imidazolyl; pyridinyl; pyrazinyl; pyrimidinyl; pyridazinyl; piperazinyl; indolyl; indazolinyl; benzimidazolyl; quinolinyl; *iso*-quinolinyl; and quinazolinyl; wherein:

---- $R^{12}_a$ is a member selected from the group consisting of hydrogen; F; Cl; -$CO_2R^4$; oxo; -OH; CN; $NH_2$;

$NH(C_1-C_2)alkyl$; $N(C_1-C_2)_2dialkyl$; $-CF_3$; $(C_1-C_4)alkyl$; $(C_2-C_4)alkenyl$; $(C_1-C_4)alkoxy$; $(C_3-C_7)$cycloalkyl; and phenyl; wherein said alkyl, alkenyl, alkoxy, cycloalkyl and phenyl are substituted with 0 to 2 substituents $R^9$ where:

----- $R^9$ is a member independently selected from the group consisting of F; Cl; $-CO_2R^4$; -OH; cyano; $-CONR^4_aR^4_b$; $-NR^4_aR^4_b$-; $-NR^4_aC(=O)R^4_b$; $-NR^4_aC(=O)OR^4_b$; $-NR^4_aS(=O)_pR^4_b$; $-S(=O)_pNR^4_aR^4_b$; $(C_1-C_4)$alkyl including dimethyl, and $(C_1-C_4)$alkoxy wherein said alkyl and alkoxy are each independently substituted with 0 to 3 substituents independently selected from F and Cl; $(C_1-C_2)$alkoxycarbonyl; $(C_1-C_2)$alkylcarbonyl; and $(C_1-C_2)$alkylcarbonyloxy; and

---- $R^{12}_b$ is absent or is a member selected from the group consisting of hydrogen; $(C_1-C_4)$alkyl; $(C_2-C_4)$alkenyl; $(C_1-C_2)$alkoxy; $(C_3-C_7)$cycloalkyl; and phenyl; wherein said alkyl, alkenyl, alkoxy, cycloalkyl and phenyl are substituted with 0 to 2 substituents $R^9$ wherein $R^9$ has the same meaning as above, except that it is selected independently selected therefrom; and

2. hetero-phenylmethylene moieties of partial Formula (1.1.0):

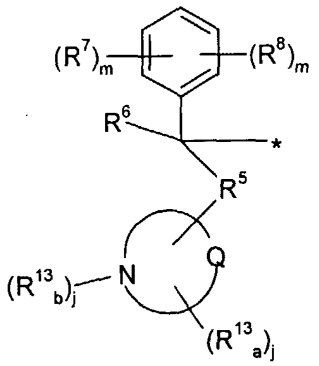

(1.1.0)

--- wherein: the symbol "*"; $R^5$; $R^6$; $R^7$; $R^8$; j and m are as defined further above, except that all of the above-recited substituents are selected independently of their selection above;

--- the moiety represented by partial Formula (1.1.1):

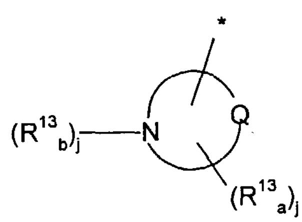

(1.1.1)

in partial Formula (1.1.0) represents:

---- a. a monocyclic heterocyclic group containing a total of 5 or 6 members of which one said member is nitrogen and Q is selected from O and S where said S may optionally be in the sulfonate form, $-S(=O)_2$; wherein said heterocyclic group is selected from the group consisting of oxazolyl; oxazolidinyl; *iso*xazolyl; thiazolyl; thiazolidinyl; *iso*-thiazolyl; morpholinyl; and thiomorpholinyl; or

---- b. a monocyclic heterocyclic group containing a total of 5- or 6- member s of which two said members are nitrogen and a third or fourth said member is independently selected from N, O, and S where said S may optionally be in the sulfonate form, $-S(=O)_2$; wherein said heterocyclic group is selected from the group consisting of triazolyl; triazinyl; tetrazolyl; oxadiazolyl; thiadiazolyl; and

---- $R^{13}_a$ is selected from the group consisting of hydrogen; F; Cl; $-CO_2R^4$; oxo; -OH; CN; $NH_2$; NH($C_1$-

$C_2$)alkyl; $N(C_1-C_2)_2$dialkyl; $-CF_3$; $(C_1-C_4)$alkyl; $(C_2-C_4)$alkenyl; $(C_1-C_2)$alkoxy; $(C_3-C_7)$cycloalkyl; and phenyl; wherein said alkyl, alkenyl, alkoxy, cycloalkyl and phenyl are substituted with 0 to 2 substituents $R^{11}$ where:

----- $R^{11}$ is a member selected from the group consisting of F; Cl; $-CO_2R^4$; -OH; -CN; $-CONR^4_aR^4_b$; $-NR^4_aR^4_b$; $-NR^4_aC(=O)R^4_b$; $-NR^4_aC(=O)OR^4_b$; $-NR^4_aS(=O)_pR^4_b$; $-S(=O)_pNR^4_aR^4_b$; $(C_1-C_4)$alkyl including dimethyl, and $(C_1-C_4)$alkoxy wherein said alkyl and alkoxy are each independently substituted with 0 to 3 substituents independently selected from F and Cl; $(C_1-C_2)$alkoxycarbonyl; $(C_1-C_2)$alkylcarbonyl; and $(C_1-C_2)$alkylcarbonyloxy; and

---- $R^{13}_b$ is a member selected from the group consisting of hydrogen; $(C_1-C_4)$alkyl; $(C_2-C_4)$alkenyl; $(C_1-C_2)$ alkoxy; $(C_3-C_7)$cycloalkyl; $C(=O)(C_1-C_4)$alkyl; $S(=O)_2(C_1-C_4)$alkyl; and phenyl; wherein said alkyl, alkenyl, alkoxy, cycloalkyl and phenyl are substituted with 0 to 2 substituents $R^{11}$ wherein $R^{11}$ has the same meaning as in above, except that it is selected independently;

- B. a (substituted)-amido-aryl or -heterocyclyl moiety selected from the group consisting of

-- 1. alkyl-, alkenyl-, and alkynyl-substituted-amido-aryl moieties of partial Formula (2.0.0):

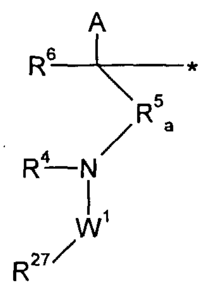

(2.0.0)

--- wherein: the symbol "*"; $R^4$ and $R^6$; are as defined above, except that all of the above-recited substituents are selected independently of their selection above;

--- A is a member selected from the group consisting of:

---- 1. the moiety of partial Formula (2.0.3)

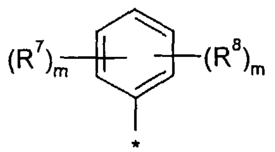

(2.0.3)

----- wherein: the symbol $R^7$; $R^8$ and m are as defined above, except that all of the above-recited substituents are selected independently of their selection above; and the symbol: "*" indicates the point of attachment of the moiety A to the, remaining portions of partial Formula (2.0.0);

---- 2. the moiety of partial Formula (2.0.4)

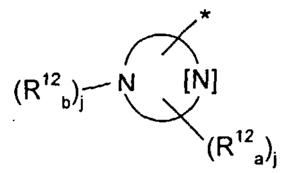

(2.0.4)

which represents a monocyclic heterocyclic group, selected from the group consisting of pyrrolyl; pyrazolyl; imidazolyl; pyridinyl; pyrazinyl; pyrimidinyl; wherein: the symbol $R^{12}_a$ and $R^{12}_b$ are as defined above, except that all of the above-recited substituents are selected independently of their selection above; and the symbol: "*" indicates the point of attachment of the moiety A to the other, remaining portions of partial Formula (2.0.0);

---- 3. the moiety of partial Formula (2.0.5)

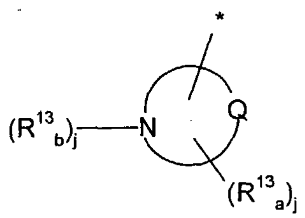

(2.0.5)

which represents

----- a. a monocyclic heteroaromatic group containing a total of 5- members of which one said member is nitrogen and Q is selected from O and S where said S may optionally be in the sulfonate form, $-S(=O)_2$; selected from the group consisting of oxazolyl; isoxazolyl; thiazolyl; and iso-thiazolyl; or

----- b. a monocyclic heterocyclic group containing a total of 5- or 6- members of which two said members are nitrogen and a third or fourth said member is independently selected from N, O, and S where said S may optionally be in the sulfonate form, $-S(=O)_2$; selected from the group consisting of triazolyl; triazinyl; tetrazolyl; oxadiazolyl; and thiadiazolyl; and

------wherein: the $R^{13}_a$, $R^{13}_b$ and j are as defined above, except that all of the above-recited substituents are selected independently of their selection above; and the symbol: "*" indicates the point of attachment of the moiety A to the other, remaining portions of partial Formula (2.0.2);

--- $R^5_a$ is a member selected from the group consisting of a direct bond; -C(=O)-; and $-S(=O)_2-$;

--- $W^1$ is (1.) a direct bond; (2.) in the case where $R^5_a$ is -C(=O)- or $-S(=O)_2$, $W^1$ is a direct bond or $-(C_1-C_3)$ alkylene- wherein any single carbon atom thereof is substituted by 0 to 2 substituents $R^{23}$ where $R^{23}$ is a member selected from the group consisting of -F; -Cl; $-CO_2R^4$; -OH; -CN; $(C_1-C_4)$alkoxy; $(C_3-C_7)$cycloalkyl; and phenyl; wherein said alkoxy, cycloalkyl, and phenyl are substituted with 0 to 2 substituents $R^{11}$, wherein said $R^{11}$ is as defined above, except that all of the above-recited substituents are selected independently of their selection above; or (3.) is a member independently selected from the group consisting of the moieties of partial Formulas (2.0.6) through (2.0.16), inclusive:

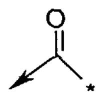 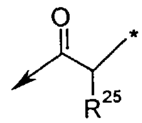 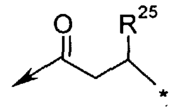

(2.0.6) (2.0.7) (2.0.8)

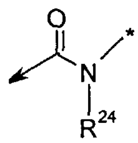 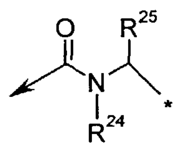 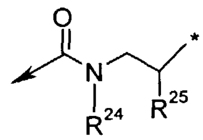

(2.0.9) (2.0.10) (2.0.11)

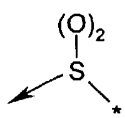 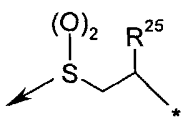 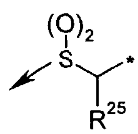 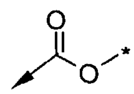 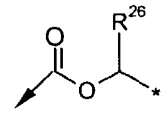

(2.0.12)

(2.0.13) (2.0.14) (2.0.15) (2.0.16)

---- wherein: the symbol: "→" indicates the point of attachment of the moiety $W^1$ to the nitrogen atom in partial Formula (2.0.0), and the symbol: "*" indicates the point of attachment of the moiety $W^1$ to the other, remaining portions of partial Formula (2.0.0); and $R^4$ is as defined further above, but selected on an independent basis;

--------$R^{24}$ is selected from the group consisting of hydrogen and $(C_1\text{-}C_4)$alkyl; and

---- $R^{25}$ and $R^{26}$are each selected from the group consisting of -OH; $(C_1\text{-}C_2)$alkyl substituted by 0 to 3 substituents selected from F; and OH; and $(C_1\text{-}C_2)$alkoxy; and

--- $R^{27}$ is selected from the group consisting of$(C_1\text{-}C_6)$alkyl; $(C_2\text{-}C_6)$alkenyl; and $(C_2\text{-}C_6)$alkynyl; wherein said alkyl, alkenyl, and alkynyl groups comprising $R^{27}$ are substituted with 0 to 3 substituents $R^{28}$ where:

---- $R^{28}$ is selected from the group consisting of phenyl; F or Cl; oxo; hydroxy; $(C_1\text{-}C_2)$alkyl; $(C_1\text{-}C_3)$alkoxy; $-C(=O)OR^{29}$; $-C(=O)(C_1\text{-}C_4)$alkyl; $-S(=O)_2(C_1\text{-}C_4)$alkyl; $-C(=O)NR^{29}R^{30}$; $-NR^{29}R^{30}$; $-NR^{29}C(=O)R^{30}$; $-NR^{29}C(=O)OR^{30}$; $-NR^{29}S(=O)_pR^{30}$; and $-S(=O)_2NR^{29}R^{30}$, where:

----- $R^{29}$ and $R^{30}$ are each a member independently selected from the group consisting of hydrogen and $(C_1\text{-}C_4)$alkyl substituted by 0 to 3 substituents selected from the group consisting of F and Cl;

-- 2. cycloalkyl-substituted-amido-aryl moieties of partial Formula (2.1.0):

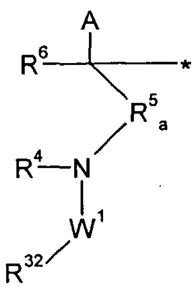

(2.1.0)

--- wherein: A; $W^1$; the symbol "*"; $R^4$; $R^5_a$; and $R^6$ have the same meaning as set out above, except that all of the above-recited substituents are selected independently of their selection above; and

--- $R^{32}$ is a member selected from the group consisting of $-(CH_2)_n-(C_3-C_7)$cycloalkyl, where n is an integer selected from 0, 1, and 2; in the event n is 0, then the α-carbon atom of said $(C_3-C_7)$cycloalkyl is substituted by 0 or 1 $(C_1-C_4)$alkyl or phenyl, where said alkyl or phenyl are substituted by 0, 1, or 2 of $CH_3$, $OCH_3$, OH or $NH_2$; and in the event that n is 1 or 2, the resulting methylene or ethylene is substituted by 0 or 1 of F; $NH_2$; $N(CH_3)_2$; OH; $OCH_3$; $(C_1-C_4)$alkyl; or phenyl; where said alkyl and phenyl are substituted by 0, 1, or 2 of $CH_3$, $OCH_3$, OH, and $NH_2$; and further wherein said $(C_3-C_7)$cycloalkyl is substituted by 0 to 3 substituents $R^{28}$ where $R^{28}$ is as defined further above, but selected independently

-- 3. aryl and heterocyclic-substituted-amido-aryl moieties of partial Formula (2.2.0):

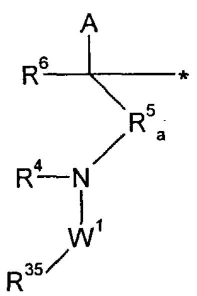

(2.2.0)

--- wherein: A; $W^1$; the symbol: "*"; $R^4$; $R^5_a$; and $R^6$ have the same meaning as set out above, except that all of the above-recited substituents are selected independently of their selection above; and

--- $R^{35}$ is selected from the group consisting of phenyl; furyl; tetrahydrofuranyl; tetrahydropyranyl; oxetanyl; thienyl; pyrrolyl; pyrrolidinyl; oxazolyl; isoxazolyl; thiazolyl; isothiazolyl; imidazolyl; pyrazolyl; oxadiazolyl; thiadiazolyl; triazolyl; pyridyl; pyrazinyl; pyridazinyl; piperazinyl; pyrimidinyl; pyranyl; azetidinyl; morpholinyl; parathiazinyl; indolyl; indolinyl; benzo[*b*]furanyl; 2;3-dihydrobenzofuranyl; benzothienyl; 1H-indazolyl; benzimidazolyl; benzoxazolyl; benzisoxazolyl; benzthiazolyl; quinolinyl; isoquinolinyl; phthalazinyl; quinazolinyl; and quinoxalinyl; wherein (1.) said group $R^{35}$ may be substituted upon any one or more carbon atoms thereof by 0 to 3 substituents $R^{28}$ where $R^{28}$ is as defined above, except that it is selected independently; (2.) said group $R^{35}$ is substituted with respect to any one or more nitrogen atoms thereof that is not a point of attachment of said aryl or heterocyclic moiety, by 0 to 3 substituents $R^{13}_b$ where $R^{13}_b$ is as defined above, except that it is selected independently; and (3.) said group $R^{35}$ with respect to any sulfur atom thereof that is not a point of attachment of said heterocyclic moiety, is substituted by 0 or 2 oxygen atoms;

**[$R_{egion}$ β]** is an alkyl bridging element of partial Formula (3.0.0):

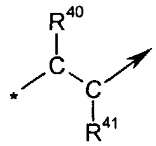

(3.0.0)

wherein:

- "*" is a symbol which represents the point of attachment of the moiety of partial Formula (3.0.0) to $R_{egion}$ α;
- "→" is a symbol which represents the point of attachment of the moiety of partial Formula (3.0.0) to $R_{egion}$ γ;
- $R^{40}$ and $R^{41}$ are both selected from the group consisting of hydrogen; ($C_1$-$C_2$) alkyl including dimethyl; hydroxy; and ($C_1$-$C_3$) alkoxy;

**[$R_{egion}$ γ]** is an aza-monocyclic moiety of partial Formula (4.0.0):

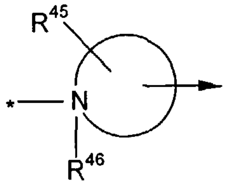

(4.0.0)

- wherein:
- "*" is a symbol which represents the point of attachment of the moiety of partial Formula (4.0.0) to $R_{egion}$ β of the compound of Formula (I);
- "*→*" is a symbol representing a covalent bond attaching any carbon atom of said aza-monocyclic moiety of partial Formula (4.0.0) to $R_{egion}$ δ;
- the moiety of partial Formula (4.0.1):

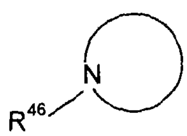

(4.0.1)

in partial Formula (4.0.0) represents a monocyclic heterocyclic group containing a total of from 4- to 7-members of which one said member is nitrogen, wherein said heterocyclic group is a member independently selected from the group consisting essentially of azetidinyl; pyrrolidinyl; piperidinyl; and azepinyl;

- $R^{45}$ is absent or is a member independently selected from the group consisting essentially of ($C_1$-$C_4$)alkyl including dimethyl; ($C_3$-$C_6$)cycloalkyl; ($C_1$-$C_4$)alkoxy; ($C_1$-$C_2$)alkoxy(C1-C2)alkyl; $CF_3$; $-CO_2R^4$ where $R^4$ is as defined further above; oxo; -OH; cyano; $-C(=O)NR^4_aR^4_b$; $-NR^4_aR^4_b$; $-NR^4_aC(=O)R^4_b$; $-NR^4_aC(=O)OR^4_b$; $-NR^4_aS(=O)_pR^4_b$; $-S(=O)_pNR^4_aR^4_b$; ($C_1$-$C_2$)alkoxycarbonyl; ($C_1$-$C_2$)alkylcarbonyl; ($C_1$-$C_2$)alkylcarbonyloxy; and ($C_1$-$C_2$)alkoxy($C_1$-$C_2$)alkyl; it being understood that in the moiety of partial Formula (4.0.0) $R^{45}$ is a substituent attached to a single carbon atom thereof; where:
  - $R^4_a$ and $R^4_b$ are each independently selected from hydrogen and ($C_1$-$C_2$)alkyl;

-- $R^{46}$ is absent or is a member independently selected from the group consisting essentially of hydrogen; and $(C_{1-}C_4)$alkyl substituted by 0 or 1 substituent independently selected from $(C_{1-}C_2)$alkoxy and $-CO_2R^4$ where $R^4$ is as defined further above; and tO; it being understood that in the case where substituent $R^{46}$ is chosen to be other than absent, that it results in said nitrogen atom and said moiety of partial Formula (4.0.0) being in quaternary form;

**[$R_{egion}$ δ]** is a (substituted)-heterocyclyl moiety selected from the group consisting of:

-- 1. a heterocyclyl moiety of partial Formula (5.3.0):

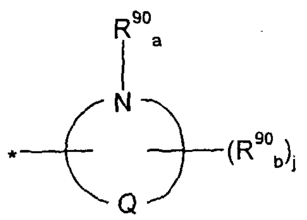

(5.3.0):

-- wherein: the symbol: " * " indicates the point of attachment of partial Formula (5.3.0) to $R_{egion}$ γ; Q is N, O or S and

-- partial Formula (5.3.0) represents:

--- a. a monocyclic heterocyclic group containing a total of 5- members of which one said member is nitrogen and a second said member is selected from O and S where said S may optionally be in the sulfonate form, wherein said heterocyclic group is selected from the group consisting of oxazolyl; *iso*xazolyl; thiazolyl; and *iso*-thiazolyl; or

---- b. a monocyclic heterocyclic group containing a total of 5- members of which two said members are nitrogen and a third or fourth said member is independently selected from N, O, and S where said S may optionally be in the sulfonate form, $-S(=O)_2$; wherein said heterocyclic group is independently selected from the group consisting of triazolyl; triazinyl; tetrazolyl; oxadiazolyl; and thiadiazolyl; and

-- $R^{90}_a$ and $R^{90}_b$ are each a member independently selected from the group consisting of hydrogen, $-(C_{1-}C_2)$alkylcarbonyl; $-(C_{1-}C_4)$alkyl; $-(CH_2)_{n-}(C_{3-}C_7)$cycloalkyl; $-(C_{2-}C_3)$alkenyl; $-(CH_2)_{n-}$(phenyl); and $-(CH_2)_{n-}(HET_1)$, where n is an integer independently selected from 0, 1, and 2; wherein said $(C_{1-}C_4)$alkyl, alkenyl, cycloalkyl, phenyl, and $HET_1$ groups are independently substituted with 0 to 3 substituents $R^{91}$, where:

--- j has the same meaning as set forth above, but is selected on an independent basis therefrom;

--- $HET_1$ is a heterocyclyl group selected from the group consisting of thienyl; oxazolyl; isoxazolyl; thiazolyl; isothiazolyl; pyrazolyl; oxadiazolyl; thiadiazolyl; triazolyl; pyridyl; pyrazinyl; pyridazinyl; pyrimidinyl; parathiazinyl; and morpholinyl; where:

---- $R^{91}$ is selected from the group consisting of -F; -Cl; $-CO_2R^4$; -oxo; -OH; -CN; $-CONR^{93}R^{94}$; $-NR^{93}R^{94}$; $C(=O)(C_1-C_4)$alkyl; $-NR^{93}C(=O)R^{94}$; $-NR^{93}C(=O)OR^{94}$; $-NR^{93}S(=O)R^{94}$; $-S(=O)NR^{93}R^{94}$; $(C_{1-}C_4)$alkyl, and $(C_{1-}C_4)$alkoxy wherein said alkyl and alkoxy are each independently substituted with 0 to 3 substituents independently selected from F and Cl; $(C_{1-}C_2)$alkoxycarbonyl; $(C_{1-}C_2)$alkylcarbonyl; and $(C_{1-}C_2)$alkylcarbonyloxy; wherein:

----- $R^{93}$and $R^{94}$ are each a member independently selected from the group consisting of hydrogen; and $(C_{1-}C_2)$alkyl; and

-- 2. a heterocyclyl moiety of partial Formula (5.4.0):

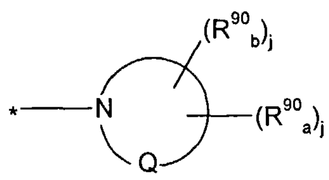

(5.4.0):

--- wherein: $R^{90}_a$; $R^{90}_b$; and j have the same meanings as set out above, but are selected independently.

Preferred compounds of WO-A-00/39125 may be selected from the group consisting of:

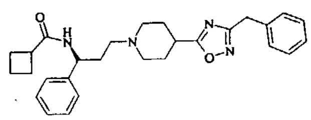

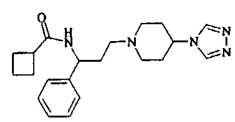

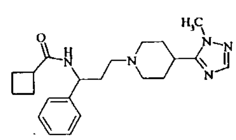

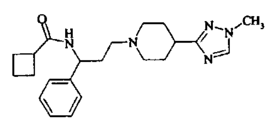

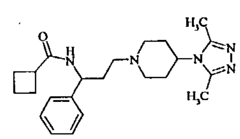

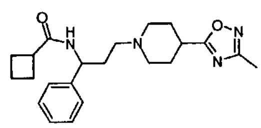

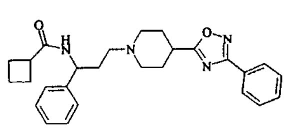

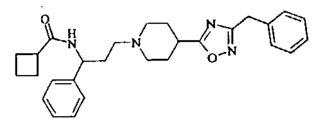

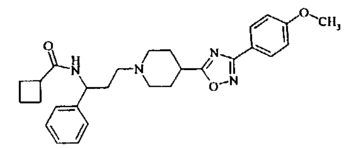

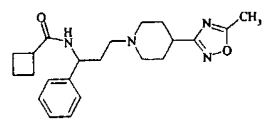

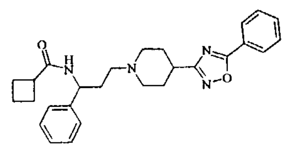

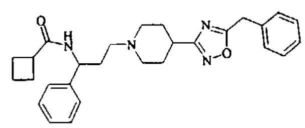

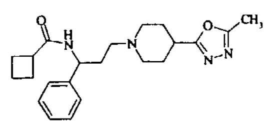

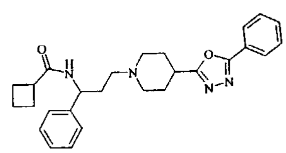

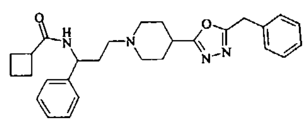

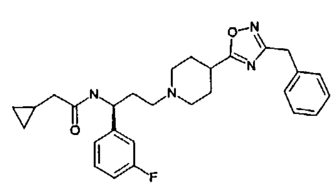

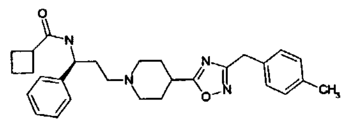

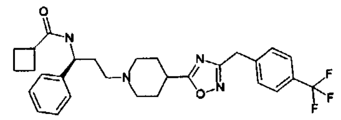

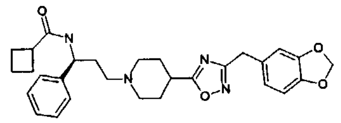

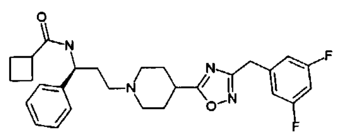

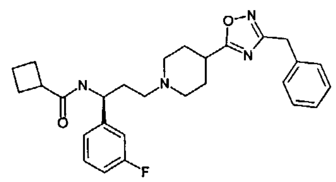

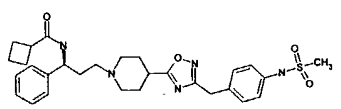

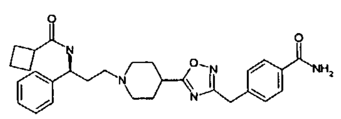

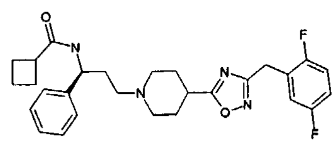

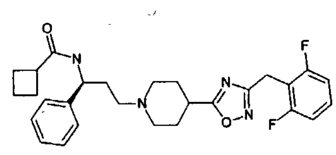

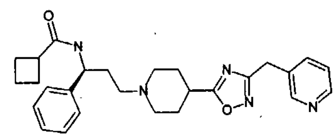

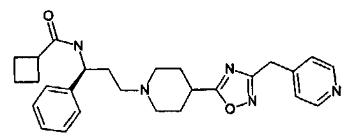

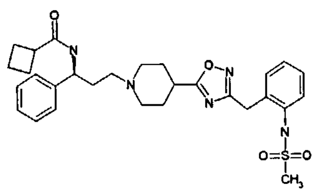

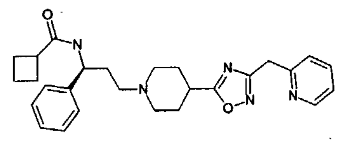

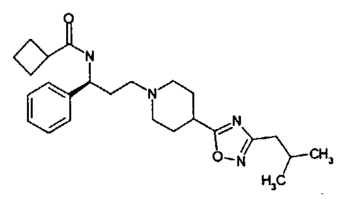

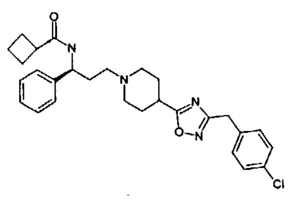

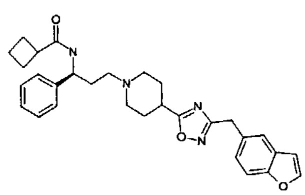

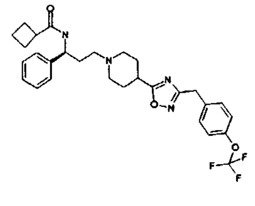

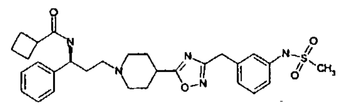

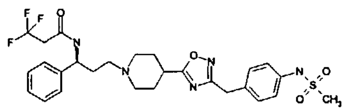

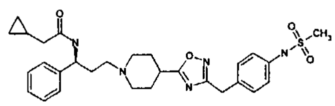

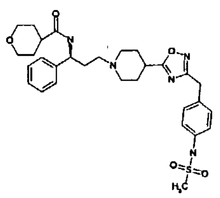

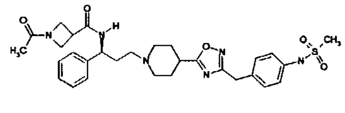

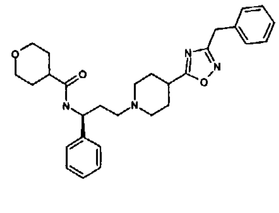

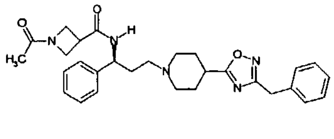

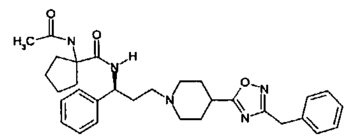

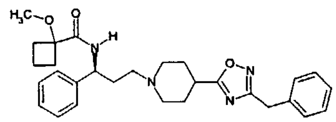

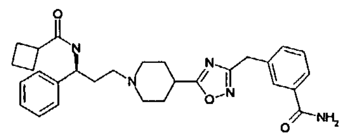

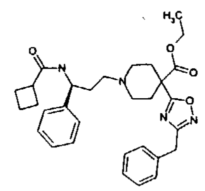

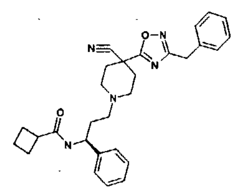

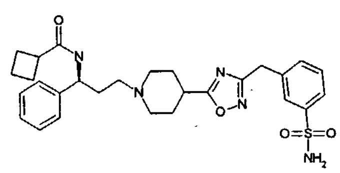

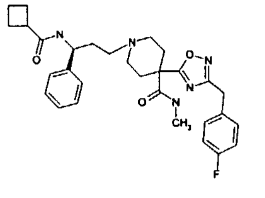

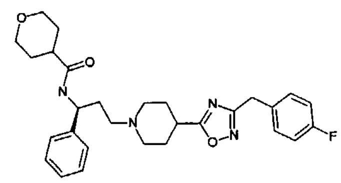

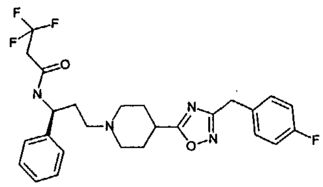

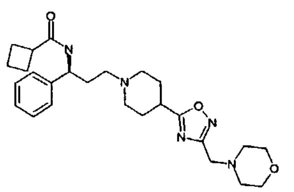

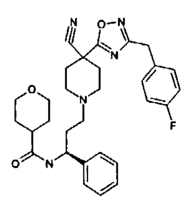

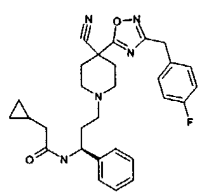

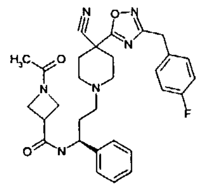

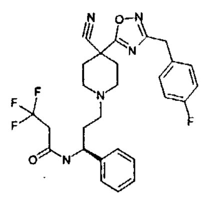

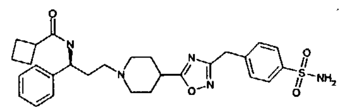

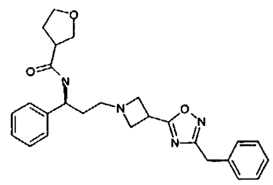

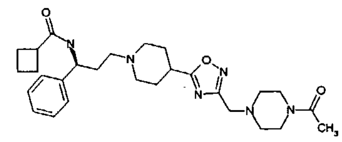

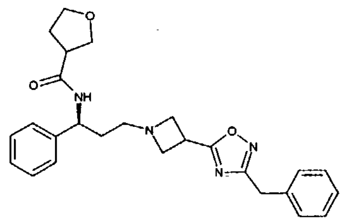

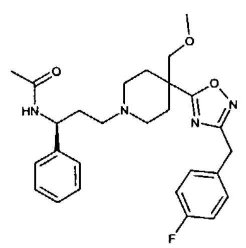

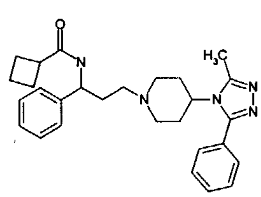

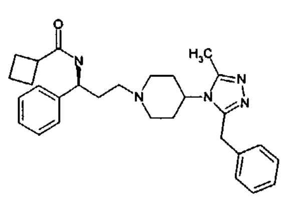

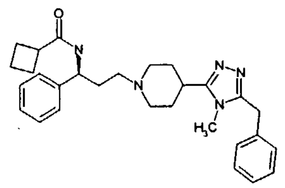

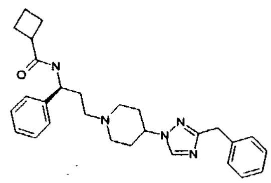

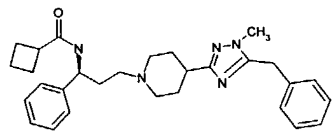

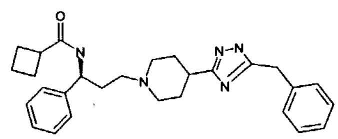

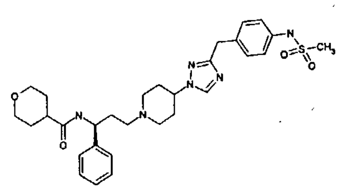

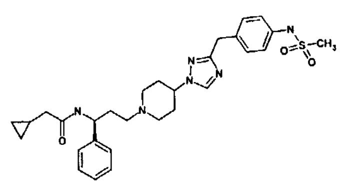

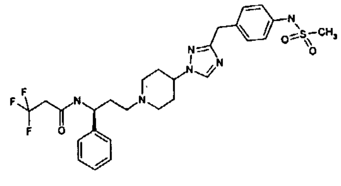

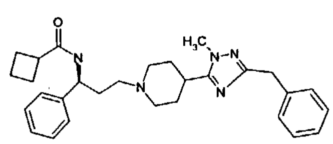

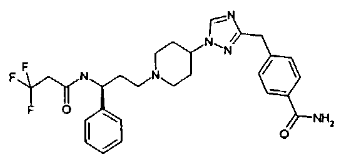

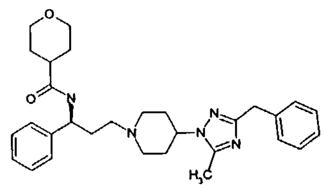

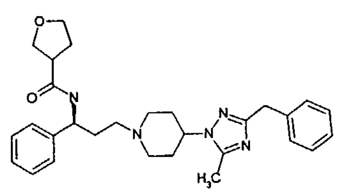

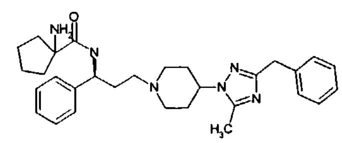

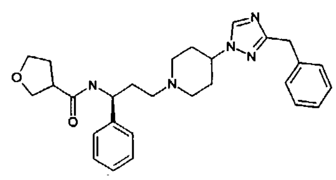

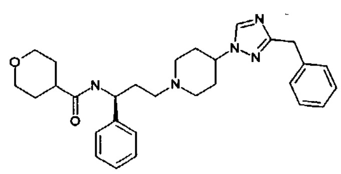

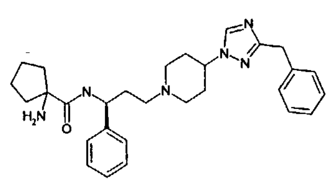

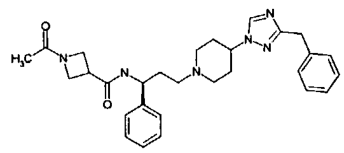

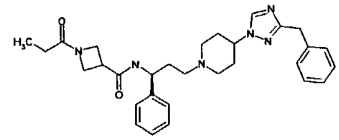

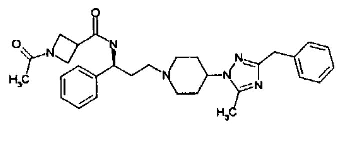

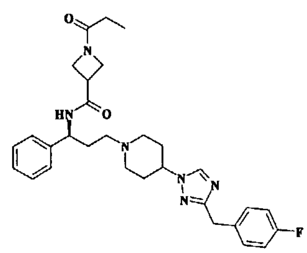

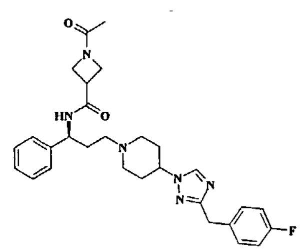

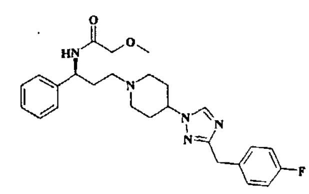

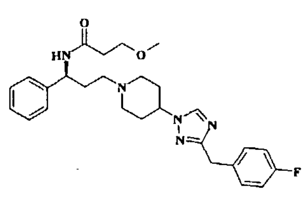

Preferred compounds of WO-A-00/39125 may be selected from the group consisiting of:

*N*-{(1*S*)-3-[4-(3-Benzyl-1,2,4-oxadiazol-5-yl)-1-piperidinyl]-1-phenylpropyl}cyclobutanecarboxamide

*N*-{1-Phenyl-3-[4-(4*H*-1,2,4-triazol-4-yl)-1-piperidinyl]-1-phenylpropyl}cyclobutanecarboxamide
*N*-{3-[4-(1-Methyl-1*H*-1,2,4-triazol-5-yl)-1-piperidinyl]-1-phenylpropyl}cyclobutanecarboxamide
*N*-{3-[4-(1-Methyl-1*H*-1,2,4-triazol-3-yl)-1-piperidinyl]-1-phenylpropyl}cyclobutanecarboxamide
*N*-{3-[4-(3,5-Dimethyl-4*H*-1,2,4-triazol-4-yl)-1-piperidinyl]-1-phenylpropyl}cyclobutanecarboxamide
*N*-{1-Phenyl-3-[4-(3-methyl-1,2,4-oxadiazol-5-yl)-1-piperidinyl]propyl}cyclobutanecarboxamide
*N*-{1-Phenyl-3-[4-(3-phenyl-1,2,4-oxadiazol-5-yl)-1-piperidinyl]propyl}cyclobutanecarboxamide
*N*-{3-[4-(3-Benzyl-1,2,4-oxadiazol-5-yl)-1-piperidinyl]-1-phenylpropyl}cyclobutanecarboxamide
*N*-(3-{4-[3-(4-Methoxyphenyl)-1,2,4-oxadiazol-5-yl]-1-piperidinyl}-1-phenylpropyl)cyclobutanecarboxamide
*N*-{3-[4-(5-Methyl-1,2,4-oxadiazol-3-yl)-1-piperidinyl]-1-phenylpropyl}cyclobutanecarboxamide
*N*-{1-Phenyl-3-[4-(5-phenyl-1,2,4-oxadiazol-3-yl)-1-piperidinyl]propyl}cyclobutanecarboxamide
*N*-{3-[4-(5-Benzyl-1,2,4-oxadiazol-3-yl)-1-piperidinyl]-1-phenylpropyl}cyclobutanecarboxamide
*N*-{3-[4-(5-Methyl-1,3,4-oxadiazol-2-yl)-1-piperidinyl]-1-phenylpropyl}cyclobutanecarboxamide
*N*-{1-Phenyl-3-[4-(5-phenyl-1,3,4-oxadiazol-2-yl)-1-piperidinyl]propyl}cyclobutanecarboxamide
*N*-{3-[4-(5-Benzyl-1,3,4-oxadiazol-2-yl)-1-piperidinyl]-1-phenylpropyl}cyclobutanecarboxamide
*N*-[(1*S*)-3-[4-(3-Benzyl-1,2,4-oxadiazol-5-yl)-1-piperidinyl]-1-(3-fluorophenyl)propyl]-2-cyclopropylacetamide
*N*-((1*S*)-3-{4-[3-(4-Methylbenzyl)-1,2,4-oxadiazol-5-y1]-1-piperidinyl}-1-phenylpropyl)cyclobutanecarboxamide
*N*-((1*S*)-3-{4-[3-(4-Trifluoromethylbenzyl)-1,2,4-oxadiazol-5-yl]-1-piperidinyl}-1-phenylpropyl)cyclobutanecarboxamide
*N*-((1*S*)-3-{4-[3-(1,3-Benzodioxol-5-ylmethyl)-1,2,4-oxadiazol-5-yl]-1-piperidinyl}-1-phenylpropyl)cyclobutanecarboxamide (UK-383290-51)
*N*-((1*S*)-3-{4-[3-(3,5-Difluorobenzyl)-1,2,4-oxadiazol-5-yl]-1-piperidinyl}-1-phenylpropyl)cyclobutanecarboxamide
*N*-[(1*S*)-3-[4-(3-Benzyl-1,2,4-oxadiazol-5-yl)-1-piperidinyl]-1-(3-fluorophenyl)propyl]cyclobutanecarboxamide
*N*-{(1*S*)-3-[4-(3-{4-[(Methylsulfonyl)amino]benzyl}-1,2,4-oxadiazol-5-yl)-1-piperidinyl]-1-phenylpropyl}cyclobutanecarboxamide
4-{[5-(1-{(3*S*)-3-[(Cyclobutylcarbonyl)amino]-3-phenylpropyl}-4-piperidinyl)-1,2,4-oxadiazol-3-yl]methyl}benzamide
*N*-((1*S*)-3-{4-[3-(2,5-Difluorobenzyl)-1,2,4-oxadiazol-5-yl]-1-piperidinyl}-1-phenylpropyl)cyclobutanecarboxamide (UK-384644-51)
*N*-((1*S*)-3-{4-[3-(2,6-Difluorobenzyl)-1,2,4-oxadiazol-5-yl]-1-piperidinyl}-1-phenylpropyl)cyclobutanecarboxamide (UK-384647-51)
*N*-((1*S*)-1-Phenyl-3-{4-[3-(3-pyridinylmethyl)-1,2,4-oxadiazol-5-yl]-1-piperidinyl}propyl)cyclobutanecarboxamide
*N*-((1*S*)-1-Phenyl-3-{4-[3-(4-pyridinylmethyl)-1,2,4-oxadiazol-5-yl]-1-piperidinyl}propyl)cyclobutanecarboxamide
*N*-{(1*S*)-3-[4-(3-{2-[(Methylsulfonyl)amino]benzyl}-1,2,4-oxadiazol-5-yl)-1-piperidinyl]-1-phenylpropyl}cyclobutanecarboxamide
*N*-((1*S*)-1-Phenyl-3-{4-[3-(2-pyridinylmethyl)-1,2,4-oxadiazol-5-yl]-1-piperidinyl}propyl)cyclobutanecarboxamide
*N*-{(1*S*)-3-[4-(3-Isobutyl-1,2,4-oxadiazol-5-yl)-1-piperidinyl]-1-phenylpropyl}cyclobutanecarboxamide
*N*-((1*S*)-3-{4-[3-(3-Chlorobenzyl)-1,2,4-oxadiazol-5-yl]-1-piperidinyl}-1-phenylpropyl)cyclobutanecarboxamide
*N*-((1*S*)-3-{4-[3-(1-Benzofuran-5-ylmethyl)-1,2,4-oxadiazol-5-yl]-1-piperidinyl}-1-phenylpropyl)cyclobutanecarboxamide
*N*-[(1*S*)-1-Phenyl-3-(4-{3-[4-(trifluoromethoxy)benzyl]-1,2,4-oxadiazol-5-yl}-1-piperidinyl)propyl]cyclobutanecarboxamide
*N*-{(1*S*)-3-[4-(3-{3-[(Methylsulfonyl)amino]benzyl}-1,2,4-oxadiazol-5-yl)-1-piperidinyl]-1-phenylpropyl}cyclobutanecarboxamide
3,3,3-Trifluoro-*N*-{(1*S*)-3-[4-(3-{4-[(methylsulfonyl)amino]benzyl}-1,2,4-oxadiazol-5-yl)-1-piperidinyl]-1-phenylpropyl}propanamide
2-Cyclopropyl-*N*-{(1*S*)-3-[4-(3-{4-[(methylsulfonyl)amino]benzyl}-1,2,4-oxadiazol-5-yl)-1-piperidinyl]-1-phenylpropyl}acetamide
*N*-{(1*S*)-3-[4-(3-{4-[(Methylsulfonyl)amino]benzyl}-1,2,4-oxadiazol-5-yl)-1-piperidinyl]-1-phenylpropyl}tetrahydro-2*H*-pyran-4-carboxamide
1-Acetyl-*N*-{(1*S*)-3-[4-(3-{4-[(methylsulfonyl)amino]benzyl}-1,2,4-oxadiazol-5-yl)-1-piperidinyl]-1-phenylpropyl}-3-azetidinecarboxamide
*N*-{(1*S*)-3-[4-(3-Benzyl-1,2,4-oxadiazol-5-yl)-1-piperidinyl]-1-phenylpropyl}tetrahydro-2*H*-pyran-4-carboxamide
1-Acetyl-*N*-{(1*S*)-3-[4-(3-benzyl-1,2,4-oxadiazol-5-yl)-1-piperidinyl]-1-phenylpropyl}-3-azetidinecarboxamide
1-(Acetylamino)-*N*-{(1*S*)-3-[4-(3-benzyl-1,2,4-oxadiazol-5-yl)-1-piperidinyl]-1-phenylpropyl}cyclopentanecarboxamide
*N*-{(1*S*)-3-[4-(3-Benzyl-1,2,4-oxadiazol-5-yl)-1-piperidinyl]-1-phenylpropyl}-1-methoxycyclobutanecarboxamide
3-{[5-(1-{(3*S*)-3-[(Cyclobutylcarbonyl)amino]-3-phenylpropyl}-4-piperidinyl)-1,2,4-oxadiazol-3-yl]methyl}benzamide

Ethyl 4-(3-benzyl-1,2,4-oxadiazol-5-yl)-1-{(3S)-3-[(cyclobutylcarbonyl)amino]-3-phenylpropyl}-4-piperidinecarboxylate
*N*-{(1*S*)-3-[4-(3-Benzyl-1,2,4-oxadiazol-5-yl)-4-cyano-1-piperidinyl]-1-phenylpropyl}cyclobutanecarboxamide
*N*-[(1*S*)-3-(4-{3-[3-(Aminosulfonyl)benzyl]-1,2,4-oxadiazol-5-yl}-1-piperidinyl)-1-phenylpropyl]cyclobutanecarboxamide
1-{(3*S*)-3-[(Cyclobutylcarbonyl)amino]-3-phenylpropyl}-4-[3-(4-fluorobenzyl)-1,2,4-oxadiazol-5-yl]-*N*-methyl-4-piperidinecarboxamide
*N*-((1*S*)-3-{4-[3-(4-Fluorobenzyl)-1,2,4-oxadiazol-5-yl]-1-piperidinyl}-1-phenylpropyl)tetrahydro-2*H*-pyran-4-carboxamide
3,3,3-Trifluoro-*N*-((1*S*)-3-{4-[3-(4-fluorobenzyl)-1,2,4-oxadiazol-5-yl]-1-piperidinyl}-1-phenylpropyl)propanamide
*N*-((1*S*)-3-{4-[3-(4-Morpholinylmethyl)-1,2,4-oxadiazol-5-yl]-1-piperidinyl}-1-phenylpropyl)cyclobutanecarboxamide
*N*-({1*S*)-3-{4-Cyano-4-[3-(4-fluorobenzyl)-1,2,4-oxadiazol-5-yl]-1-piperidinyl}-1-phenylpropyl)tetrahydro-2*H*-pyran-4-carboxamide
*N*-((1*S*)-3-{4-Cyano-4-[3-(4-fluorobenzyl)-1,2,4-oxadiazol-5-yl]-1-piperidinyl}-1-phenylpropyl)-2-cyclopropylacetamide
1-Acetyl-*N*-((1*S*)-3-{4-cyano-4-[3-(4-fluorobenzyl)-1,2,4-oxadiazol-5-yl]-1-piperidinyl}-1-phenylpropyl)-3-azetidinecarboxamide
*N*-((1*S*)-3-{4-Cyano-4-[3-(4-fluorobenzyl)-1,2,4-oxadiazol-5-yl]-1-piperidinyl}-1-phenylpropyl)-3,3,3-trifluoropropanamide
*N*-[(1*S*)-3-(4-{3-[4-(Aminosulfonyl)benzyl]-1,2,4-oxadiazol-5-yl}-1-piperidinyl)-1-phenylpropyl]cyclobutanecarboxamide
*N*-{(1*S*)-3-[3-Benzyl-1,2,4-oxadiazol-5-yl)-1-azetidinyl]-1-phenylpropyl}tetrahydro-3-furancarboxamide
*N*-[(1*S*)-3-(4-{3-[(4-Acetyl-1-piperazinyl)methyl]-1,2,4-oxadiazol-5-yl}-1-piperidinyl)-1-phenylpropyl]cyclobutanecarboxamide
*N*-{(1*S*)-3-[3-Benzyl-1,2,4-oxadiazol-5-yl)-1-azetidinyl]-1-phenylpropyl}tetrahydro-3-furancarboxamide
*N*-{(1*S*)-3-[4-[3-(4-Fluorobenzyl)-1,2,4-oxadiazol-5-yl]-4-(methoxymethyl)-1-piperidinyl]-1-phenylpropyl}acetamide
*N*-{3-[4-(3-Methyl-5-phenyl-4*H*-1,2,4-triazol-4-yl)-1-piperidinyl]-1-phenylpropyl}cyclobutanecarboxamide
*N*-{(1*S*)-3-[4-(3-Benzyl-5-methyl-4*H*-1,2,4-triazol-4-yl)-1-piperidinyl]-1-phenylpropyl}cyclobutanecarboxamide
*N*-{(1*S*)-3-[4-(5-Benzyl-4-methyl-4*H*-1,2,4-triazol-3-yl)-1-piperidinyl]-1-phenylpropyl}cyclobutanecarboxamide
*N*-{(1*S*)-3-[4-(3-Benzyl-1*H*-1,2,4-triazol-1-yl)-1-piperidinyl]-1-phenylpropyl}-cyclobutanecarboxamide
*N*-{(1*S*)-3-[4-(5-Benzyl-1-methyl-1*H*-1,2,4-triazol-3-yl)-1-piperidinyl]-1-phenylpropyl}cyclobutanecarboxamide
*N*-{3-[4-(5-Benzyl-1*H*-1,2,4-triazol-3-yl)-1-piperidinyl]-1-phenylpropyl}cyclobutanecarboxamide
*N*-{(1*S*)-3-[4-(3-{4-[(methylsulfonyl)amino]benzyl}-1*H*-1,2,4-triazol-1-yl)-1-piperidinyl]-1-phenylpropyl}tetrahydro-2*H*-pyran-4-carboxamide
2-Cyclopropyl-*N*-{(1*S*)-3-[4-(3-{4-[(methylsulfonyl)amino]benzyl}-1*H*-1,2,4-triazol-1-yl)-1-piperidinyl]-1-phenylpropyl}acetamide
3,3,3-Trifluoro-*N*-{(1*S*)-3-[4-(3-{4-[(methylsulfonyl)amino]benzyl}-1*H*-1,2,4-triazol-1-yl)-1-piperidinyl]-1-phenylpropyl}propanamide
*N*-(1*S*)-{3-[4-(3-Benzyl-1-methyl-1*H*-1,2,4-triazol-5-yl)-1-piperidinyl]-1-phenylpropyl}cyclobutanecarboxamide
4-{[1-(1-{(3*S*)-3-Phenyl-3-[(3,3,3-trifluoropropanoyl)amino]propyl}-4-piperidinyl)-1*H*-1,2,4-triazol-3-yl]methyl}benzamide
*N*-{(1*S*)-3-[4-(3-Benzyl-5-methyl-1*H*-1,2,4-triazol-1-yl)-1-piperidinyl]-1-phenylpropyl}tetrahydro-2*H*-pyran-4-carboxamide
*N*-{(1*S*)-3-[4-(3-Benzyl-5-methyl-1*H*-1,2,4-triazol-1-yl)-1-piperidinyl]-1-phenylpropyl}tetrahydro-3-furancarboxamide
1-Amino-*N*-{(1*S*)-3-[4-(3-benzyl-5-methyl-1*H*-1,2,4-triazol-1-yl)-1-piperidinyl]-1-phenylpropyl}cyclopentanecarboxamide
*N*-{(1*S*)-3-[4-(3-Benzyl-1*H*-1,2,4-triazol-1-yl)-1-piperidinyl]-1-phenylpropyl}tetrahydro-3-furancarboxamide
*N*-{(1*S*)-3-[4-(3-Benzyl-1*H*-1,2,4-triazol-1-yl)-1-piperidinyl]-1-phenylpropyl}tetrahydro-2*H*-pyran-4-carboxamide
1-Amino-*N*-{(1*S*)-3-[4-(3-benzyl-1*H*-1,2,4-triazol-1-yl)-1-piperidinyl]-1-phenylpropyl}cyclopentanecarboxamide
1-Acetyl-*N*-{(1*S*)-3-[4-(3-benzyl-1*H*-1,2,4-triazol-1-yl)-1-piperidinyl]-1-phenylpropyl}-3-azetidinecarboxamide
*N*-{(1*S*)-3-[4-(3-Benzyl-1*H*-1,2,4-triazol-1-yl)-1-piperidinyl]-1-phenylpropyl}-1-propionyl-3-azetidinecarboxamide
1-Acetyl-*N*-{(1*S*)-3 - [4-(3 -benzyl-5-methyl-1*H*-1,2,4-triazol-1-yl)-1-piperidinyl]-1-phenylpropyl}-3-azetidinecarboxamide
*N*-{(1*S*)-3-[4-(3-(4-Fluorobenzyl)-1*H*-1,2,4-triazol-1-yl)-1-piperidinyl]-1-phenylpropyl}-1-propionyl-3-azetidinecarboxamide

1-Acetyl-*N*-{(1*S*)-3-[4-(3-(4-fluorobenzyl)-1*H*-1,2,4-triazol-1-yl)-1-piperidinyl]-1-phenylpropyl}-3-azetidinecarboxamide
2-Methoxy-*N*-{(1*S*)-3-[4-(3-(4-fluorobenzyl)-1*H*-1,2,4-triazol-1-yl)-1-piperidinyl]-1-phenylpropyl}acetamide
3-Methoxy-*N*-{(1*S*)-3-[4-(3-(4-fluorobenzyl)-1*H*-1,2,4-triazol-1-yl)-1-piperidinyl]-1-phenylpropyl}propanamide

Examples of agents may be the compounds disclosed in EP-A-1013276 (claiming priority from *inter alia* GB patent application No. 9922702 filed 25 September 1999)-the contents of which are incorporated herein by reference.
The compounds of EP-A-1013276 may be of the Formula (I):

$$[R_{egion}\ \alpha] - [R_{egion}\ \beta] - [R_{egion}\ \gamma] - [R_{egion}\ \delta] \quad (I)$$

wherein **[$R_{egion}$ α]** is selected from the group consisting of:

- A. Aryl heterocyclyl substituent components comprising:

  - 1. hetero-phenylmethylene moieties of partial Formula (1.0.0):

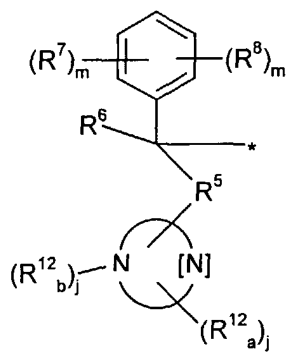

(1.0.0)

--- wherein: the symbol "*" indicates the point of attachment of the moiety of partial Formula (1.0.0) to $R_{egion}$ β, as hereinafter defined;
--- $R^5$ is a member selected from the group consisting of a direct bond; -O-; -C(=O)-; $-NR^4-$; and $-S(=O)_p-$; where:

---- $R^4$ is hydrogen or $(C_1\text{-}C_2)$alkyl;

--- $R^6$ is a member selected from the group consisting of hydrogen; $(C_1\text{-}C_2)$alkyl; $(C_1\text{-}C_2)$alkoxy; -CN; -OH; and $-C(=O)NH_2$;
--- j is an integer selected from 0, 1, and 2;
--- m is an integer selected from 0, 1, and 2;
--- $R^7$ and $R^8$ are each a member selected from the group consisting of -F; -Cl; $-CO_2R^4$; -OH; -CN; $-CONR^4_aR^4_b$; $-NR^4_aR^4_b$-; $-NR^4_aC(=O)R^4_b$; $-NR^4_aC(=O)OR^4_b$; $-NR^4_aS(=O)_pR^4_b$; $-S(=O)_pNR^4_aR^4_b$; $(C_1\text{-}C_4)$alkyl, and $(C_1\text{-}C_4)$alkoxy wherein said alkyl and alkoxy are each substituted with 0 to 3 substituents independently selected from F and Cl; $(C_1\text{-}C_2)$alkoxycarbonyl; $(C_1\text{-}C_2)$alkylcarbonyl; and $(C_1\text{-}C_2)$alkylcarbonyloxy; where:

---- p is an integer selected from 0, 1, and 2;
---- $R^4_a$ and $R^4_b$ are each independently selected from hydrogen and $(C_1\text{-}C_2)$alkyl;

--- the moiety represented by partial Formula (1.0.1):

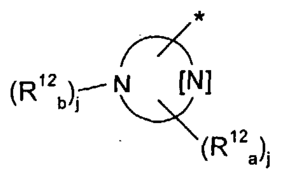

(1.0.1)

in partial Formula (1.0.0) represents a monocyclic heterocyclic group, or a bicyclic benzofused ring system containing said heterocyclic group wherein said heterocyclic group contains a total of 5- or 6- members of which one or two of said members is nitrogen, the presence of the optional second nitrogen atom being represented by: "[N]"; wherein said heterocyclic group or ring system are selected from the group consisting of pyrrolyl; pyrazolyl; imidazolyl; pyridinyl; pyrazinyl; pyrimidinyl; pyridazinyl; piperazinyl; indolyl; indazolinyl; benzimidazolyl; quinolinyl; *iso*-quinolinyl; and quinazolinyl; wherein:

---- $R^{12}_a$ is a member selected from the group consisting of hydrogen; F; Cl; $-CO_2R^4$; oxo; -OH; CN; $NH_2$; $NH(C_1\text{-}C_2)$alkyl; $N(C_1\text{-}C_2)_2$dialkyl; $-CF_3$; $(C_1\text{-}C_4)$alkyl; $(C_2\text{-}C_4)$alkenyl; $(C_1\text{-}C_4)$alkoxy; $(C_3\text{-}C_7)$cycloalkyl; and phenyl; wherein said alkyl, alkenyl, alkoxy, cycloalkyl and phenyl are substituted with 0 to 2 substituents $R^9$ where:

----- $R^9$ is a member independently selected from the group consisting of F; Cl; $-CO_2R^4$; -OH; cyano; $-CONR^4_aR^4_b$; $-NR^4_aR^4_b$-; $-NR^4_aC(=O)R^4_b$; $-NR^4_aC(=O)OR^4_b$; $-NR^4_aS(=O)_pR^4_b$; $-S(=O)_pNR^4_aR^4_b$; $(C_1\text{-}C_4)$alkyl including dimethyl, and $(C_1\text{-}C_4)$alkoxy wherein said alkyl and alkoxy are each independently substituted with 0 to 3 substituents independently selected from F and Cl; $(C_1\text{-}C_2)$alkoxycarbonyl; $(C_1\text{-}C_2)$alkylcarbonyl; and $(C_1\text{-}C_2)$alkylcarbonyloxy; and

---- $R^{12}_b$ is absent or is a member selected from the group consisting of hydrogen; $(C_1\text{-}C_4)$alkyl; $(C_2\text{-}C_4)$alkenyl; $(C_1\text{-}C_2)$alkoxy; $(C_3\text{-}C_7)$cycloalkyl; and phenyl; wherein said alkyl, alkenyl, alkoxy, cycloalkyl and phenyl are substituted with 0 to 2 substituents $R^9$ wherein $R^9$ has the same meaning as above, except that it is selected independently selected therefrom; and

2. hetero-phenylmethylene moieties of partial Formula (1.1.0):

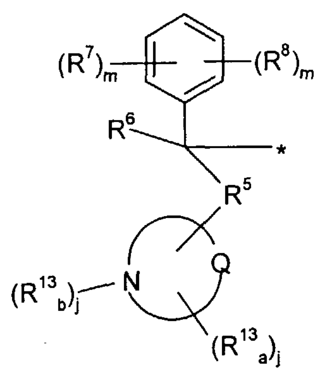

(1.1.0)

--- wherein: the symbol "*"; $R^5$; $R^6$; $R^7$; $R^8$; j and m are as defined further above, except that all of the above-recited substituents are selected independently of their selection above;

--- the moiety represented by partial Formula (1.1.1):

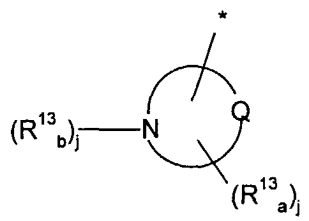

(1.1.1)

in partial Formula (1.1.0) represents:

---- a. a monocyclic heterocyclic group containing a total of 5 or 6 members of which one said member is nitrogen and Q is selected from O and S where said S may optionally be in the sulfonate form, $-S(=O)_2$; wherein said heterocyclic group is selected from the group consisting of oxazolyl; oxazolidinyl; *iso*xazolyl; thiazolyl; thiazolidinyl; *iso*-thiazolyl; morpholinyl; and thiomorpholinyl; or

---- b. a monocyclic heterocyclic group containing a total of 5- or 6- member s of which two said members are nitrogen and a third or fourth said member is independently selected from N, O, and S where said S may optionally be in the sulfonate form, $-S(=O)_2$; wherein said heterocyclic group is selected from the group consisting of triazolyl; triazinyl; tetrazolyl; oxadiazolyl; thiadiazolyl; and

---- $R^{13}{}_a$ is selected from the group consisting of hydrogen; F; Cl; $-CO_2R^4$; oxo; -OH; CN; $NH_2$; $NH(C_1-C_2)$alkyl; $N(C_1-C_2)_2$dialkyl; $-CF_3$; $(C_1-C_4)$alkyl; $(C_2-C_4)$alkenyl; $(C_1-C_2)$alkoxy; $(C_3-C_7)$cycloalkyl; and phenyl; wherein said alkyl, alkenyl, alkoxy, cycloalkyl and phenyl are substituted with 0 to 2 substituents $R^{11}$ where:

----- $R^{11}$ is a member selected from the group consisting of F; Cl; $-CO_2R^4$; -OH; -CN; $-CONR^4{}_aR^4{}_b$; $-NR^4{}_aR^4{}_b$; $-NR^4{}_aC(=O)R^4{}_b$; $-NR^4{}_aC(=O)OR^4{}_b$; $-NR^4{}_aS(=O)_pR^4{}_b$; $-S(=O)_pNR^4{}_aR^4{}_b$; $(C_1-C_4)$alkyl including dimethyl, and $(C_1-C_4)$alkoxy wherein said alkyl and alkoxy are each independently substituted with 0 to 3 substituents independently selected from F and Cl; $(C_1-C_2)$alkoxycarbonyl; $(C_1-C_2)$alkylcarbonyl; and $(C_1-C_2)$alkylcarbonyloxy; and

---- $R^{13}{}_b$ is a member selected from the group consisting of hydrogen; $(C_1-C_4)$alkyl; $(C_2-C_4)$alkenyl; $(C_1-C_2)$alkoxy; $(C_3-C_7)$cycloalkyl; $C(=O)(C_1-C_4)$alkyl; $S(=O)_2(C_1-C_4)$alkyl; and phenyl; wherein said alkyl, alkenyl, alkoxy, cycloalkyl and phenyl are substituted with 0 to 2 substituents $R^{11}$ wherein $R^{11}$ has the same meaning as in above, except that it is selected independently;

- B. a (substituted)-amido-aryl or -heterocyclyl moiety selected from the group consisting of

-- 1. alkyl-, alkenyl-, and alkynyl-substituted-amido-aryl moieties of partial Formula (2.0.0):

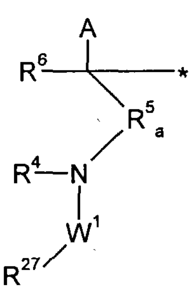

(2.0.0)

--- wherein: the symbol "*"; $R^4$ and $R^6$; are as defined above, except that all of the above-recited substituents are selected independently of their selection above;

--- A is a member selected from the group consisting of:

---- 1. the moiety of partial Formula (2.0.3)

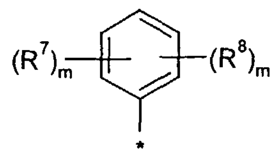

(2.0.3)

----- wherein: the symbol $R^7$; $R^8$ and m are as defined above, except that all of the above-recited substituents are selected independently of their selection above; and the symbol: "*" indicates the point of attachment of the moiety A to the, remaining portions of partial Formula (2.0.0);

---- 2. the moiety of partial Formula (2.0.4)

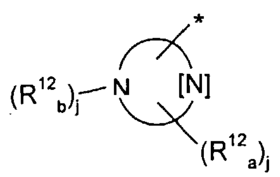

(2.0.4)

which represents a monocyclic heterocyclic group, selected from the group consisting of pyrrolyl; pyrazolyl; imidazolyl; pyridinyl; pyrazinyl; pyrimidinyl; wherein: the symbol $R^{12}{}_a$ and $R^{12}{}_b$ are as defined above, except that all of the above-recited substituents are selected independently of their selection above; and the symbol: " * " indicates the point of attachment of the moiety A to the other, remaining portions of partial Formula (2.0.0);

---- 3. the moiety of partial Formula (2.0.5)

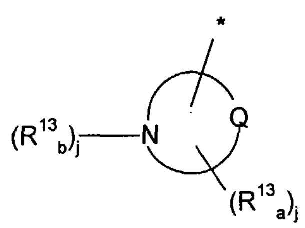

(2.0.5)

which represents

----- a. a monocyclic heteroaromatic group containing a total of 5- members of which one said member is nitrogen and Q is selected from O and S where said S may optionally be in the sulfonate form, $-S(=O)_2$; selected from the group consisting of oxazolyl; *iso*xazolyl; thiazolyl; and *iso*-thiazolyl; or

----- b. a monocyclic heterocyclic group containing a total of 5- or 6- members of which two said members are nitrogen and a third or fourth said member is independently selected from N, O, and S where said S may optionally be in the sulfonate form, $-S(=O)_2$; selected from the group consisting of triazolyl; triazinyl; tetrazolyl; oxadiazolyl; and thiadiazolyl; and

------wherein: the $R^{13}{}_a$, $R^{13}{}_b$ and j are as defined above, except that all of the above-recited substituents are selected independently of their selection above; and the symbol: "*" indicates

the point of attachment of the moiety A to the other, remaining portions of partial Formula (2.0.2);

--- $R^5{}_a$ is a member selected from the group consisting of a direct bond; -C(=O)-; and $-S(=O)_2-$;

--- $W^1$ is (1.) a direct bond; (2.) in the case where $R^5{}_a$ is -C(=O)- or $-S(=O)_2$, $W^1$ is a direct bond or $-(C_1-C_3)$ alkylene- wherein any single carbon atom thereof is substituted by 0 to 2 substituents $R^{23}$ where $R^{23}$ is a member selected from the group consisting of -F; -Cl; $-CO_2R^4$; -OH; -CN; $(C_1-C_4)$alkoxy; $(C_3-C_7)$cycloalkyl; and phenyl; wherein said alkoxy, cycloalkyl, and phenyl are substituted with 0 to 2 substituents $R^{11}$, wherein said $R^{11}$ is as defined above, except that all of the above-recited substituents are selected independently of their selection above; or (3.) is a member independently selected from the group consisting of the moieties of partial Formulas (2.0.6) through (2.0.16), inclusive:

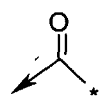

(2.0.6)

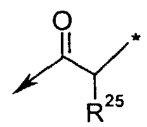

(2.0.7)

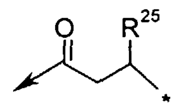

(2.0.8)

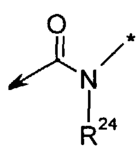

(2.0.9)

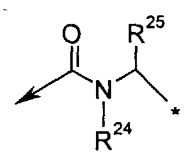

(2.0.10)

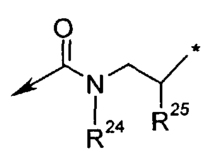

(2.0.11)

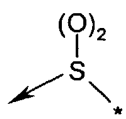

(2.0.12)

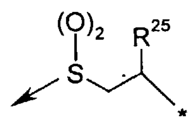

(2.0.13)

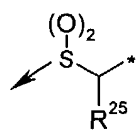

(2.0.14)

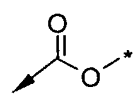

(2.0.15)

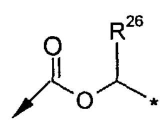

(2.0.16)

---- wherein: the symbol: "→" indicates the point of attachment of the moiety $W^1$ to the nitrogen atom in partial Formula (2.0.0), and the symbol: " * " indicates the point of attachment of the moiety $W^1$ to the other, remaining portions of partial Formula (2.0.0); and $R^4$ is as defined further above, but selected on an independent basis;

--------$R^{24}$ is selected from the group consisting of hydrogen and $(C_1-C_4)$alkyl; and

---- $R^{25}$ and $R^{26}$are each selected from the group consisting of-OH; $(C_{1-}C_2)$alkyl substituted by 0 to 3 substituents selected from F; and OH; and $(C_{1-}C_2)$alkoxy; and

--- $R^{27}$ is selected from the group consisting of $(C_{1-}C_6)$alkyl; $(C_{2-}C_6)$alkenyl; and $(C_{2-}C_6)$alkynyl; wherein said alkyl, alkenyl, and alkynyl groups comprising $R^{27}$ are substituted with 0 to 3 substituents $R^{28}$ where:

---- $R^{28}$ is selected from the group consisting of phenyl; F or Cl; oxo; hydroxy; $(C_{1-}C_2)$alkyl; $(C_{1-}C_3)$alkoxy; $-C(=O)OR^{29}$; $-C(=O)(C_1-C_4)$alkyl; $-S(=O)_2(C_1-C_4)$alkyl; $-C(=O)NR^{29}R^{30}$; $-NR^{29}R^{30}$; $-NR^{29}C(=O)R^{30}$; $-NR^{29}C(=O)OR^{30}$; $-NR^{29}S(=O)_pR^{30}$; and $-S(=O)_2NR^{29}R^{30}$, where:

----- $R^{29}$ and $R^{30}$ are each a member independently selected from the group consisting of hydrogen

and ($C_{1-}C_4$)alkyl substituted by 0 to 3 substituents selected from the group consisting of F and Cl;

-- 2. cycloalkyl-substituted-amido-aryl moieties of partial Formula (2.1.0):

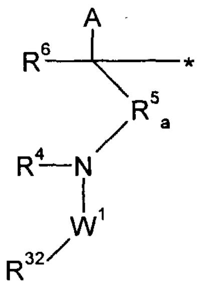

(2.1.0)

--- wherein: A; $W^1$; the symbol "*"; $R^4$; $R^5_a$; and $R^6$ have the same meaning as set out above, except that all of the above-recited substituents are selected independently of their selection above; and

--- $R^{32}$ is a member selected from the group consisting of-$(CH_2)_{n-}(C_{3-}C_7)$cycloalkyl, where n is an integer selected from 0, 1, and 2; in the event n is 0, then the α-carbon atom of said ($C_{3-}C_7$)cycloalkyl is substituted by 0 or 1 ($C_{1-}C_4$)alkyl or phenyl, where said alkyl or phenyl are substituted by 0, 1, or 2 of $CH_3$, $OCH_3$, OH or $NH_2$; and in the event that n is 1 or 2, the resulting methylene or ethylene is substituted by 0 or 1 of F; $NH_2$; $N(CH_3)_2$; OH; $OCH_3$; ($C_{1-}C_4$)alkyl; or phenyl; where said alkyl and phenyl are substituted by 0, 1, or 2 of $CH_3$, $OCH_3$, OH, and $NH_2$; and further wherein said ($C_{3-}C_7$)cycloalkyl is substituted by 0 to 3 substituents $R^{28}$ where $R^{28}$ is as defined further above, but selected independently

-- 3. aryl and heterocyclic-substituted-amido-aryl moieties of partial Formula (2.2.0):

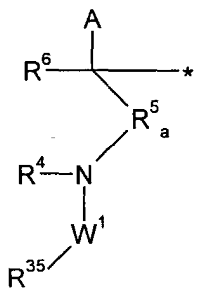

(2.2.0)

--- wherein: A; $W^1$; the symbol: "*"; $R^4$; $R^5_a$; and $R^6$ have the same meaning as set out above, except that all of the above-recited substituents are selected independently of their selection above; and

--- $R^{35}$ is selected from the group consisting of phenyl; furyl; tetrahydrofuranyl; tetrahydropyranyl; oxetanyl; thienyl; pyrrolyl; pyrrolidinyl; oxazolyl; isoxazolyl; thiazolyl; isothiazolyl; imidazolyl; pyrazolyl; oxadiazolyl; thiadiazolyl; triazolyl; pyridyl; pyrazinyl; pyridazinyl; piperazinyl; pyrimidinyl; pyranyl; azetidinyl; morpholinyl; parathiazinyl; indolyl; indolinyl; benzo[*b*]furanyl; 2;3-dihydrobenzofuranyl; benzothienyl; 1H-indazolyl; benzimidazolyl; benzoxazolyl; benzisoxazolyl; benzthiazolyl; quinolinyl; isoquinolinyl; phthalazinyl; quinazolinyl; and quinoxalinyl; wherein (1.) said group $R^{35}$ may be substituted upon any one or more carbon atoms thereof by 0 to 3 substituents $R^{28}$ where $R^{28}$ is as defined above, except that it is selected independently; (2.) said group $R^{35}$ is substituted with respect to any one or more nitrogen atoms thereof that is not a point of attachment of said aryl or heterocyclic moiety, by 0 to 3 substituents $R^{13}_b$ where $R^{13}_b$ is as defined above, except that it is selected independently; and (3.) said group $R^{35}$ with respect to any sulfur atom thereof that is not a point of attachment of said heterocyclic moiety, is substituted by 0 or 2 oxygen atoms;

**[$R_{egion}$ β]** is an alkyl bridging element of partial Formula (3.0.0):

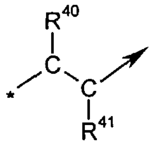

(3.0.0)

wherein:

- "*" is a symbol which represents the point of attachment of the moiety of partial Formula (3.0.0) to $R_{egion}$ $\alpha$;
- "→" is a symbol which represents the point of attachment of the moiety of partial Formula (3.0.0) to $R_{egion}$ $\gamma$;
- $R^{40}$ and $R^{41}$ are both selected from the group consisting of hydrogen; ($C_1$-$C_2$) alkyl including dimethyl; hydroxy; and ($C_1$-$C_3$) alkoxy;

**[$R_{egion}$ $\gamma$]** is an aza-monocyclic moiety of partial Formula (4.0.0):

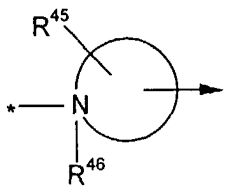

(4.0.0)

- wherein:
- "*" is a symbol which represents the point of attachment of the moiety of partial Formula (4.0.0) to $R_{egion}$ $\beta$ of the compound of Formula (I);
- "*→*" is a symbol representing a covalent bond attaching any carbon atom of said aza-monocyclic moiety of partial Formula (4.0.0) to $R_{egion}$ $\delta$;
- the moiety of partial Formula (4.0.1):

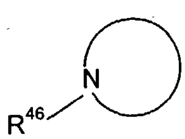

(4.0.1)

in partial Formula (4.0.0) represents a monocyclic heterocyclic group containing a total of from 4- to 7-members of which one said member is nitrogen, wherein said heterocyclic group is a member independently selected from the group consisting essentially of azetidinyl; pyrrolidinyl; piperidinyl; and azepinyl;

- $R^{45}$ is absent or is a member independently selected from the group consisting essentially of ($C_1$-$C_4$)alkyl including dimethyl; ($C_3$-$C_6$)cycloalkyl; ($C_1$-$C_4$)alkoxy; $CF_3$; $-CO_2R^4$ where $R^4$ is as defined further above; oxo; -OH; cyano; $-C(=O)NR^4_aR^4_b$; $-NR^4_aR^4_b$; $-NR^4_aC(=O)R^4_b$; $-NR^4_aC(=O)OR^4_b$; $-NR^4_aS(=O)_pR^4_b$; $-S(=O)_pNR^4_aR^4_b$; ($C_1$-$C_2$)alkoxycarbonyl; ($C_1$-$C_2$)alkylcarbonyl; ($C_1$-$C_2$)alkylcarbonyloxy and ($C_1$-$C_2$)alkoxy($C_1$-$C_2$)alkyl; it being understood that in the moiety of partial Formula (4.0.0) $R^{45}$ is a substituent attached to a single carbon atom thereof; where:
  - $R^4_a$ and $R^4_b$ are each independently selected from hydrogen and ($C_1$-$C_2$)alkyl;

-- $R^{46}$ is absent or is a member independently selected from the group consisting essentially of hydrogen; and ($C_1$-$C_4$)alkyl substituted by 0 or 1 substituent independently selected from ($C_1$-$C_2$)alkoxy and -$CO_2R^4$ where $R^4$ is as defined further above; and →O; it being understood that in the case where substituent $R^{46}$ is present, that it results in said nitrogen atom and said moiety of partial Formula (4.0.0) is in quaternary form;

**[$R_{egion}$ δ]** is a member consisting of:

- an aryl and heterocyclyl-(substituted) amide, carbamate; or urea moiety of partial Formula (5.1.0):

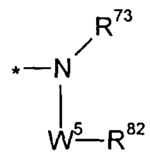

--- wherein: the symbol " * " is as defined above;

--- $R^{73}$ is a member selected from the group consisting of hydrogen and ($C_1$-$C_2$)alkyl;

--- $W^5$ is selected from the group consisting the moieties of partial Formulas (5.1.1) through (5.1.12):

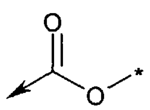 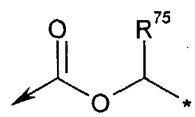 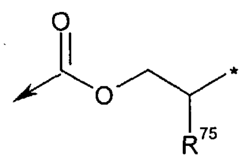 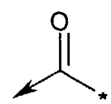

(5.1.1) (5.1.2) (5.1.3) (5.1.4)

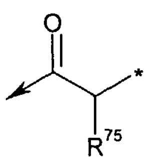 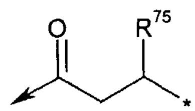 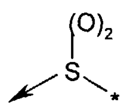 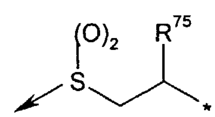

(5.1.5) (5.1.6) (5.1.7) (5.1.8)

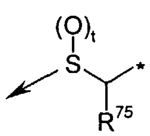 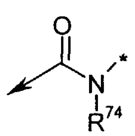 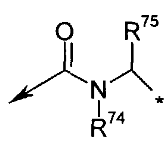 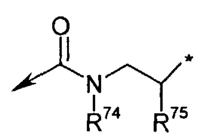

(5.1.9) (5.1.10) (5.1.11) (5.1.12)

---- wherein: the symbol: "→" indicates the point of attachment of the moiety $W^5$ represented by partial Formulas (5.1.1) through (5.1.12), inclusive, to the nitrogen atom in partial Formula (5.1.0), and the symbol: " * " indicates the point of attachment of the moiety $W^5$ to $R^{82}$ as defined further below;

---- $R^{74}$ and $R^{75}$ are each selected from the group consisting of hydrogen; ($C_1$-$C_2$)alkyl substituted by 0 or 1 substituent independently selected from OH; and ($C_1$-$C_2$)alkoxy; and

--- $R^{82}$ is a member selected from the group consisting of phenyl; cinnolinyl; furyl; thienyl; pyrrolyl; oxazolyl; isoxazolyl; thiazolyl; isothiazolyl; imidazolyl; imidazolinyl; pyrazolyl; pyrazolinyl; oxadiazolyl; thiadiazolyl; triazolyl; pyridyl; pyrazinyl; pyridazinyl; pyrimidinyl; parathiazinyl; indolyl; isoindolyl; indolinyl; benzo[*b*]furanyl; 2; 3-dihydrobenzofuranyl; benzo[*b*]thiophenyl; 1H-indazolyl; benzimidazolyl; benzthiazolyl; quinolinyl; isoquinolinyl; phthalazinyl; quinazolinyl; quinoxalinyl; wherein:

---- the aryl or heterocyclyl moiety is substituted by 0 to 3 substituents $R^{78}$, where:

- $R^{78}$ is a member selected from the group consisting of oxo; -Cl; -F; -OH; $-(C_{1-}C_2)$alkyl; $-(C_{1-}C_3)$alkoxy; $-CF_3$; -CN; $-C(=O)OR^{79}$; $-C(=O)NR^{79}K^{80}$; $-NR^{79}R^{80}$; $-NR^{79}C(=O)R^{80}$; $-NK^{79}C(=O)OR^{80}$; $-NR^{79}S(=O)_2R^{80}$; and $-S(=O)_2NR^{79}R^{80}$, where:
  - $R^{79}$ and $R^{80}$ are each a member independently selected from the group consisting of hydrogen; and $(C_{1-}C_4)$alkyl.

For the compounds of EP-A-10132762, the group of Formula (5.1.0) may be selected from the group consisting of: carbamates, ureas and amides.

For the compounds of EP-A-10132762, the group $W^5$ of Formula (5.1.0) may be selected from the group consisting of partial Formulas (5.1.4) to (5.1.12):

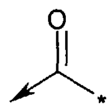

(5.1.4)

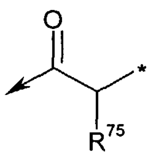 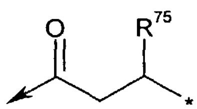 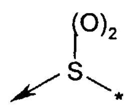 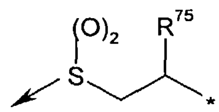

(5.1.5) (5.1.6) (5.1.7) (5.1.8)

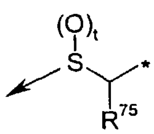 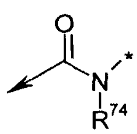 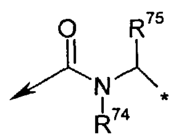 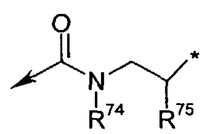

(5.1.9) (5.1.10) (5.1.11) (5.1.12)

Preferred compounds of EP-A-1013276 may be selected from the group consisting of:

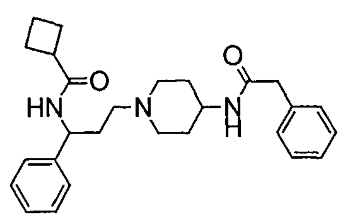

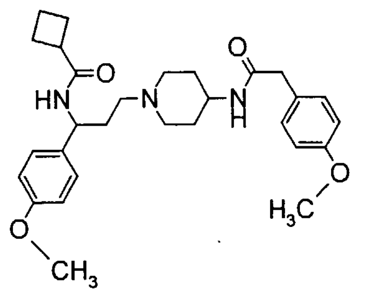

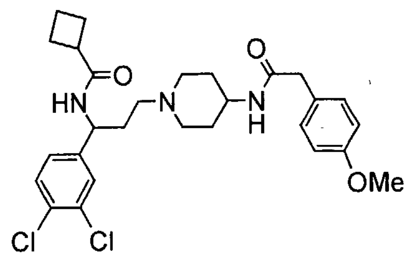

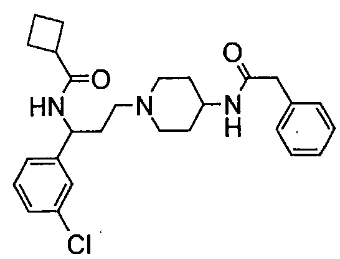

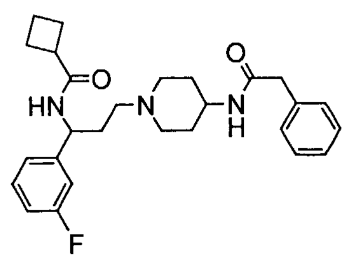

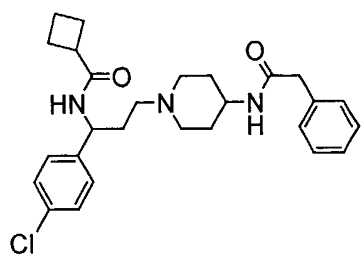

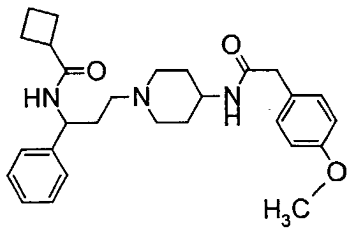

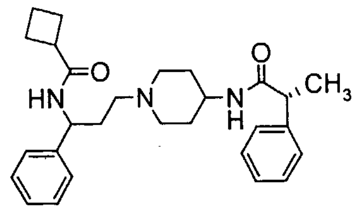

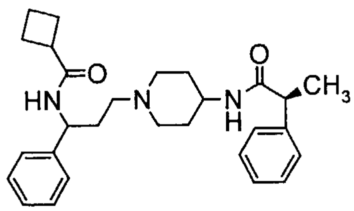

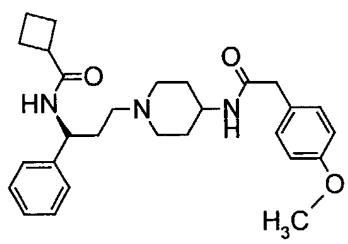

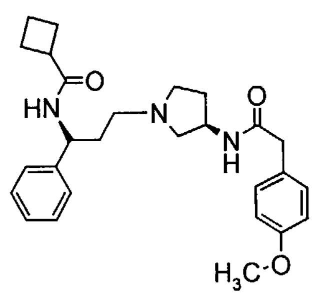

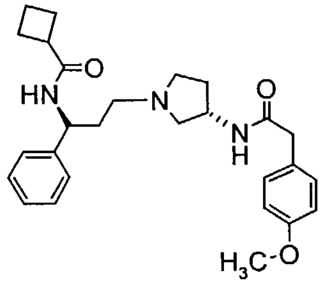

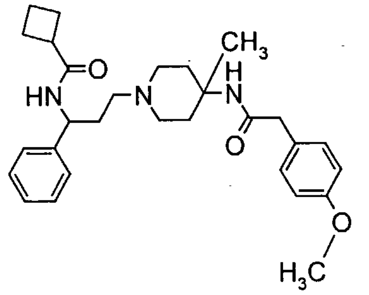

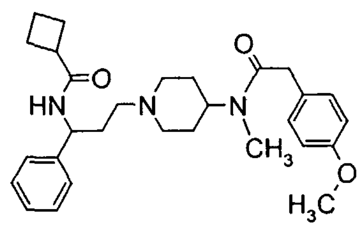

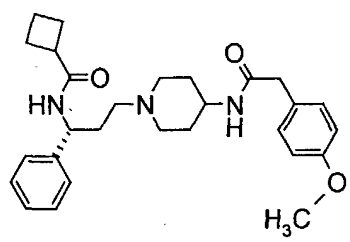

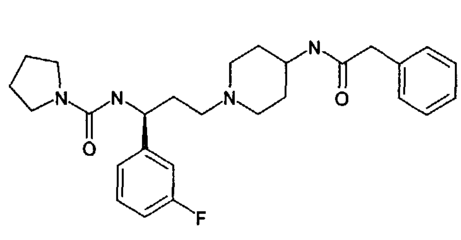

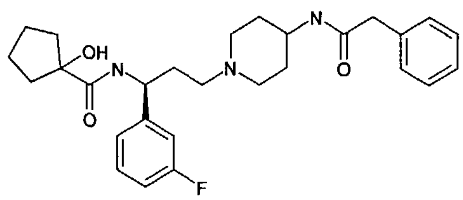

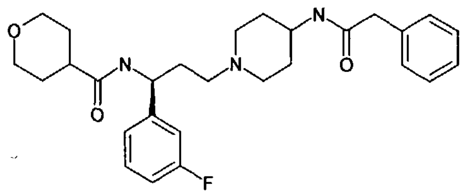

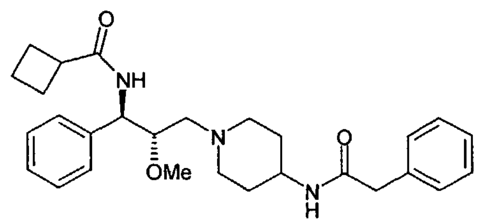

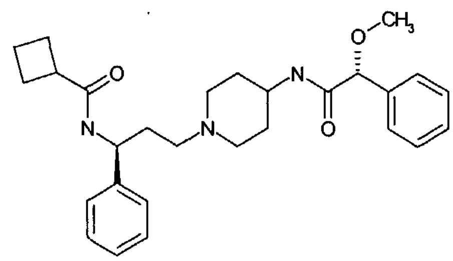

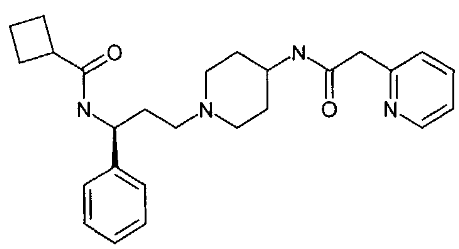

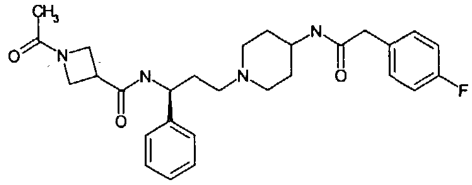

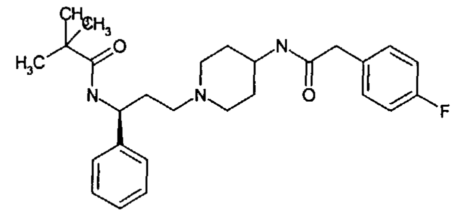

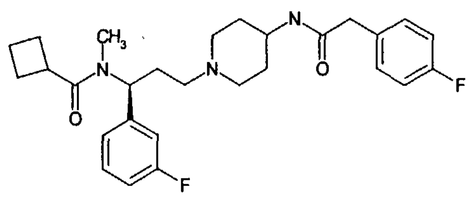

The preferred compounds of EP-A-1013276 may be selected from the group consisting of:

*N*-(1-Phenyl-3-{4-[(2-phenylacetyl)amino]-1piperidinyl}propyl)cyclobutanecarboxamide
*N*-[3-(4-{[2-(4-Methoxyphenyl)acetyl]amino}-1-piperidinyl)-1-(4-methoxyphenylpropyl]cyclobutanecarboxamide
*N*-[3-(4-{[2-(4-Methoxyphenyl)acetyl]amino}-1-piperidinyl)-1-(3,4-dichlorophenylpropyl]cyclobutanecarboxamide
*N*-[3-(4-{[2-Phenylacetyl]amino}-1-piperidinyl)-1-(3-chlorophenylpropyl]cyclobutanecarboxamide
*N*-[3-(4-{[2-Phenylacetyl]amino}-1-piperidinyl)-1-(3-fluorophenylpropyl]cyclobutanecarboxamide
*N*-[3-(4-{[2-Phenylacetyl]amino}-1-piperidinyl)-1-(4-chlorophenylpropyl]cyclobutanecarboxamide
*N*-[3-(4-{[2-(4-Methoxyphenyl)acetyl]amino}-1-piperidinyl)-1-phenylpropyl]cyclobutanecarboxamide
*N*-[1-Phenyl-3-(4-{[(2*R*)-2-phenylpropanoyl]amino}-1-piperidinyl)propyl]cyclobutanecarboxamide
*N*-[1-Phenyl-3-(4-{[(2*S*)-2-phenylpropanoyl]amino}-1-piperidinyl)propyl]cyclobutanecarboxamide
*N*-[(1*S*)-3-(4-{[2-(4-methoxyphenyl)acetyl]amino}-1-piperidinyl)-1-phenylpropyl]cyclobutanecarboxamide
*N*-[(1*S*)-3-((3*R*)-3-{[2-(4-methoxyphenyl)acetyl]amino}pyrrolidinyl)-1-phenylpropyl]cyclobutanecarboxamide
*N*-[(1*S*)-3-((3*S*)-3-{[2-(4-methoxyphenyl)acetyl]amino}pyrrolidinyl)-1-phenylpropyl]cyclobutanecarboxamide
*N*-(3-{4-[[2-(4-methoxyphenyl)acetyl](methyl)amino]-1-piperidinyl}-1-phenylpropyl)cyclobutanecarboxamide
*N*-[3-(4-{[2-(4-methoxyphenyl)acetyl]amino}-4-methyl-1-piperidinyl)-1-phenylpropyl]cyclobutanecarboxamide
*N*-[(1*R*)-3-(4-{[2-(4-Methoxyphenyl)acetyl]amino}-1-piperidinyl)-1-phenylpropyl]cyclobutanecarboxamide
*N*-((1*S*)-1-(3-Fluorophenyl)-3-{4-[(2-phenylacetyl)amino]-1-piperidinyl}propyl)-1-pyrrolidinecarboxamide
*N*-((1*S*)-1-(3-Fluorophenyl)-3-{4-[(2-phenylacetyl)amino]-1-piperidinyl}propyl)-1-hydroxycyclopentanecarboxamide
*N*-((1*S*)-1-(3 -Fluorophenyl)-3-{4-[(2-phenylacetyl)amino]-1-piperidinyl}propyl)tetrahydro-2*H*-pyran-4-carboxamide
*N*-((1*R*,2*S*)-2-Methoxy-1-phenyl-3-{4-[(2-phenylacetyl)amino]-1-piperidinyl}propyl)cyclobutanecarboxamide
*N*-[(1*S*)-3-(4-{[(2*R*)-2-methoxy-2-phenylethanoyl]amino}-1-piperidinyl)-1-phenylpropyl]cyclobutanecarboxamide
*N*-[(1*S*)-1-Phenyl-3-(4-{[2-(2-pyridinyl)acetyl]amino}-1-piperidinyl)propyl]cyclobutanecarboxamide
1-Acetyl-*N*-[(1*S*)-3-(4-{[2-(4-fluorophenyl)acetyl]amino}-1-piperidinyl)-1-phenylpropyl]-3-azetidinecarboxamide
*N*-[(1*S*)-3-(4-{[2-(4-Fluorophenyl)acetyl]amino}-1-piperidinyl)-1-phenylpropyl]-2,2-dimethylpropanamide
*N*-[(1*S*)-1-(3-Fluorophenyl)-3-(4-{[2-(4-fluorophenyl)acetyl]amino}-1-piperidinyl)propyl]-*N*-methylcyclobutanecarboxamide

SELECTIVITY

In one preferred aspect, the present invention relates to a method of identifying agents that selectively modulate the interaction between gp120 and CCR5.

For some applications, preferably the agent has at least about a 100 fold selectivity to a particular desired target, preferably at least about a 150 fold selectivity to the desired target, preferably at least about a 200 fold selectivity to the desired target, preferably at least about a 250 fold selectivity to the desired target, preferably at least about a 300 fold selectivity to the desired target, preferably at least about a 350 fold selectivity to the desired target, preferably at least about a 400 fold selectivity to the desired target, preferably at least about a 450 fold selectivity to the desired

target, preferably at least about a 500 fold selectivity to the desired target, preferably at least about a 600 fold selectivity to the desired target, preferably at least about a 700 fold selectivity to the desired target, preferably at least about an 800 fold selectivity to the desired target, preferably at least about a 900 fold selectivity to the desired target, preferably at least about a 1000 fold selectivity to the desired target.

## STEREO AND GEOMETRIC ISOMERS

Some of the agents may exist as stereoisomers and/or geometric isomers - e.g. they may possess one or more asymmetric and/or geometric centres and so may exist in two or more stereoisomeric and/or geometric forms. The present invention contemplates the use of all the individual stereoisomers and geometric isomers of those agents, and mixtures thereof. The terms used in the claims encompass these forms, provided said forms retain the appropriate functional activity (though not necessarily to the same degree).

## PHARMACEUTICAL SALT

The agent of the present invention may be administered in the form of a pharmaceutically acceptable salt.

Pharmaceutically-acceptable salts are well known to those skilled in the art, and for example include those mentioned by Berge *et al,* in J.Pharm.Sci., 66, 1-19 (1977). Suitable acid addition salts are formed from acids which form non-toxic salts and include the hydrochloride, hydrobromide, hydroiodide, nitrate, sulphate, bisulphate, phosphate, hydrogenphosphate, acetate, trifluoroacetate, gluconate, lactate, salicylate, citrate, tartrate, ascorbate, succinate, maleate, fumarate, gluconate, formate, benzoate, methanesulphonate, ethanesulphonate, benzenesulphonate and p-toluenesulphonate salts.

When one or more acidic moieties are present, suitable pharmaceutically acceptable base addition salts can be formed from bases which form non-toxic salts and include the aluminium, calcium, lithium, magnesium, potassium, sodium, zinc, and pharmaceutically-active amines such as diethanolamine, salts.

A pharmaceutically acceptable salt of an agent of the present invention may be readily prepared by mixing together solutions of the agent and the desired acid or base, as appropriate. The salt may precipitate from solution and be collected by filtration or may be recovered by evaporation of the solvent.

The agent of the present invention may exisit in polymorphic form.

The agent of the present invention may contain one or more asymmetric carbon atoms and therefore exists in two or more stereoisomeric forms. Where an agent contains an alkenyl or alkenylene group, cis (E) and trans (Z) isomerism may also occur. The present invention includes the individual stereoisomers of the agent and, where appropriate, the individual tautomeric forms thereof, together with mixtures thereof.

Separation of diastereoisomers or cis and trans isomers may be achieved by conventional techniques, e.g. by fractional crystallisation, chromatography or H.P.L.C. of a stereoisomeric mixture of the agent or a suitable salt or derivative thereof. An individual enantiomer of the agent may also be prepared from a corresponding optically pure intermediate or by resolution, such as by H.P.L.C. of the corresponding racemate using a suitable chiral support or by fractional crystallisation of the diastereoisomeric salts formed by reaction of the corresponding racemate with a suitable optically active acid or base, as appropriate.

The present invention also includes all suitable isotopic variations of the agent or a pharmaceutically acceptable salt thereof. An isotopic variation of an agent of the present invention or a pharmaceutically acceptable salt thereof is defined as one in which at least one atom is replaced by an atom having the same atomic number but an atomic mass different from the atomic mass usually found in nature. Examples of isotopes that can be incorporated into the agent and pharmaceutically acceptable salts thereof include isotopes of hydrogen, carbon, nitrogen, oxygen, phosphorus, sulphur, fluorine and chlorine such as $^{2}H$, $^{3}H$, $^{13}C$, $^{14}C$, $^{15}N$, $^{17}O$, $^{18}O$, $^{31}P$, $^{32}P$, $^{35}S$, $^{18}F$ and $^{36}Cl$, respectively. Certain isotopic variations of the agent and pharmaceutically acceptable salts thereof, for example, those in which a radioactive isotope such as $^{3}H$ or $^{14}C$ is incorporated, are useful in drug and/or substrate tissue distribution studies. Tritiated, i.e., $^{3}H$, and carbon-14, i.e., $^{14}C$, isotopes are particularly preferred for their ease of preparation and detectability. Further, substitution with isotopes such as deuterium, i.e., $^{2}H$, may afford certain therapeutic advantages resulting from greater metabolic stability, for example, increased *in vivo* half-life or reduced dosage requirements and hence may be preferred in some circumstances. Isotopic variations of the agent of the present invention and pharmaceutically acceptable salts thereof of this invention can generally be prepared by conventional procedures using appropriate isotopic variations of suitable reagents.

It will be appreciated by those skilled in the art that the agent of the present invention may be derived from a prodrug. Examples of prodrugs include entities that have certain protected group(s) and which may not possess pharmacological activity as such, but may, in certain instances, be administered (such as orally or parenterally) and thereafter metabolised in the body to form the agent of the present invention which are pharmacologically active.

It will be further appreciated that certain moieties known as "pro-moieties", for example as described in "Design

of Prodrugs" by H. Bundgaard, Elsevier, 1985 (the disclosured of which is hereby incorporated by reference), may be placed on appropriate functionalities of the agents. Such prodrugs are also included within the scope of the invention.

The present invention also includes (wherever appropriate) the use of zwitterionic forms of the agent of the present invention.

The terms used in the claims encompass one or more of the forms just mentioned.

SOLVATES

The present invention also includes the use of solvate forms of the agent of the present invention.

PRO-DRUG

As indicated, the present invention also includes the use of pro-drug forms of the agent of the present invention.

CHEMICAL SYNTHESIS METHODS

Typically the agent of the present invention will be prepared by chemical synthesis techniques.

It will be apparent to those skilled in the art that sensitive functional groups may need to be protected and deprotected during synthesis of a compound of the invention. This may be achieved by conventional techniques, for example as described in "Protective Groups in Organic Synthesis" by T W Greene and P G M Wuts, John Wiley and Sons Inc. (1991), and by P.J.Kocienski, in "Protecting Groups", Georg Thieme Verlag (1994).

It is possible during some of the reactions that any stereocentres present could, under certain conditions, be racemised, for example if a base is used in a reaction with a substrate having an having an optical centre comprising a base-sensitive group. This is possible during e.g. a guanylation step. It should be possible to circumvent potential problems such as this by choice of reaction sequence, conditions, reagents, protection/deprotection regimes, etc. as is well-known in the art.

The compounds and salts of the invention may be separated and purified by conventional methods.

Separation of diastereomers may be achieved by conventional techniques, e.g. by fractional crystallisation, chromatography or H.P.L.C. of a stereoisomeric mixture of a compound of formula (I) or a suitable salt or derivative thereof. An individual enantiomer of a compound of formula (I) may also be prepared from a corresponding optically pure intermediate or by resolution, such as by H.P.L.C. of the corresponding racemate using a suitable chiral support or by fractional crystallisation of the diastereomeric salts formed by reaction of the corresponding racemate with a suitably optically active acid or base.

The agent of the present invention or variants, homologues, derivatives, fragments or mimetics thereof may be produced using chemical methods to synthesize the agent in whole or in part. For example, if they are peptides, then peptides can be synthesized by solid phase techniques, cleaved from the resin, and purified by preparative high performance liquid chromatography (e.g., Creighton (1983) Proteins Structures And Molecular Principles, WH Freeman and Co, New York NY). The composition of the synthetic peptides may be confirmed by amino acid analysis or sequencing (e.g., the Edman degradation procedure; Creighton, *supra).*

Syntesis of peptide agents (or variants, homologues, derivatives, fragments or mimetics thereof) can be performed using various solid-phase techniques (Roberge JY *et al* (1995) Science 269: 202-204) and automated synthesis may be achieved, for example, using the ABI 43 1 A Peptide Synthesizer (Perkin Elmer) in accordance with the instructions provided by the manufacturer. Additionally, the amino acid sequences comprising the agent or any part thereof, may be altered during direct synthesis and/or combined using chemical methods with a sequence from other subunits, or any part thereof, to produce a variant agent.

In an alternative embodiment of the invention, the coding sequence of a peptide agent (or variants, homologues, derivatives, fragments or mimetics thereof) may be synthesized, in whole or in part, using chemical methods well known in the art (see Caruthers MH *et al* (1980) Nuc Acids Res Symp Ser 215-23, Horn T *et al* (1980) Nuc Acids Res Symp Ser 225-232).

MIMETIC

The present invention also encompasses mimetics of agents. Here, the term "mimetic" relates to any chemical which includes, but is not limited to, a peptide, polypeptide, antibody or other organic chemical which has the same qualitative activity or effect as a reference agent.

CHEMICAL DERIVATIVE

The term "derivative" or "derivatised" as used herein includes chemical modification of an agent. Illustrative of such chemical modifications would be replacement of hydrogen by a halo group, an alkyl group, an acyl group or an amino group.

CHEMICAL MODIFICATION

In one embodiment of the present invention, the agent may be a chemically modified agent.
The chemical modification of an agent of the present invention may either enhance or reduce hydrogen bonding interaction, charge interaction, hydrophobic interaction, Van Der Waals interaction or dipole interaction between the agent and the target.
In one aspect, the identified agent may act as a model (for example, a template) for the development of other compounds.

RECOMBINANT METHODS

If the agent is or comprisies an amino acid sequence and/or a nucleotide sequence, all or part of the agent may be prepared by recombinant DNA techniques.

THERAPY

The agents identified by the assay method of the present invention may be used as therapeutic agents - i.e. in therapy applications.
The term "therapy" includes curative effects, alleviation effects, and prophylactic effects.
The therapy may be on humans or animals.
If any agent(s) adversely modulate the CCR5/gp120 interaction, then those agent(s) may be useful in the treatment of anti-inflammatory diseases and conditions and in the treatment and prevention of HIV-1 and genetically related retroviral infections.

HIV

In one aspect of the present invention, the assay method is used to identify agents that could be used to treat or prevent the spread or onset of a retroviral infection, especially human immunodeficiency virus (HIV) infection - i.e. treating or preventing HIV infection - and/or as treating or preventing AIDS.
The expressions "treating or preventing AIDS", and "preventing or treating infection by HIV" as used herein are intended to mean the treatment of a wide range of states of HIV infection: AIDS, ARC (AIDS related complex), both symptomatic and asymptomatic, and actual or potential exposure to HIV. The quoted expressions are not intended, however, to be limited to the recited treatments, but rather are contemplated to include all beneficial uses relating to conditions attributable to an AIDS causative agent. For example, the identified agents may be useful in treating infection by HIV after suspected past exposure to HIV by, e.g., blood transfusion, organ transplant, exchange of body fluids, sexual intercourse, bites, needle stick, or exposure to patient blood. In addition, an identified agent may be used for the prevention of infection by HIV and the prevention of AIDS, such as in pre-or post-coital prophylaxis or in the prevention of maternal transmission of the HIV virus to a fetus or a child, whether at the time of birth, during the period of nursing, or in any other manner.
With respect to the spread of HIV infection, it has been shown that for entry into target cells, human immunodeficiency viruses require a chemokine receptor, CCR5 and CXCR-4 among others, as well as the virus's primary receptor CD4. The principal cofactor for entry mediated by the envelope glycoproteins of primary macrophage-tropic strains of HIV-1 is CCR5. Deng, et al., Nature, 381, 661-666 (1996) further discuss aspects of CCR5 mediated HIV entry.
In short, HIV attaches to the CD4 molecule on cells through a region of its envelope protein, gp120, and gp120 is part of a multi-subunit complex, most likely a trimer of gp160, i.e., gp120 + gp41. It is believed that the CD4 binding site on the gp120 of HIV interacts with the CD4 molecule on the cell surface, triggering conformational changes across the trimer, which allow it to bind to another cell-surface receptor, such as CCR5. This in turn enables gp41 to induce fusion with the cell membrane, and entry of the viral core into the cell. In addition, macrophage-tropic HIV and SIV envelope proteins have been shown to induce a signal through CCR5 on CD4+ cells, which may enhance the replication of the virus. See Weissman, et al., Nature, 389, 981-985 (1997) for a description of this phenomenon. Further, it has been shown that a complex of gp120 and soluble CD4 interacts specifically with CCR5 and inhibits the binding of the natural CCR5 ligands, as described in Wu, et al., Nature, 384, 179-183 (1996); and Trkola, et al., Nature, 384, 184-187

(1996). It has further been demonstrated that β-chemokines and related molecules, e.g., (AOP)-RANTES, prevent HIV fusion to the cell membrane and subsequent infection, both in vitro, as described in Dragic, et al., Nature, 381, 667-673 (1996), and in animal models. Finally, absence of CCR5 appears to confer protection from HIV-1 infection, as described in Nature, 382, 668-669 (1996). In particular, an inherited frame-shifting mutation in the CCR5 gene has been shown to abolish functional expression of the gene in vitro, and individuals homozygous for the mutation are apparently not susceptible to HIV infection, while at the same time they do not seem to be immuno-compromised by this variant. Furthermore, those heterozygote individuals that have been infected by HIV progress more slowly to full-blown clinical AIDS. In addition to validating the role of CCR5 in the infectious cycle of HIV, the above observations suggest that CCR5 is dispensable in the adult organism.

Although most HIV-1 isolates studied to date utilize CCR5 or CXCR-4, at least nine other chemokine receptors, or structurally related molecules, have also been described as supporting HIV-1 env-mediated membrane fusion or viral entry in vitro. These include CCR2b, CCR3, BOB/GPR15, Bonzo/STRL33/TYMSTR, GPR1, CCR8, US28, V28/CX3CR1, LTB-4, and APJ. There is good evidence that CCR3 can be used efficiently by a significant fraction of HIV-1 isolates in vitro, provided that this protein is over-expressed in transfected cells. Nevertheless, consistent evidence indicates that anti-HIV drugs targeted to chemokine receptors may not be compromised by this variability. Indeed, the chemokines RANTES, MIP-1α, SDF-1 have been shown to suppress replication of primary HIV isolates. A derivative of RANTES, (AOP)-RANTES, is a sub-nanomolar antagonist of CCR5 function in monocytes. Monoclonal antibodies to CCR5 have been reported to block infection of cells by HIV in vitro. A small molecule antagonist of CXCR4, identified as AMD3100, has been reported to inhibit infection of susceptible cultures by CXCR4 dependent primary and lab-adapted HIV viruses while another small molecule called TAK 779 blocks entry of CCR5-tropic strains (Baba, et al. PNAS, 96 (10), 5698-5703 (1999); In addition, the majority of primary strains from early and late disease stages utilize CCR5 exclusively or in addition to other chemokine receptors, indicating that CCR5 dependent infection may play an essential role in the initiation and maintenance of productive HIV infection in a host. Accordingly, an agent which blocks or adversely modulates CCR5 in patients including mammals, and especially humans who possess normal chemokine receptors, can reasonably be expected to prevent infection in healthy individuals and slow or halt viral progression in infected patients.

ADDITIONAL BIOLOGICAL STUDIES

If desired, the agents identified by the assay method of the present invention can be further investigated using other assay systems.

By way of example, referance may be made to the CCR5 binding assay procedures disclosed in Combadiere et al., J. Leukoc. Biol. 60, 147-52 (1996); and/or intracellular calcium mobilisation assays as described by the same authors. Cell lines expressing the receptor of interest include those naturally expressing the receptor, such as PM-1, or IL-2 stimulated peripheral blood lymphocytes (PBL), or a cell engineered to express a recombinant receptor, such as CHO, 300.19, L1.2 or HEK-293. In particular, the agents may be further investigated to see if they can prevent the binding of further known chemokine ligands to CCR5 In addition, the agents may be studied further to see if they prevent intracellular calcium mobilization in response to endogenous agonists, which is consistent with their functioning as CCR5 antagonists.

Further examples of other assays include those the HIV microculture assays described in Dimitrov et al., J. Clin. Microbiol. 28, 734-737 (1990)), and the pseudotyped HIV reporter assay described in Connor et al., Virology 206 (2) 935-44 (1995). In particular, the agents can be investigated to see if they inhibit p24 production following replication of laboratory-adapted and primary HIV strains in primary blood lymphocytes (PBLs) and clonal cell-lines known to support replication of both CCR5 and CXCR-4 tropic viruses, e.g., PM-1 and MOLT4-clone 8. Furthermore, the agents can be investigated to see if they inhibit entry of chimeric HIV reporter viruses pseudotyped with envelope from a CCR5 dependent strain (ADA). Furthermore, the agents can be investigated to see if they inhibit infection of primary cells by HIV isolated from infected patient blood.

PHARMACEUTICAL COMPOSITIONS

The present invention also provides a pharmaceutical composition comprising administering a therapeutically effective amount of the agent of the present invention and a pharmaceutically acceptable carrier, diluent or excipients (including combinations thereof).

The pharmaceutical compositions may be for human or animal usage in human and veterinary medicine and will typically comprise any one or more of a pharmaceutically acceptable diluent, carrier, or excipient. Acceptable carriers or diluents for therapeutic use are well known in the pharmaceutical art, and are described, for example, in Remington's Pharmaceutical Sciences, Mack Publishing Co. (A. R. Gennaro edit. 1985). The choice of pharmaceutical carrier, excipient or diluent can be selected with regard to the intended route of administration and standard pharmaceutical practice. The pharmaceutical compositions may comprise as - or in addition to - the carrier, excipient or diluent any suitable binder(s), lubricant(s), suspending agent(s), coating agent(s), solubilising agent(s).

Preservatives, stabilizers, dyes and even flavoring agents may be provided in the pharmaceutical composition. Examples of preservatives include sodium benzoate, sorbic acid and esters of p-hydroxybenzoic acid. Antioxidants and suspending agents may be also used.

There may be different composition/formulation requirements dependent on the different delivery systems. By way of example, the pharmaceutical composition of the present invention may be formulated to be administered using a mini-pump or by a mucosal route, for example, as a nasal spray or aerosol for inhalation or ingestable solution, or parenterally in which the composition is formulated by an injectable form, for delivery, by, for example, an intravenous, intramuscular or subcutaneous route. Alternatively, the formulation may be designed to be administered by a number of routes.

Where the agent is to be administered mucosally through the gastrointestinal mucosa, it should be able to remain stable during transit though the gastrointestinal tract; for example, it should be resistant to proteolytic degradation, stable at acid pH and resistant to the detergent effects of bile.

Where appropriate, the pharmaceutical compositions can be administered by inhalation, in the form of a suppository or pessary, topically in the form of a lotion, solution, cream, ointment or dusting powder, by use of a skin patch, orally in the form of tablets containing excipients such as starch or lactose, or in capsules or ovules either alone or in admixture with excipients, or in the form of elixirs, solutions or suspensions containing flavouring or colouring agents, or they can be injected parenterally, for example intravenously, intramuscularly or subcutaneously. For parenteral administration, the compositions may be best used in the form of a sterile aqueous solution which may contain other substances, for example enough salts or monosaccharides to make the solution isotonic with blood. For buccal or sublingual administration the compositions may be administered in the form of tablets or lozenges which can be formulated in a conventional manner.

For some embodiments, the agent of the present invention may also be used in combination with a cyclodextrin. Cyclodextrins are known to form inclusion and non-inclusion complexes with drug molecules. Formation of a drug-cyclodextrin complex may modify the solubility, dissolution rate, bioavailability and/or stability property of a drug molecule. Drug-cyclodextrin complexes are generally useful for most dosage forms and administration routes. As an alternative to direct complexation with the drug the cyclodextrin may be used as an auxiliary additive, e.g. as a carrier, diluent or solubiliser. Alpha-, beta- and gamma-cyclodextrins are most commonly used and suitable examples are described in WO-A-91/11172, WO-A-94/02518 and WO-A-98/55148.

If the agent is a protein, then said protein may be prepared *in situ* in the subject being treated. In this respect, nucleotide sequences encoding said protein may be delivered by use of non-viral techniques (e.g. by use of liposomes) and/or viral techniques (e.g. by use of retroviral vectors) such that the said protein is expressed from said nucleotide sequence.

## PHARMACEUTICAL COMBINATIONS

The agent of the present invention may be administered with one or more other pharmaceutically active substances. By way of example, the present invention covers the simultaneous, or sequential treatments with an agent according to the present invention and one or more inhibitors of HIV protease and/or inhibitors of HIV reverse transcriptase, preferably selected from the class of non-nucleoside reverse transcriptase inhibitors (NNRTI), including but not limited to nevirapine, delavirdine, and efavirenz; from among the nucleoside/nucleotide inhibitors, including but not limited to zidovudine, didanosine, zalcitabine, stavudine, lamivudine, abacavir, and adefovir dipivoxil; and from among the protease inhibitors, including but not limited to indinavir, ritonavir, saquinavir, nelfinavir, and amprenavir. Other agents useful in the above-described preferred embodiment combinations of the present invention include current and to-be-discovered investigational drugs from any of the above classes of inhibitors, including but not limited to FTC, PMPA, fozivudine tidoxil, talviraline, S-1153, MKC-442, MSC-204, MSH-372, DMP450, PNU-140690, ABT-378, and KNI-764.

There is also included within the scope of the preferred embodiments of the present invention, combinations of an agent according to the present invention together with a supplementary therapeutic agent used for the purpose of auxiliary treatment, wherein said supplementary therapeutic agent comprises one or more members independently selected from the group consisting of proliferation inhibitors, e.g., hydroxyurea; immunomodulators, e.g., sargramostim, and various forms of interferon or interferon derivatives; fusion inhibitors, e.g., AMD3100, T-20, PRO-542, AD-349, BB-10010 and other chemokine receptor agonists/antagonists; integrase inhibitors, e.g., AR177; RNaseH inhibitors; inhibitors of viral transcription and RNA replication; and other agents that inhibit viral infection or improve the condition or outcome of HIV-infected individuals through different mechanisms.

For some applications, preferred methods of treatment of the present invention for the prevention of HIV infection, or treatment of aviremic and asymptomatic subjects potentially or effectively infected with HIV, include but

are not limited to administration of a member independently selected from the group consisting of: (i) an agent according to the present invention; (ii) one NNRTI in addition to a compound of (i); (iii) two NRTI in addition to a compound of (i); (iv) one NRTI in addition to the combination of (ii); and (v) a compound selected from the class of protease inhibitors used in place of an NRTI in combinations (iii) and (iv).

For some applications, preferred methods of treatment of the present invention for the therapy of HIV-infected individuals with detectable viremia or abnormally low CD4 counts further include as a member to be selected: (vi) treatment according to (i) above in addition to the standard recommended initial regimens for the therapy of established HIV infections, e.g., as described in Bartlett, J. G., "1998 Medical management of HIV infection", Johns Hopkins University publishers, ISBN 0-9244-2809-0. Such standard regimens include but are not limited to an agent from the class of protease inhibitors in combination with two NRTIs; and (vii) a standard recommended initial regimens for the therapy of established HIV infections, e.g., as described in Bartlett, J. G., "1998 Medical management of HIV infection", Johns Hopkins University publishers, ISBN 0-9244-2809-0), where either the protease inhibitor component, or one or both of the NRTIs is/are replaced by an agent according to the present invention.

For some applications, preferred methods of treatment of the present invention for the therapy of HIV-infected individuals that have failed antiviral therapy further include as a member to be selected: (viii) treatment according to (i) above, in addition to the standard recommended regimens for the therapy of such patients, e.g., as described in Bartlett, J. G., "1998 Medical management of HIV infection", Johns Hopkins University publishers, ISBN 0-9244-2809-0); and (ix) a standard recommended initial regimens for the therapy of patients who have failed antiretroviral therapy, e.g., as described in Bartlett, J. G., "1998 Medical management of HIV infection", Johns Hopkins University publishers, ISBN 0-9244-2809-0), where either one of the protease inhibitor components, or one or both of the NRTIs is/are replaced by an agent according to the present invention.

In the above-described combinations of the present invention, the agent of the present invention and other therapeutic active agents may be administered in terms of dosage forms either separately or in conjunction with each other, and in terms of their time of administration, either serially or simultaneously. Thus, the administration of one component agent may be prior to, concurrent with, or subsequent to the administration of the other component agent(s).

## ADMINISTRATION

The term "administered" includes delivery by viral or non-viral techniques. Viral delivery mechanisms include but are not limited to adenoviral vectors, adeno-associated viral (AAV) vectos, herpes viral vectors, retroviral vectors, lentiviral vectors, and baculoviral vectors. Non-viral delivery mechanisms include lipid mediated transfection, liposomes, immunoliposomes, lipofectin, cationic facial amphiphiles (CFAs) and combinations thereof.

The agent of the present invention may be administered alone but will generally be administered as a pharmaceutical composition - e.g. when the components are is in admixture with a suitable pharmaceutical excipient, diluent or carrier selected with regard to the intended route of administration and standard pharmaceutical practice.

For example, the agent can be administered (e.g. orally or topically) in the form of tablets, capsules, ovules, elixirs, solutions or suspensions, which may contain flavouring or colouring agents, for immediate-, delayed-, modified-, sustained-, pulsed- or controlled-release applications.

If the pharmaceutical is a tablet, then the tablet may contain excipients such as microcrystalline cellulose, lactose, sodium citrate, calcium carbonate, dibasic calcium phosphate and glycine, disintegrants such as starch (preferably corn, potato or tapioca starch), sodium starch glycollate, croscarmellose sodium and certain complex silicates, and granulation binders such as polyvinylpyrrolidone, hydroxypropylmethylcellulose (HPMC), hydroxypropylcellulose (HPC), sucrose, gelatin and acacia. Additionally, lubricating agents such as magnesium stearate, stearic acid, glyceryl behenate and talc may be included.

Solid compositions of a similar type may also be employed as fillers in gelatin capsules. Preferred excipients in this regard include lactose, starch, a cellulose, milk sugar or high molecular weight polyethylene glycols. For aqueous suspensions and/or elixirs, the agent may be combined with various sweetening or flavouring agents, colouring matter or dyes, with emulsifying and/or suspending agents and with diluents such as water, ethanol, propylene glycol and glycerin, and combinations thereof.

The routes for administration (delivery) include, but are not limited to, one or more of: oral (e.g. as a tablet, capsule, or as an ingestable solution), topical, mucosal (e.g. as a nasal spray or aerosol for inhalation), nasal, parenteral (e.g. by an injectable form), gastrointestinal, intraspinal, intraperitoneal, intramuscular, intravenous, intrauterine, intraocular, intradermal, intracranial, intratracheal, intravaginal, intracerebroventricular, intracerebral, subcutaneous, ophthalmic (including intravitreal or intracameral), transdermal, rectal, buccal, vaginal, epidural, sublingual.

It is to be understood that not all of the pharmaceutical, or even all of the components of the pharmaceutical, need be administered by the same route. Likewise, if the composition comprises more than one active component, then those components may be administered by different routes.

If a component of the present invention is administered parenterally, then examples of such administration

include one or more of: intravenously, intra-arterially, intraperitoneally, intrathecally, intraventricularly, intraurethrally, intrasternally, intracranially, intramuscularly or subcutaneously administering the component; and/or by using infusion techniques.

For parenteral administration, the component is best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well-known to those skilled in the art.

As indicated, the agent of the present invention can be administered intranasally or by inhalation and is conveniently delivered in the form of a dry powder inhaler or an aerosol spray presentation from a pressurised container, pump, spray or nebuliser with the use of a suitable propellant, e.g. dichlorodifluoromethane, trichlorofluoromethane, dichlorotetrafluoroethane, a hydrofluoroalkane such as 1,1,1,2-tetrafluoroethane (HFA 134A™) or 1,1,1,2,3,3,3-heptafluoropropane (HFA 227EA™), carbon dioxide or other suitable gas. In the case of a pressurised aerosol, the dosage unit may be determined by providing a valve to deliver a metered amount. The pressurised container, pump, spray or nebuliser may contain a solution or suspension of the active compound, e.g. using a mixture of ethanol and the propellant as the solvent, which may additionally contain a lubricant, e.g. sorbitan trioleate. Capsules and cartridges (made, for example, from gelatin) for use in an inhaler or insufflator may be formulated to contain a powder mix of the agent and a suitable powder base such as lactose or starch.

Alternatively, the agent of the present invention can be administered in the form of a suppository or pessary, or it may be applied topically in the form of a gel, hydrogel, lotion, solution, cream, ointment or dusting powder. The component(s) of the present invention may also be dermally or transdermally administered, for example, by the use of a skin patch. They may also be administered by the pulmonary or rectal routes. They may also be administered by the ocular route. For ophthalmic use, the compounds can be formulated as micronised suspensions in isotonic, pH adjusted, sterile saline, or, preferably, as solutions in isotonic, pH adjusted, sterile saline, optionally in combination with a preservative such as a benzylalkonium chloride. Alternatively, they may be formulated in an ointment such as petrolatum.

For application topically to the skin, the agent of the present invention can be formulated as a suitable ointment containing the active compound suspended or dissolved in, for example, a mixture with one or more of the following: mineral oil, liquid petrolatum, white petrolatum, propylene glycol, polyoxyethylene polyoxypropylene compound, emulsifying wax and water. Alternatively, it can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, cetearyl alcohol, 2-octyldodecanol, benzyl alcohol and water.

For some applications, preferably the agent is administered orally.

## DOSE LEVELS

Typically, a physician will determine the actual dosage which will be most suitable for an individual subject. The specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the individual undergoing therapy.

By way of example, the agents of the present invention may be administered in accordance with a regimen of 1 to 4 times per day, preferably once or twice per day. In particular, however, the treatment of retroviral infections, and more particularly HIV, may be guided by genotyping and phenotyping the virus in the course of or prior to the initiation of administration of the therapeutic agent. In this way, it is possible to optimise dosing regimens and efficacy when administering an agent according to the present invention for the prevention or treatment of infection by a retrovirus, in particular, the human immunodeficiency virus (HIV).

Depending upon the need, the agent may be administered at a dose of from 0.01 to 30 mg/kg body weight, such as from 0.1 to 10 mg/kg, more preferably from 0.1 to 1 mg/kg body weight.

## FORMULATION

The agent of the present invention may be formulated into a pharmaceutical composition, such as by mixing with one or more of a suitable carrier, diluent or excipient, by using techniques that are known in the art.

## PHARMACEUTICALLY ACTIVE SALT

The agent of the present invention may be administered as a pharmaceutically acceptable salt. Typically, a

pharmaceutically acceptable salt may be readily prepared by using a desired acid or base, as appropriate. The salt may precipitate from solution and be collected by filtration or may be recovered by evaporation of the solvent.

ANIMAL TEST MODELS

*In vivo* models may be used to investigate and/or design therapies or therapeutic agents to treat HIV. The models could be used to investigate the effect of various tools/lead compounds on a variety of paramaters which are implicated in the development of ro treatment of HIV. These animal test models can be used in conjunction with the assay of the present invention. The animal test model will be a non-human animal test model.

GENERAL RECOMBINANT DNA METHODOLOGY TECHNIQUES

Although in general the techniques mentioned herein are well known in the art, reference may be made in particular to Sambrook et al., Molecular Cloning, A Laboratory Manual (1989) and Ausubel et al., Short Protocols in Molecular Biology (1999) 4th Ed, John Wiley & Sons, Inc. PCR is described in US-A-4683195, US-A-4800195 and US-A-4965188.

SUMMARY

Thus, in summary, the present invention provides an assay method for determining whether an agent is capable of modulating the interaction of CCR5 with gp120. The assay method comprises incubating the agent with CCR5 and gp120 to form a first reaction mixture; and determining whether said agent modulates the interaction of CCR5 with gp120. In the method, gp120 is associated with CD4. In particular, in the assay method the interaction of CCR5 with gp120 is a low affinity binding.

The present invention also relates to agents identified using said method, as well as pharmaceutical compositions comprising same, as well as methods of therapy using same.

In a preferred embodiment the present invention provides an assay method for determining whether an agent is capable of modulating the interaction of CCR5 with gp120; the method comprising: incubating the agent with CCR5 and gp120 to form a first reaction mixture; and determining whether said agent modulates the interaction of CCR5 with gp120; wherein said gp120 is associated with CD4; wherein said interaction is a low affinity binding; and wherein said method includes the step of adding a ligand to said first reaction mixture to form a second reaction mixture; wherein said ligand is capable of indicating whether said agent has modulated said interaction; and wherein said ligand has a detectable label.

In a highly preferred embodiment the present invention provides an assay method for determining whether an agent is capable of modulating the interaction of CCR5 with gp120; the method comprising: incubating the agent with CCR5 and gp120 to form a first reaction mixture; and determining whether said agent modulates the interaction of CCR5 with gp120; wherein said gp120 is associated with CD4; wherein said interaction is a low affinity binding; and wherein said method includes the step of adding a ligand to said first reaction mixture to form a second reaction mixture; wherein said ligand is capable of indicating whether said agent has modulated said interaction; wherein said ligand has a detectable label; and wherein said ligand comprises at least a first antibody, wherein said first antibody is capable of binding to gp120 and wherein said binding is high affinity binding; and wherein said ligand possibly comprises a second antibody, wherein said second antibody is capable of binding to said first antibody; and wherein said detectable label is associated with said second antibody, or with first antibody if second antibody is not required.

EXAMPLES

The present invention will now be described, by way of examples only. In the Examples the symbol @ means "at about".

## EXAMPLE 1

In accordance with the present invention, TR-FIA (time resolved fluorescence immunoassay) is used to determine IC50 of inhibition of gp120 binding to the receptor CCR-5 on MIP34.10 in 96 well plates.

The principle of this assay is based on competitive inhibition of gp120 binding to the receptor CCR-5 on transformed HEK-293 MIP34.10 cells. Varying concentrations of compounds and chemokines are incubated with a constant amount of the gp120, prior to addition of an anti-gp120 antibody and a secondary $Eu^{3+}$ labelled anti-IgG. The displacement of the gp120/CD4 /antibody/antibody complex is measured by time resolved fluorescence (TRF) in a DELFIA Counter and % inhibitions are calculated using XL software.

**MATERIALS AND METHODS :**

**Cell Culture**

MIP34.10 cells supplied by Cell Biology and continued passage within the laboratory. PBS (Dulbecco's) without $Ca^{2+}$ and $Mg^{2+}$ - HyQ Reagents,HyClone (cat no : B-4004-L) 1x Cell Dissociation Solution non-enzymatic - Sigma (cat no : C-5914)

**Growth medium**

500ml 1x Dulbecco's Modified Eagle's Medium (DMEM) with 3.7g/l sodium bicarbonate without L-glutamine - HyQ Reagents, HyClone (cat no : B-7501-L) 50ml foetal calf serum (FCS) - PAA Laboratories, Austria (cat no : A15-041) 5ml 200mM L-Glutamine - Gibco BRL (cat no : 25030-024) 5ml Penicillin/Streptomycin (100U/ml Pen/10mg/ml Strep) - Sigma (cat no : P-7539) 6.5ml 50mg/ml Geneticin - Gibco BRL (cat no : 10131-019)

162cm2 Cell Culture Flask Tissue Culture Treated - Costar (cat no : 3151) Incubator set @ 37°C, 5% CO2 humidified

**Labelling reagents**

Labelling Buffer:

50mM NaHCO3 pH 8.5 - Sigma (cat no : S6014)
0.9% NaCl - Sigma (cat no : S5150)

Elution Buffer:

50mM Tris-HCl pH 7.8 - Sigma (cat no : T2913)
0.9% NaCl - Sigma (cat no : S5150)
0.05% NaN3- Sigma (cat no : S8032)

Donkey anti-sheep IgG - Sigma (cat no: S2763)
Delfia Eu-labelling kit - EG&G Wallac (cat no : 1244-302)
PD-10 Sephadex G-25 pre-packed columns - Amersham Pharmacia (cat no : 17-0851-01)
UV detector and tubing
Collection tubes
Spectrophotometer

**Assay Reagents**

Dilution / Wash 1 / Assay Buffer
500ml 1x Dulbecco's Modified Eagle's Medium (DMEM) with 3.7g/l sodium bicarbonate without L-glutamine - HyQ Reagents, HyClone (cat no : B-7501-L)
50ml foetal calf serum (FCS) - PAA Laboratories, Austria (cat no : A15-041)

Wash 2 Buffers :

1) Wash concentrate (x25) - EG&G Wallac (cat no : 1380-0865/R) dilute 1:25 with double distilled water
Dulbecco's PBS - Gibco BRL (cat no : 14190-094)
Enhancement
    Enhancement solution - EG&G Wallac (cat no : 1244-104)
sCD4 (human recombinant T-cell) - ImmunoDiagnostics Inc (cat no : 7001)
M-Tropic BaL sgp120
Sheep anti-HIV-1 gp120 antibody (anti C terminal) - Aalto Bio Reagents Ltd, Ireland
(cat no : D7324)
Eu3+ labelled donkey anti-sheep IgG
MIP-1 a, b or RANTES - R & D Systems (cat no : 270-LD-010, 271-BM-010 and 278-RN-010)
DMSO tissue culture grade - Sigma (cat no : D-2650)
Biocoat Cell Environments Poly-D-Lysine 96-well black/clear plates - Becton Dickinson

(cat no : 6640)
Haemocytometer Counting Chamber
Multichannel Pipettes - Labsystems Finnpipette (cat no : 4510-000/020/030/040/050) Pipette Tips - Radleys ABT Aerosol Barrier Tips (cat no : ABT-20/100/2000/1000) Reagent Reservoirs for multichannel pipettes - Costar (cat no : 4870) DELFIA Fluorometer (cat no : 1234-001)

*Cell Culture*

MIP34.10 cells supplied by Cell Biology are cultured in $162cm^2$ cell culture flasks to a confluency between 50-70% in the above growth medium @ 37°C for 2-3 days in the humidified incubator.
The cells are grown at low density because of their clumpy nature and a tendency to lose their receptor at full confluency.
For cell passage, 5ml of cell dissociation solution (non-enzymatic) is added to a 1x washed (PBS) 162cm2 flask of cells, the flask rapped on the side to dislodge the cells and split according to usage, usually 1:5 or 1:10 will give sufficient cells for use within 2 -4 days.
Each 162cm2 flask used for the TR-FIA assay is washed once in 20ml PBS and 5ml cell dissociation solution (non-enzymatic) added, the flask is again rapped on the side to dislodge the cells.
The cells are placed into a 50ml centrifuge tube and 10-20ml assay buffer1 (care should be taken not to overdilute or the cells will need to be spun down and resuspended in a smaller volume) is added into the flask to wash any residual cells out and then combined in the centrifuge tube.
The cells are gently shaken and then counted in a haemocytometer (do appropriate dilutions to enable a count).
Dilute the cells to a density of 1x106 cells/ml and plate 100mM into every well of the poly-D-Lysine plates and incubate overnight @ 37°C in the humidified incubator.

*$Eu^{3+}$ Antibody Preparation*

Add 500ml of labelling buffer to the anti-sheep IgG to solubilize and transfer to the Eu-labelling reagent vial, mix thoroughly and incubate overnight at 28°C
To purify, equilibrate a PD-10 column with 3x the void volume of elution buffer, add the reaction mix and rinse the labelling vial with a small volume of elution buffer and elute.
Monitor the eluate by UV absorbance at 280nm, collect 1-2ml fractions.
Pool the protein peak fractions and measure the $Eu^{3+}$ concentration.
$Eu^{3+}$ Content: serially dilute above with enhancement solution from kit (1 E-04 - 1 E-06) and compare to the fluorescence of 1 nM $Eu^{3+}$ standard supplied in the kit.
Protein Content: calculated from the measured absorbance at 280nm after subtracting the absorbance of the formed aromatic thiourea bonds.
Calculations

$$Eu^{3+}\ mM = (Eu\ counts \times dilution\ factor) / (1000 \times counts\ of\ Eu\ standard)$$

$$Protein\ mg/ml = (Abs\ (280) - 0.008 \times Eu\ mM) / 1.34$$

$$Protein\ mM = (protein\ mg/ml \times 1000000) / 160000$$

$$Yield = Eu\ mM / protein\ mM$$

$$Recovery = 100 \times protein\ mg/ml \times volume\ of\ fraction\ ml / protein\ added\ mg$$

*Compound Preparation*

Solubilize 1mg compound in DMSO to a final concentration of 10mM.
2) Mix well to solubilize compound, Vortex for 10 seconds or sonibath for 2-5 minutes if necessary. Make a

record of compound solubility.

Prepare a dilution series in assay buffer as necessary down at least 6 dilutions (recommended starting concn of 1(M).

Chemokines, if used. MIP-1 α, MIP-1 β and RANTES are prepared in assay buffer alone. These are sticky proteins and care must be taken on preparing these. 10(g of lyophilised material is resuspended to give a final concentration of 10(M (sonibath for 2 minutes) and dilution series prepared from this from 200nM down 6 three-fold dilutions. SP3 special tips are used for all dilutions (ABT aerosol barrier - see assay reagents above).

*Assay Protocol*

Add an equal mix of 36nM sCD4 (100mg/ml solution diluted 1/40 in assay buffer) and 60nM gp120 (1/120 of 1mg/ml) and incubate on ice for 15 minutes.

2) Wash the overnight plates in 100(l wash 1 buffer/well once.

Add 20(l compound or control chemokine except for the background control which is replaced with 40(l assay buffer.

Add 20(l gp120 to each background control well and add 40(l of the sCD4/gp120 complex to each well with compound and reaction control except for the background control wells.

Each mixture contains :

| | |
|---|---|
| Reaction control | 20(l assay buffer |
| | 40(l gp120/sCD4 complex |
| Compound reaction | 20(l compound dilution |
| | 40(l gp120/sCD4 complex |
| Standard reaction | 20(l MIP-1 α, MIP-1 β or RANTES dilution |
| | 40(l gp120/sCD4 complex |
| Background control | 40(l binding buffer |
| | 20(l gp120 |

Incubate the 96 well plates @37° C on a rocking platform for 60 minutes.

Empty the plate, blot and wash with 100(l/well wash 1 buffer once.

7) Add 100(l/well of a 1/50 dilution of the sheep anti-HIV-1 gp120 antibody to each well and incubate @RT for 90 minutes with rocking.

8) Empty the plate, blot and wash with 100(l/well wash 1 buffer once.

9) Add 100(l/well of a 1/1000 dilution of the Eu3+ labelled donkey anti-sheep IgG antibody to each well and incubate @RT for 15 minutes with rocking.

10) Empty the plate, blot and wash with 100(l/well wash 2 buffer once.

Empty the plate, blot and wash with 100(l/well PBS buffer 2x.

12) Empty the plate, blot and add 200(l/well enhancement solution and vortex for 2-3 minutes.

13) The plates are read in a DELFIA 1234 Fluorometer, plates should be read between 15 and 60 minutes.

*Data Analysis*

All calculations are performed in Microsoft Excel or Genesis (Lab Systems version 2.12).

1) Averages and standard deviations of the replicates are computed, along with CVs.

5) An IC50 value is generated by a four point logistic sigmoid curve fit using XL software.

**EXAMPLE 2**

In accordance with the present invention, TR-FIA (time resolved fluorescence immunoassay) is used to determine $IC_{50}$ of inhibition of gp120 binding to the receptor CCR-5 on MIP34.10 in 96 or 384 well plates.

The principle of this assay is based on competitive inhibition of gp120 binding to the receptor CCR-5 on transformed HEK 293 cells MIP34.10 cells. Varying concentrations of compounds and chemokines are incubated with a constant amount of the gp120, prior to addition of an $Eu^{3+}$ labelled anti-gp120 antibody. The displacement of the gp120 / CD4 / antibody complex is measured by time resolved fluorescence (TRF) in a DELFIA Counter and % inhibitions are calculated using XL software.

**MATERIALS AND METHODS :**

Cell Culture

CCR5 transfected HEK293 cells supplied by Cell Biology and continued passage within the laboratory.
PBS (Dulbecco's) without $Ca^{2+}$ and $Mg^{2+}$ - HyQ Reagents, HyClone (cat no : B-4004-L)
1x Cell Dissociation Solution non-enzymatic - Sigma (cat no : C-5914)

Growth medium

500ml 1x Dulbecco's Modified Eagle's Medium (DMEM) with 3.7g/l sodium bicarbonate without L-glutamine - HyQ Reagents, HyClone (cat no : B-7501-L)
50ml foetal calf serum (FCS) - PAA Laboratories, Austria (cat no : A15-041) 5ml 200mM L-Glutamine - Gibco BRL (cat no : 25030-024)
5ml Penicillin/Streptomycin (100U/ml Pen/10mg/ml Strep) - Sigma (cat no : P-7539)
6.5ml 50mg/ml Geneticin - Gibco BRL (cat no : 10131-019)

162cm$^2$ Cell Culture Flask Tissue Culture Treated - Costar (cat no : 3151)
Incubator set @ 37°C, 5% $CO_2$ humidified

Labelling reagents

Labelling Buffer :

50mM $NaHCO_3$ pH 8.5 - Sigma (cat no : S6014)
0.9% NaCl - Sigma (cat no : S5150)

Elution Buffer :

50mM Tris-HCl pH 7.8 - Sigma (cat no : T2913)
0.9% NaCl - Sigma (cat no : S5150)
0.05% $NaN_3$- Sigma (cat no : S8032)

Delfia Eu-labelling kit - EG&G Wallac (cat no : 1244-302)
PD-10 Sephadex G-25 pre-packed columns - Amersham Pharmacia (cat no : 17-0851-01) UV detector and tubing
Collection tubes
Spectrophotometer
Time Resolved Fluorescence reader (DELFIA 1234 or Victor - EG&G Wallac)

Assay Reagents

Dilution / Wash 1 / Assay Buffer :

500ml 1x Dulbecco's Modified Eagle's Medium (DMEM) with 3.7g/l sodium bicarbonate without L-glutamine - HyQ Reagents, HyClone (cat no : B-7501-L)
50ml foetal calf serum (FCS) - PAA Laboratories, Austria (cat no : A15-041)

Wash 2 Buffer :
Dulbecco's PBS - Gibco BRL (cat no : 14190-094)
Enhancement :

Enhancement solution - EG&G Wallac (cat no : 1244-104)
sCD4 (human recombinant T-cell) - ImmunoDiagnostics Inc (cat no : 7001)
M-Tropic BaL sgp120
$Eu^{3+}$ labelled Affinity purified Sheep anti-HIV-1 gp120 antibody (anti C terminal) - Aalto Bio Reagents Ltd, Ireland (cat no : D7324)

MIP-1 α, β or RANTES - R & D Systems (cat no : 270-LD-010, 271-BM-010 and 278-RN-010)
DMSO tissue culture grade - Sigma (cat no : D-2650)
Biocoat Cell Environments Poly-D-Lysine 96-well black/clear plates - Becton Dickinson (cat no : 6640)
Haemocytometer Counting Chamber
Multichannel Pipettes - Labsystems Finnpipette (cat no : 4510-000/020/030/040/050)
Pipette Tips - Radleys ABT Aerosol Barrier Tips (cat no : ABT-20/100/2000/1000)
Reagent Reservoirs for multichannel pipettes - Costar (cat no : 4870)
DELFIA Fluorometer (cat no : 1234-001)

**Procedure**

Cell Culture

1) MIP34.10 cells are cultured in 162cm$^2$ cell culture flasks to a confluency between 50-70% in the above growth medium @ 37°C for 2-3 days in the humidified incubator.

2) The cells are grown at low density because of their clumpy nature and a tendency to lose their receptor at full confluency.

3) For cell passage, 5ml of cell dissociation solution (non-enzymatic) is added to a 1x washed (PBS) 162cm$^2$ flask of cells, the flask rapped on the side to dislodge the cells and split according to usage, usually 1:5 or 1:10 will give sufficient cells for use within 2 -4 days.

4) Each 162cm$^2$ flask used for the TR-FIA assay is washed once in a small volume of growth media and 5-10ml growth media added, the flask is rapped on the side to dislodge the cells.

5) The cells are placed into a 50ml centrifuge tube and a further 5-10ml growth media is added into the flask to wash any residual cells out and then combined in the centrifuge tube. (Care should be taken not to overdilute or the cells will need to be spun down and resuspended in a smaller volume)

6) The cells are gently shaken and then counted in a haemocytometer (do appropriate dilutions to enable a count).

7) Dilute the cells to a density of 1x10$^6$ cells/ml and plate 100μl into every well of the poly-D-Lysine plates and incubate overnight @ 37°C in the humidified incubator.

$Eu^{3+}$ Antibody Preparation

1) Add 1ml of labelling buffer to 2mg of the sheep anti-HIV-1 gp120 IgG (as supplied - lyophilised) to solubilize and transfer 500μl to each Eu-labelling reagent vial (maximum of 1mg of protein / 0.2mg europium labelling reagent), mix thoroughly and incubate overnight at RT. Care should be taken to ensure that any protein supplied must be free of any amines (buffers such as tris, glycine, HEPES, MOPS, BICINE, etc) or azide. Purification must be undertaken before labelling

2) To purify, equilibrate 2 PD-10 columns with 3x the void volume of elution buffer, add 500μl of the reaction mix to each and rinse the labelling vial with a small volume of elution buffer and elute with the elution buffer.

3) Monitor the eluate by UV absorbance at 280nm, collect 0.5ml fractions.

4) Pool the protein peak fractions, repeat steps 2 and 3 if necessary and measure the $Eu^{3+}$ concentration.

5) **$Eu^{3+}$ Content :** serially dilute above with enhancement solution from kit (1E-04 - 1E-06) and compare to the fluorescence of 1nM $Eu^{3+}$ standard supplied in the kit.

6) **Protein Content:** calculated from the measured absorbance at 280nm after subtracting the absorbance of the formed aromatic thiourea bonds.

7) **Calculations**

$$Eu^{3+}\ \mu M = (Eu\ counts \times dilution\ factor) / (1000 \times counts\ of\ Eu\ standard)$$

$$Protein\ mg/ml = (Abs\ (280) - 0.008 \times Eu\ \mu M) / 1.34$$

$$Protein\ \mu M = (protein\ mg/ml \times 1000000) / 160000$$

$$Yield = Eu\ \mu M / protein\ \mu M$$

$$Recovery = 100 \times protein\ mg/ml \times volume\ of\ fraction\ ml / protein\ added\ mg$$

**Compound Preparation**

1) Solubilize 1mg compound in DMSO to a final concentration of 1mM.

2) Mix well to solubilize compound, Vortex for 10 seconds or sonibath for 2-5 minutes if necessary. Make a record of compound solubility.

3) Prepare a dilution series in assay buffer as necessary down at least 12 dilutions (recommended starting conc$^{\underline{n}}$ of 1μM).

4) Chemokines, if used. MIP-1 α, MIP-1β and RANTES are prepared in assay buffer alone. 10μg of lyophilised material is resuspended to give a final concentration of 10μM (sonibath for 2 minutes) and dilution series prepared from this from 200nM down 6 three-fold dilutions. SP3 special tips should be used for all dilutions (ABT aerosol barrier - see assay reagents above).

**Assay Protocol**

1) Wash the overnight plates in 100μl/well wash 1 buffer once.

2) Add 20μl relevant compound dilutions (3000nM to 1.47nM(8 dilutions,one in three ) - starting concentration of 1000nM in assay - 0.49nM) to each well except for the background control which is replaced with 20μl 1/10 gp120. 20μl assay buffer is used for the control reaction.

3) Mix an equal amount of 36nM sCD4 (100μg/ml solution diluted 1/40 in assay buffer) and gp120 (1/10 dilution of current [Jan 99] crude supernatant stock of BaL gp120) sufficient for number of plates within the assay (2ml of each / per plate).

4) Add 40μl of the sCD4/gp120 complex to each well with compound and reaction control. Add 40μl of assay buffer to the gp120 background.

| Each mixture contains : | |
|---|---|
| Reaction control | 20µl assay buffer |
| | 40µl sCD4/gp120 complex |
| Compound reaction | 20µl compound dilution |
| | 40µl sCD4/gp120 complex |
| Background control | 20µl gp120 |
| | 40µl assay buffer |

5) Incubate the plates @37°C in 5% $CO_2$ on a rocking platform for lhr.

6) Empty the plate, blot and wash once with 100µl/well wash 1 buffer.

7) Empty the plate, blot and add 50µl/well 1/2000 dilution of the $Eu^{3+}$ labelled Affinity purified Sheep anti-HIV-1 gp120 (Dec 99 or Jan 00) and incubate on a rocking platform for 1hr at RT (Finland batch $Eu^{3+}$ labelled Affinity purified Sheep anti-HIV-1 gp120 - 1/500 dilution).

8) Empty the plate, blot and wash with 100µl/well wash 2 buffer 3 times.

9) Empty the plate, blot and add 100µl/well enhancement solution and vortex for 2-3 minutes.

10) The plates are read in a DELFIA 1234 Fluorometer or Victor.

**Data Analysis**

All calculations are performed in Microsoft Excel or Genesis (Lab Systems version 2.12).

1) Averages and standard deviations of the replicates are computed, along with CVs.

2) An $IC_{50}$ value is generated by a four point logistic sigmoid curve fit using XL software.

**COMPOUNDS TESTED**

Compounds of the type described above were tested in accordance with the present invention and were found to be effective in accordance with the present invention - i.e. they can modulate the interaction of CCR5 with gp120. In addition, we found that activity in the described assay correlates well with potency against HIV in microculture assays referenced above.

**SEQUENCE LISTINGS**

Sequence listings, which are part of the description, are presented after the claims.

All publications mentioned in the above specification are herein incorporated by reference. Various modifications and variations of the described methods and system of the present invention will be apparent to those skilled in the art without departing from the scope and spirit of the present invention. Although the present invention has been described in connection with specific preferred embodiments, it should be understood that the invention as claimed should not be unduly limited to such specific embodiments. Indeed, various modifications of the described modes for carrying out the invention which are obvious to those skilled in biochemistry and biotechnology or related fields are intended to be within the scope of the following claims.

REFERENCES FOR CCR5 SECTION

Alkhatib, G.; Combadiere, C.; Broder, C. C.; Feng, Y.; Kennedy, P. E.; Murphy, P. M.; Berger, E. A. : CC CKR5: a RANTES, MIP-1-α, MIP-1-β receptor as a fusion cofactor for macrophage-tropic HIV-1. Science 272: 1955-1958, 1996.

Alvarez, V.; Lopez-Larrea, C.; Coto, E. : Mutational analysis of the CCR5 and CXCR4 genes (HIV-1 co-receptors) in resistance to HIV-1 infection and AIDS development among intravenous drug users. Hum. Genet. 102: 483-486, 1998.

Ansari-Lari, M. A.; Liu, X.-M.; Metzker, M. L.; Rut, A. R.; Gibbs, R. A. : The extent of genetic variation in the CCR5 gene. (Letter) Nature Genet. 16: 221-222, 1997.

Biti, R.; Ffrench, R.; Young, J.; Bennetts, B.; Stewart, G.; Liang, T. : HIV-1 infection in an individual homozygous for the CCR5 deletion allele. (Letter) Nature Med. 3: 252-253, 1997.

Browning, J.; Horner, J. W.; Pettoello-Mantovani, M.; Raker, C.; Yurasov, S.; DePinho, R. A.; Goldstein, H. : Mice transgenic for human CD4 and CCR5 are susceptible to HIV infection. Proc. Nat. Acad. Sci. 94: 14637-14641, 1997.

Carrington, M.; Dean, M.; Martin, M. P.; O'Brien, S. J. : Genetics of HIV-1 infection: chemokine receptor CCR5 polymorphism and its consequences. Hum. Molec. Genet. 8: 1939-1945, 1999.

Choe, H.; Farzan, M.; Sun, Y.; Sullivan, N.; Rollins, B.; Ponath, P. D.; Wu, L.; Mackay, C. R.; LaRosa, G.; Newman, W.; Gerard, N.; Gerard, C.; Sodroski, J. : The β-chemokine receptors CCR3 and CCR5 facilitate infection by primary HIV-1 isolates. Cell 85: 1135-1148, 1996.

Cohen, O. J.; Vaccarezza, M.; Lam, G. K.; Baird, B. F.; Wildt, K.; Murphy, P. M.; Zimmerman, P. A.; Nutman, T. B.; Fox, C. H.; Hoover, S.; Adelsberger, J.; Baseler, M.; and 14 others : Heterozygosity for a defective gene in CC chemokine receptor 5 is not the sole determinant for the immunologic and virologic phenotype of HIV-infected long-term nonprogressors. J. Clin. Invest. 100:1581-1589, 1997. Dean, M.; Carrington, M.; Winkler, C.; Huttley, G. A.; Smith, M. W.; Allikmets, R.; Goedert, J. J.; Buchbinder, S. P.; Vittinghoff, E.; Gomperts, E.; Donfield, S.; Vlahov, D.; Kaslow, R.; Saah, A.; Rinaldo, C.; Detels, R.; Hemophilia Growth and Development Study; Multicenter AIDS Cohort Study; Multicenter Hemophilia Cohort Study; San Francisco City Cohort; ALIVE Study; O'Brien, S. J. : Genetic restriction of HIV-1 infection and progression to AIDS by a deletion allele of the CKR5 structural gene. Science 273: 1856-1861, 1996.

Deng, H.; Liu, R.; Ellmeier, W.; Choe, S.; Unutmaz, D.; Burkhart, M.; Di Marzio, P.; Marmon, S.; Sutton, R. E.; Hill, C. M.; Davis, C. B.; Peiper, S. C.; Schall, T. J.; Littman, D. R.; Landau, N. R. : Identification of a major co-receptor for primary isolates of HIV-1. Nature 381: 661-666, 1996.

Dragic, T.; Litwin, V.; Allaway, G. P.; Martin, S. R.; Huang, Y.; Nagashima, K. A.; Cayanan, C.; Maddon, P. J.; Koup, R. A.; Moore, J. P.; Paxton, W. A. : HIV-1 entry into CD4(+) cells is mediated by the chemokine receptor CC-CKR-5. Nature 381: 667-673, 1996.

Farzan, M.; Mirzabekov, T.; Kolchinsky, P.; Wyatt, R.; Cayabyab, M.; Gerard, N. P.; Gerard, C.; Sodroski, J.; Choe, H. : Tyrosine sulfation of the amino terminus of CCR5 facilitates HIV-1 entry. Cell 96: 667-676, 1999.

Huang, Y.; Paxton, W. A.; Wolinsky, S. M.; Neumann, A. U.; Zhang, L.; He, T.; Kang, S.; Ceradini, D.; Jin, Z.; Yazdanbakhsh, K.; Kunstman, K.; Erickson, D.; Dragon, E.; Landau, N. R.; Phair, J.; Ho, D. D.; Koup, R. A. : The role of a mutant CCR5 allele in HIV-1 transmission and disease progression. Nature Med. 2: 1240-1243, 1996.

Husain, S.; Goila, R.; Shahi, S.; Banerjea, A. C. : First report of a healthy Indian heterozygous for delta-32 mutant of HIV-1 co-receptor-CCR5 gene. Gene 207: 141-147, 1998.

Libert, F.; Cochaux, P.; Beckman, G.; Samson, M.; Aksenova, M.; Cao, A.; Czeizel, A.; Claustres, M.; de la Rua, C.; Ferrari, M.; Ferrec, C.; Glover, G.; Grinde, B.; Guran, S.; Kucinskas, V.; Lavinha, J.; Mercier, B.; Ogur, G.; Peltonen, L.; Rosatelli, C.; Schwartz, M.; Spitsyn, V.; Timar, L.; Beckman, L.; Parmentier, M.; Vassart, G. : The delta-ccr5 mutation conferring protection against HIV-1 in Caucasian populations has a single and recent origin in northeastern Europe. Hum. Molec. Genet. 7: 399-406, 1998.

Liu, R.; Paxton, W. A.; Choe, S.; Ceradini, D.; Martin, S. R.; Horuk, R.; MacDonald, M. E.; Stuhlmann, H.; Koup, R. A.; Landau, N. R. : Homozygous defect in HIV-1 coreceptor accounts for resistance of some multiply-exposed individuals to HIV-1 infection. Cell 86: 367-377, 1996.

Martin, M. P.; Dean, M.; Smith, M. W.; Winkler, C.; Gerrard, B.; Michael, N. L.; Lee, B.; Doms, R. W.; Margolick, J.; Buchbinder, S.; Goedert, J. J.; O'Brien, T. R.; Hilgartner, M. W.; Vlahov, D.; O'Brien, S. J.; Carrington, M. : Genetic acceleration of AIDS progression by a promoter variant of CCR5. Science 282: 1907-1911, 1998.

Martinson, J. J.; Chapman, N. H.; Rees, D. C.; Liu, Y.-T.; Clegg, J. B. : Global distribution of the CCR5 gene 32-basepair deletion. Nature Genet. 16: 100-103, 1997.

McDermott, D. H.; Zimmerman, P. A.; Guignard, F.; Kleeberger, C. A.; Leitman, S. F.; Murphy, P. M. : CCR5 promoter polymorphism and HIV-1 disease progression: multicenter AIDS cohort study (MACS). Lancet 352: 866-870, 1998.

Quillent, C.; Oberlin, E.; Braun, J.; Rousset, D.; Gonzalez-Canali, G.; Metais, P.; Montagnier, L.; Virelizier, J.-L.; Arenzana-Seisdedos, F.; Beretta, A. : HIV-1-resistance phenotype conferred by combination of two separate inherited mutations of CCR5 gene. Lancet 351: 14-18, 1998.

Rottman, J. B.; Ganley, K. P.; Williams, K.; Wu, L.; Mackay, C. R.; Ringler, D. J. : Cellular localization of the chemokine receptor CCR5: correlation to cellular targets of HIV-1 infection. Am. J. Path. 151: 1341-1351, 1997.

Samson, M.; Labbe, O.; Mollereau, C.; Vassart, G.; Parmentier, M. : Molecular cloning and functional expression of a new human CC-chemokine receptor gene. Biochemistry 35: 3362-3367, 1996.

Samson, M.; Libert, F.; Doranz, B. J.; Rucker, J.; Liesnard, C.; Farber, C.-M.; Saragosti, S.; Lapoumeroulie, C.; Cogniaux, J.; Forceille, C.; Muyldermans, G.; Verhofstede, C.; Burtonboy, G.; Georges, M.; Imai, T.; Rana, S.; Yi, Y.; Smyth, R. J.; Collman, R. G.; Doms, R. W.; Vassart, G.; Parmentier, M. : Resistance to HIV-1 infection in Caucasian individuals bearing mutant alleles of the CCR-5 chemokine receptor gene. Nature 382: 722-725, 1996.

Smith, M. W.; Dean, M.; Carrington, M.; Winkler, C.; Huttley, G. A.; Lomb, D. A.; Goedert, J. J.; O'Brien, T. R.; Jacobson, L. P.; Kaslow, R.; Buchbinder, S.; Vittinghoff, E.; Vlahov, D.; Hoots, K.; Hilgartner, M. W.; Hemophilia Growth and Development Study (HGDS); Multicenter AIDS Cohort Study (MACS); Multicenter Hemophilia Cohort Study (MHCS); San Francisco City Cohort (SFCC); ALIVE Study; O'Brien, S. J. : Contrasting genetic influence of CCR2 and CCR5 variants on HIV-1 infection and disease progression. Science 277: 959-965, 1997.

Stephens, J. C.; Reich, D. E.; Goldstein, D. B.; Shin, H. D.; Smith, M. W.; Carrington, M.; Winkler, C.; Huttley, G. A.; Allikmets, R.; Schriml, L.; Gerrard, B.; Malasky, M.; and 27 others : Dating the origin of the CCR5-de132 AIDS-resistance allele by the coalescence of haplotypes. Am. J. Hum. Genet. 62: 1507-1515, 1998.

Zagury, D.; Lachgar, A.; Chams, V.; Fall, L. S.; Bernard, J.; Zagury, J.-F.; Bizzini, B.; Gringeri, A.; Santagostino, E.; Rappaport, J.; Feldman, M.; O'Brien, S. J.; Burny, A.; Gallo, R. C. : C-C chemokines, pivotal in protection against HIV type 1 infection. Proc. Nat. Acad. Sci. 95: 3857-3861, 1998.

Zimmerman, P. A.; Buckler-White, A.; Alkhatib, G.; Spalding, T.; Kubofcik, J.; Combadiere, C.; Weissman, D.; Cohen, O.; Rubbert, A.; Lam, G.; Vaccarezza, M.; Kennedy, P. E.; Kumaraswami, V.; Giorgi, J. V.; Detels, R.; Hunter, J.; Chopek, M.; Berger, E. A.; Fauci, A. S.; Nutman, T. B.; Murphy, P. M. : Inherited resistance to HIV-1 conferred by an inactivating mutation in CC chemokine receptor 5: studies in populations with contrasting clinical phenotypes, defined racial background, and quantified risk. Molec. Med. 3: 23-26, 1997.

REFERENCES FOR CD4 SECTION

Amadori, A.; Zamarchi, R.; De Silvestro, G.; Forza, G.; Cavatton, G.; Danieli, G. A.; Clememti, M.; Chieco-Bianchi, L. : Genetic control of the CD4/CD8 T-cell ratio in humans. Nature Med. 1: 1279-1283, 1995.

Chakravarti, A. : The CD4/CD8 ratio: message in a bottle? Nature Med. 1: 1240-1241, 1995.

Clementi, M.; Forabosco, P.; Amadori, A.; Zamarchi, R.; De Silvestro, G.; Di Gianantonio, E.; Chieco-Bianchi, L.; Tenconi, R. : CD4 and CD8 T lymphocyte inheritance: evidence for major autosomal recessive genes. Hum. Genet. 105: 337-342, 1999.

Kraal, G.; Weissman, I. L.; Butcher, E. C. : Genetic control of T-cell subset representation in inbred mice. Immunogenetics. 18: 585-592, 1983.

## SEQUENCE LISTINGS (PART OF THE DESCRIPTION)

**CCR5**

```
ID   T90117 standard; cDNA; 1477 BP.
DE   cDNA for human CCR5.
KW   Human Cys-Cys chemokine receptor 5; CCR5; human immunodeficiency virus;
type 1; type 2; HIV-1; HIV-2; diagnosis; treatment; prevention; inflammatory
disease; rheumatoid arthritis; glomerulonephritis; asthma; idiopathic
pulmonary fibrosis; psoriasis; viral infection; cancer; atherosclerosis;
autoimmune disorder; ss.
OS   Homo sapiens.
FH   Key             Location/Qualifiers
FT   CDS             240..1298

SQ   Sequence 1477 BP; 374 A; 349 C; 320 G; 431 T; 3 other

     gaattccccc aacagagcca agctctccat ctagtggaca gggaagctag cagcaaacct
60
     tcccttcact acaaaacttc attgcttggc caaaaagaga gttaattcaa tgtagacatc
120
     tatgtaggca attaaaaacc tattgatgta taaaacagtt tgcattcatg gagggcaact
180
     aaatacattc taggacttta taaaagatca ctttttattt atgcacaggg tggaacaaga
240
     tggattatca agtgtcaagt ccaatctatg acatcaatta ttatacatcg gagccctgcc
300
     aaaaaatcaa tgtgaagcaa atcgcagccc gcctcctgcc tccgctctac tcactggtgt
360
     tcatctttgg ttttgtgggc aacatgctgg tcatcctcat cctgataaac tgcaaaaggc
420
     tgaagagcat gactgacatc tacctgctca acctggccat ctctgacctg tttttccttc
480
     ttactgtccc cttctgggct cactatgctg ccgcccagtg ggactttgga aatacaatgt
540
     gtcaactctt gacagggctc tattttatag gcttcttctc tggaatcttc ttcatcatcc
600
     tcctgacaat cgataggtac ctggctgtcg tccatgctgt gtttgcttta aaagccagga
660
     cggtcacctt tggggtggtg acaagtgtga tcacttgggt ggtggctgtg tttgcgtctc
720
     tcccaggaat catctttacc agatctcaaa aagaaggtct tcattacacc tgcagctctc
780
     attttccata cagtcagtat caattctgga agaatttcca gacattaaag atagtcatct
840
     tggggctggt cctgccgctg cttgtcatgg tcatctgcta ctcgggaatc ctaaaaactc
900
     tgcttcggtg tcgaaatgag aagaagaggc acagggctgt gaggcttatc ttcaccatca
960
     tgattgttta ttttctcttc tgggctccct acaacattgt ccttctcctg aacaccttcc
1020
     aggaattctt tggcctgaat aattgcagta gctctaacag gttggaccaa gctatgcagg
1080
     tgacagagac tcttgggatg acgcactgct gcatcaaccc catcatctat gcctttgtcg
1140
     gggagaagtt cagaaactac ctcttagtct tcttccaaaa gcacattgcc aaacgcttct
1200
     gcaaatgctg ttctattttc cagcaagagg ctcccgagcg agcaagctca gtttacaccc
1260
```

gatccactgg ggagcaggaa atatctgtgg gcttgtgaca cggactcaag tgggctggtg
1320
acccagtcag agttgtgcac atggcttagt tttcatacac agcctgggct gggggtnggt
1380
tggnngaggt ctttttttaaa aggaagttac tgttatagag ggtctaagat tcatccattt
1440
atttggcatc tgtttaaagt agattagatc cgaattc
1477

SQ SEQUENCE 352 AA; 40524 MW; 7E61FEA5 CRC32;
MDYQVSSPIY DINYYTSEPC QKINVKQIAA RLLPPLYSLV FIFGFVGNML VILILINCKR
LKSMTDIYLL NLAISDLFFL LTVPFWAHYA AAQWDFGNTM CQLLTGLYFI GFFSGIFFII
LLTIDRYLAV VHAVFALKAR TVTFGVVTSV ITWVVAVFAS LPGIIFTRSQ KEGLHYTCSS
HFPYSQYQFW KNFQTLKIVI LGLVLPLLVM VICYSGILKT LLRCRNEKKR HRAVRLIFTI
MIVYFLFWAP YNIVLLLNTF QEFFGLNNCS SSNRLDQAMQ VTETLGMTHC CINPIIYAFV
GEKFRNYLLV FFQKHIAKRF CKCCSIFQQE APERASSVYT RSTGEQEISV GL

**gp120**

Gp120 Sequences, HIV-1 BaL **strain**

Nucleotide

atggatgcaatgaagagagggctctgctgtgtgctgctgctgtgtggagcagtcttcgtttcggctagcgtagaaaa
tttgtgggtcacagtttattatggggtacctgtgtggaaagaagcaaccaccactctattttgtgcatcagatgcta
aagcatatgatacagaggtacataatgtttgggccacacatgcctgtgtacccacagaccccaacccacaagaagta
gaattggaaaatgtgacagaaaattttaacatgtggaaaaataacatggtagaacagatgcatgaggatataatcag
tttatgggatcaaagcctaaagccatgtgtaaaattaactccactctgtgttactttaaattgcactgatttgagga
atgctactaatgggaatgacactaataccactagtagtagcagggaaatgatgggggaggagaaatgaaaaattgc
tctttcaaaatcaccacaaacataagaggtaaggtgcagaaagaatatgcactttttatgaacttgatatagtacc
aatagataataatagtaataatagatataggttgataagttgtaacacctcagtcattacacaggcctgtccaaaga
tatcctttgagccaattcccatacattattgtgccccggctggttttgcgattctaaagtgtaaagataagaagttc
aatggaaaaggaccatgttcaaatgtcagcacagtacaatgtacacatgggattaggccagtagtatcaactcaact
gctgttaaatggcagtctagcagaagaagaggtagtaattagatccgaaaatttcgcggacaatgctaaaaccataa
tagtacagctgaatgaatctgtagaaattaattgtacaagacccaacaacaatacaagaaaaagtatacatatagga
ccaggcagagcattatatacaacaggagaaataataggagatataagacaagcacattgtaaccttagtagagcaaa
atggaatgacactttaaataagatagttataaaattaagagaacaatttgggaataaaacaatagtctttaagcatt
cctcaggaggggacccagaaattgtgacgcacagttttaattgtggaggggaatttttctactgtaattcaacacaa
ctgtttaatagtacttggaatgttactgaagagtcaaataacactgtagaaaataacacaatcacactcccatgcag
aataaaacaaattataaacatgtggcagaaagtaggaagagcaatgtatgcccctcccatcagaggacaaattagat
gttcatcaaatattacagggctgctattaacaagagatggtggtcccgaggccaacaagaccgaggtcttcagacct
ggaggaggagatatgagggacaattggagaagtgaattatataaatataaagtagtaaaaattgaaccattaggagt
agcacccaccaaggcaaagagaagagtggtgcagagagaaaaaagataa

Amino-acid

MDAMKRGLCCVLLLCGAVFVSASVENLWVTVYYGVPVWKEATTTLFCASDAKAYDTEVHNVWATHACVPTDPNPQEV
ELENVTENFNMWKNNMVEQMHEDIISLWDQSLKPCVKLTPLCVTLNCTDLRNATNGNDTNTTSSSREMMGGGEMKNC
SFKITTNIRGKVQKEYALFYELDIVPIDNNSNNRYRLISCNTSVITQACPKISFEPIPIHYCAPAGFAILKCKDKKF
NGKGPCSNVSTVQCTHGIRPVVSTQLLLNGSLAEEEVVIRSENFADNAKTIIVQLNESVEINCTRPNNNTRKSIHIG
PGRALYTTGEIIGDIRQAHCNLSRAKWNDTLNKIVIKLREQFGNKTIVFKHSSGGDPEIVTHSFNCGGEFFYCNSTQ
LFNSTWNVTEESNNTVENNTITLPCRIKQIINMWQKVGRAMYAPPIRGQIRCSSNITGLLLTRDGGPEANKTEVFRP
GGGDMRDNWRSELYKYKVVKIEPLGVAPTKAKRRVVQREKR*

CD4

```
ID   N90764 standard; cDNA; 1113 BP.
KW   Human soluble CD4 protein; T4 SEC1 cDNA; T4ex1; HIV gp120 envelope
protein; T-lymphocyte.
OS   Homo sapiens.
FH   Key               Location/Qualifiers
FT   CDS               1..1113
FT                     /*tag=  a
FT   misc_difference   385..387
FT                     /*tag=  b
FT   misc_difference   487..489
FT                     /*tag=  c
FT   misc_difference   547..549
FT                     /*tag=  d
FT   misc_difference   727..729
FT                     /*tag=  e
FT   misc_difference   1108..1110
FT                     /*tag=  f
Sequence 1113 BP; 302 A; 276 C; 314 G; 221 T; 0 other;

          aagaaagtgg tgctgggcaa aaaaggggat acagtggaac tgacctgtac agcttcccag
60
          aagaagagca tacaattcca ctggaaaaac tccaaccaga taaagattct gggaaatcag
120
          ggctccttct taactaaagg tccatccaag ctgaatgatc gcgctgactc aagaagaagc
180
          ctttgggacc aaggaaactt cccccctgatc atcaagaatc ttaagataga agactcagat
240
          acttacatct gtgaagtgga ggaccagaag gaggaggtgc aattgctagt gttcggattg
300
          actgccaact ctgacaccca cctgcttcag gggcagagcc tgaccctgac cttggagagc
360
          ccccctggta gtagcccctc agtgcaatgt aggagtccaa ggggtaaaaa catacagggg
420
          gggaagaccc tctccgtgtc tcagctggag ctccaggata gtggcacctg gacatgcact
480
          gtcttgcaga accagaagaa ggtggagttc aaaatagaca tcgtggtgct agctttccag
540
          aaggcctcca gcatagtcta taagaaagag ggggaacagg tggagttctc cttcccactc
600
          gcctttacag ttgaaaagct gacgggcagt ggcgagctgt ggtggcaggc ggagagggct
660
          tcctcctcca agtcttggat cacctttgac ctgaagaaca aggaagtgtc tgtaaaacgg
720
          gttacccagg accctaagct ccagatgggc aagaagctcc cgctccacct caccctgccc
780
          caggccttgc ctcagtatgc tggctctgga aacctcaccc tggcccttga agcgaaaaca
840
          ggaaagttgc atcaggaagt gaacctggtg gtgatgagag ccactcagct ccagaaaaat
900
          ttgacctgtg aggtgtgggg acccacctcc cctaagctga tgctgagctt gaaactggag
960
          aacaaggagg caaaggtctc gaagcgggag aaggcggtgt gggtgctgaa ccctgaggcg
1020
          gggatgtggc agtgtctgct gagtgactcg ggacaggtcc tgctggaatc caacatcaag
1080
          gttctgccca catggtccac ccggttcat taa
1113
```

```
ID    P93528 standard; protein; 370 AA.
FH    Key              Location/Qualifiers
FT    Misc-difference 129
FT    Misc-difference 183
FT    Misc-difference 163
FT    Misc-difference 243
FT    Misc-difference 370
SQ    Sequence   370 AA;
SQ    16 A;  9 R;  16 N;  13 D;  0 B;  6 C;  27 Q;  24 E;  0 Z;  23 G;  5 H;
SQ    13 I;  48 L;  39 K;  4 M;  10 F;  16 P;  35 S;  25 T;  10 W;  3 Y;  28
V;
SQ    0 Others;
      kkvvlgkkgd tveltctasq kksiqfhwkn snqikilgnq gsfltkgpsk lndradsrrs
      lwdqgnfpli iknlkiedsd tyicevedqk eevqllvfgl tansdthllq gqsltltles
      ppgsspsvqc rsprgkniqg gktlsvsqle lqdsgtwtct vlqnqkkvef kidivvlafq
      kassivykke geqvefsfpl aftvekltgs gelwwqaera ssskswitfd lknkevsvkr
      vtqdpklqmg kklplhltlp qalpqyagsg nltlaleakt gklhqevnlv vmratqlqkn
      ltcevwgpts pklmlslkle nkeakvskre kavwvlnpea gmwqcllsds gqvllesnik
      vlptwstpvh
```

SEQUENCE LISTING

<110> Pfizer Limited
Pfizer Inc.

<120> Assay Method

<130> PCS10348A

<140>
<141>

<150> GB 0000661.9
<151> 2000-01-12

<150> GB 0000663.5
<151> 2000-01-12

<150> GB 0000659.3
<151> 2000-01-12

<160> 6

<170> PatentIn Ver. 2.1

<210> 1
<211> 1477
<212> DNA
<213> Homo sapiens

<220>
<221> misc_feature
<222> 1377, 1384, 1385
<223> n is a or g or c or t/u

<400> 1
gaattccccc aacagagcca agctctccat ctagtggaca gggaagctag cagcaaacct 60

```
tcccttcact acaaaacttc attgcttggc caaaaagaga gttaattcaa tgtagacatc 120
tatgtaggca attaaaaacc tattgatgta taaaacagtt tgcattcatg gagggcaact 180
aaatacattc taggacttta taaaagatca ctttttattt atgcacaggg tggaacaaga 240
tggattatca agtgtcaagt ccaatctatg acatcaatta ttatacatcg gagccctgcc 300
aaaaaatcaa tgtgaagcaa atcgcagccc gcctcctgcc tccgctctac tcactggtgt 360
tcatctttgg ttttgtgggc aacatgctgg tcatcctcat cctgataaac tgcaaaaggc 420
tgaagagcat gactgacatc tacctgctca acctggccat ctctgacctg tttttccttc 480
ttactgtccc cttctgggct cactatgctg ccgcccagtg ggactttgga aatacaatgt 540
gtcaactctt gacagggctc tattttatag gcttcttctc tggaatcttc ttcatcatcc 600
tcctgacaat cgataggtac ctggctgtcg tccatgctgt gtttgcttta aaagccagga 660
cggtcacctt tggggtggtg acaagtgtga tcacttgggt ggtggctgtg tttgcgtctc 720
tcccaggaat catctttacc agatctcaaa aagaaggtct tcattacacc tgcagctctc 780
attttccata cagtcagtat caattctgga agaatttcca gacattaaag atagtcatct 840
tggggctggt cctgccgctg cttgtcatgg tcatctgcta ctcgggaatc ctaaaaactc 900
tgcttcggtg tcgaaatgag aagaagaggc acagggctgt gaggcttatc ttcaccatca 960
tgattgttta ttttctcttc tgggctccct acaacattgt ccttctcctg aacaccttcc 1020
aggaattctt tggcctgaat aattgcagta gctctaacag gttggaccaa gctatgcagg 1080
tgacagagac tcttgggatg acgcactgct gcatcaaccc catcatctat gcctttgtcg 1140
gggagaagtt cagaaactac ctcttagtct tcttccaaaa gcacattgcc aaacgcttct 1200
gcaaatgctg ttctattttc cagcaagagg ctcccgagcg agcaagctca gtttacaccc 1260
gatccactgg ggagcaggaa atatctgtgg gcttgtgaca cggactcaag tgggctggtg 1320
acccagtcag agttgtgcac atggcttagt tttcatacac agcctgggct gggggtnggt 1380
tggnngaggt ctttttaaa aggaagttac tgttatagag ggtctaagat tcatccattt 1440
atttggcatc tgtttaaagt agattagatc cgaattc                         1477
```

<210> 2

<211> 352

<212> PRT

<213> Homo sapiens

<400> 2

```
Met Asp Tyr Gln Val Ser Ser Pro Ile Tyr Asp Ile Asn Tyr Tyr Thr
 1               5                  10                  15

Ser Glu Pro Cys Gln Lys Ile Asn Val Lys Gln Ile Ala Ala Arg Leu
            20                  25                  30
```

Leu Pro Pro Leu Tyr Ser Leu Val Phe Ile Phe Gly Phe Val Gly Asn
35 40 45

Met Leu Val Ile Leu Ile Leu Ile Asn Cys Lys Arg Leu Lys Ser Met
50 55 60

Thr Asp Ile Tyr Leu Leu Asn Leu Ala Ile Ser Asp Leu Phe Phe Leu
65 70 75 80

Leu Thr Val Pro Phe Trp Ala His Tyr Ala Ala Ala Gln Trp Asp Phe
85 90 95

Gly Asn Thr Met Cys Gln Leu Leu Thr Gly Leu Tyr Phe Ile Gly Phe
100 105 110

Phe Ser Gly Ile Phe Phe Ile Ile Leu Leu Thr Ile Asp Arg Tyr Leu
115 120 125

Ala Val Val His Ala Val Phe Ala Leu Lys Ala Arg Thr Val Thr Phe
130 135 140

Gly Val Val Thr Ser Val Ile Thr Trp Val Val Ala Val Phe Ala Ser
145 150 155 160

Leu Pro Gly Ile Ile Phe Thr Arg Ser Gln Lys Glu Gly Leu His Tyr
165 170 175

Thr Cys Ser Ser His Phe Pro Tyr Ser Gln Tyr Gln Phe Trp Lys Asn
180 185 190

Phe Gln Thr Leu Lys Ile Val Ile Leu Gly Leu Val Leu Pro Leu Leu
195 200 205

Val Met Val Ile Cys Tyr Ser Gly Ile Leu Lys Thr Leu Leu Arg Cys
210 215 220

Arg Asn Glu Lys Lys Arg His Arg Ala Val Arg Leu Ile Phe Thr Ile
225 230 235 240

Met Ile Val Tyr Phe Leu Phe Trp Ala Pro Tyr Asn Ile Val Leu Leu
245 250 255

Leu Asn Thr Phe Gln Glu Phe Phe Gly Leu Asn Asn Cys Ser Ser Ser
260 265 270

Asn Arg Leu Asp Gln Ala Met Gln Val Thr Glu Thr Leu Gly Met Thr
275 280 285

His Cys Cys Ile Asn Pro Ile Ile Tyr Ala Phe Val Gly Glu Lys Phe
290 295 300

Arg Asn Tyr Leu Leu Val Phe Phe Gln Lys His Ile Ala Lys Arg Phe
305 310 315 320

Cys Lys Cys Cys Ser Ile Phe Gln Gln Glu Ala Pro Glu Arg Ala Ser
325 330 335

Ser Val Tyr Thr Arg Ser Thr Gly Glu Gln Glu Ile Ser Val Gly Leu
340 345 350

<210> 3
<211> 1512
<212> DNA
<213> Human immunodeficiency virus type 1

<400> 3
atggatgcaa tgaagagagg gctctgctgt gtgctgctgc tgtgtggagc agtcttcgtt 60
tcggctagcg tagaaaattt gtgggtcaca gtttattatg gggtacctgt gtggaaagaa 120
gcaaccacca ctctattttg tgcatcagat gctaaagcat atgatacaga ggtacataat 180
gtttgggcca cacatgcctg tgtacccaca gaccccaacc cacaagaagt agaattggaa 240
aatgtgacag aaaattttaa catgtggaaa aataacatgg tagaacagat gcatgaggat 300
ataatcagtt tatgggatca aagcctaaag ccatgtgtaa aattaactcc actctgtgtt 360
actttaaatt gcactgattt gaggaatgct actaatggga atgacactaa taccactagt 420
agtagcaggg aaatgatggg gggaggagaa atgaaaaatt gctctttcaa aatcaccaca 480
aacataagag gtaaggtgca gaaagaatat gcactttttt atgaacttga tatagtacca 540
atagataata atagtaataa tagatatagg ttgataagtt gtaacacctc agtcattaca 600

caggcctgtc caaagatatc ctttgagcca attcccatac attattgtgc cccggctggt 660
tttgcgattc taaagtgtaa agataagaag ttcaatggaa aaggaccatg ttcaaatgtc 720
agcacagtac aatgtacaca tgggattagg ccagtagtat caactcaact gctgttaaat 780
ggcagtctag cagaagaaga ggtagtaatt agatccgaaa atttcgcgga caatgctaaa 840
accataatag tacagctgaa tgaatctgta gaaattaatt gtacaagacc caacaacaat 900
acaagaaaaa gtatacatat aggaccaggc agagcattat atacaacagg agaaataata 960
ggagatataa gacaagcaca ttgtaacctt agtagagcaa aatggaatga cactttaaat 1020
aagatagtta taaaattaag agaacaattt gggaataaaa caatagtctt taagcattcc 1080
tcaggagggg acccagaaat tgtgacgcac agttttaatt gtggagggga atttttctac 1140
tgtaattcaa cacaactgtt taatagtact tggaatgtta ctgaagagtc aaataacact 1200
gtagaaaata acacaatcac actcccatgc agaataaaac aaattataaa catgtggcag 1260
aaagtaggaa gagcaatgta tgcccctccc atcagaggac aaattagatg ttcatcaaat 1320
attacagggc tgctattaac aagagatggt ggtcccgagg ccaacaagac cgaggtcttc 1380
agacctggag gaggagatat gagggacaat tggagaagtg aattatataa atataaagta 1440
gtaaaaattg aaccattagg agtagcaccc accaaggcaa agagaagagt ggtgcagaga 1500
gaaaaaagat aa 1512

<210> 4
<211> 503
<212> PRT
<213> Human immunodeficiency virus type 1

<400> 4
Met Asp Ala Met Lys Arg Gly Leu Cys Cys Val Leu Leu Leu Cys Gly
1 5 10 15

Ala Val Phe Val Ser Ala Ser Val Glu Asn Leu Trp Val Thr Val Tyr
20 25 30

Tyr Gly Val Pro Val Trp Lys Glu Ala Thr Thr Thr Leu Phe Cys Ala
35 40 45

Ser Asp Ala Lys Ala Tyr Asp Thr Glu Val His Asn Val Trp Ala Thr
50 55 60

His Ala Cys Val Pro Thr Asp Pro Asn Pro Gln Glu Val Glu Leu Glu
65 70 75 80

Asn Val Thr Glu Asn Phe Asn Met Trp Lys Asn Asn Met Val Glu Gln
85 90 95

Met His Glu Asp Ile Ile Ser Leu Trp Asp Gln Ser Leu Lys Pro Cys
100 105 110

Val Lys Leu Thr Pro Leu Cys Val Thr Leu Asn Cys Thr Asp Leu Arg
115 120 125

Asn Ala Thr Asn Gly Asn Asp Thr Asn Thr Thr Ser Ser Ser Arg Glu
130 135 140

Met Met Gly Gly Gly Glu Met Lys Asn Cys Ser Phe Lys Ile Thr Thr
145 150 155 160

Asn Ile Arg Gly Lys Val Gln Lys Glu Tyr Ala Leu Phe Tyr Glu Leu
165 170 175

Asp Ile Val Pro Ile Asp Asn Asn Ser Asn Asn Arg Tyr Arg Leu Ile
180 185 190

Ser Cys Asn Thr Ser Val Ile Thr Gln Ala Cys Pro Lys Ile Ser Phe
195 200 205

Glu Pro Ile Pro Ile His Tyr Cys Ala Pro Ala Gly Phe Ala Ile Leu
210 215 220

Lys Cys Lys Asp Lys Lys Phe Asn Gly Lys Gly Pro Cys Ser Asn Val
225 230 235 240

Ser Thr Val Gln Cys Thr His Gly Ile Arg Pro Val Val Ser Thr Gln
245 250 255

Leu Leu Leu Asn Gly Ser Leu Ala Glu Glu Glu Val Val Ile Arg Ser
260 265 270

Glu Asn Phe Ala Asp Asn Ala Lys Thr Ile Ile Val Gln Leu Asn Glu
275 280 285

Ser Val Glu Ile Asn Cys Thr Arg Pro Asn Asn Asn Thr Arg Lys Ser
290 295 300

Ile His Ile Gly Pro Gly Arg Ala Leu Tyr Thr Thr Gly Glu Ile Ile
305 310 315 320

Gly Asp Ile Arg Gln Ala His Cys Asn Leu Ser Arg Ala Lys Trp Asn
325 330 335

Asp Thr Leu Asn Lys Ile Val Ile Lys Leu Arg Glu Gln Phe Gly Asn
340 345 350

Lys Thr Ile Val Phe Lys His Ser Ser Gly Gly Asp Pro Glu Ile Val
355 360 365

Thr His Ser Phe Asn Cys Gly Gly Glu Phe Phe Tyr Cys Asn Ser Thr
370 375 380

Gln Leu Phe Asn Ser Thr Trp Asn Val Thr Glu Glu Ser Asn Asn Thr
385 390 395 400

Val Glu Asn Asn Thr Ile Thr Leu Pro Cys Arg Ile Lys Gln Ile Ile
405 410 415

Asn Met Trp Gln Lys Val Gly Arg Ala Met Tyr Ala Pro Pro Ile Arg
420 425 430

Gly Gln Ile Arg Cys Ser Ser Asn Ile Thr Gly Leu Leu Leu Thr Arg
435 440 445

Asp Gly Gly Pro Glu Ala Asn Lys Thr Glu Val Phe Arg Pro Gly Gly
450 455 460

Gly Asp Met Arg Asp Asn Trp Arg Ser Glu Leu Tyr Lys Tyr Lys Val
465 470 475 480

Val Lys Ile Glu Pro Leu Gly Val Ala Pro Thr Lys Ala Lys Arg Arg
485 490 495

Val Val Gln Arg Glu Lys Arg
500

<210> 5
<211> 1113
<212> DNA
<213> Homo sapiens

<400> 5
aagaaagtgg tgctgggcaa aaaaggggat acagtggaac tgacctgtac agcttcccag 60
aagaagagca tacaattcca ctggaaaaac tccaaccaga taaagattct gggaaatcag 120
ggctccttct taactaaagg tccatccaag ctgaatgatc gcgctgactc aagaagaagc 180
ctttgggacc aaggaaactt ccccctgatc atcaagaatc ttaagataga agactcagat 240
acttacatct gtgaagtgga ggaccagaag gaggaggtgc aattgctagt gttcggattg 300
actgccaact ctgacaccca cctgcttcag ggcagagcc tgaccctgac cttggagagc 360
ccccctggta gtagcccctc agtgcaatgt aggagtccaa ggggtaaaaa catacagggg 420
gggaagaccc tctccgtgtc tcagctggag ctccaggata gtggcacctg gacatgcact 480
gtcttgcaga accagaagaa ggtggagttc aaaatagaca tcgtggtgct agctttccag 540
aaggcctcca gcatagtcta taagaaagag gggaacagg tggagttctc cttcccactc 600
gcctttacag ttgaaaagct gacgggcagt ggcgagctgt ggtggcaggc ggagagggct 660
tcctcctcca agtcttggat cacctttgac ctgaagaaca aggaagtgtc tgtaaaacgg 720
gttacccagg accctaagct ccagatgggc aagaagctcc cgctccacct caccctgccc 780
caggccttgc ctcagtatgc tggctctgga aacctcaccc tggcccttga agcgaaaaca 840
ggaaagttgc atcaggaagt gaacctggtg gtgatgagag ccactcagct ccagaaaaat 900
ttgacctgtg aggtgtgggg acccacctcc cctaagctga tgctgagctt gaaactggag 960
aacaaggagg caaaggtctc gaagcgggag aaggcggtgt gggtgctgaa ccctgaggcg 1020
gggatgtggc agtgtctgct gagtgactcg ggacaggtcc tgctggaatc caacatcaag 1080
gttctgccca catggtccac cccggttcat taa 1113

<210> 6
<211> 370
<212> PRT
<213> Homo sapiens

<400> 6
Lys Lys Val Val Leu Gly Lys Lys Gly Asp Thr Val Glu Leu Thr Cys
1 5 10 . 15

Thr Ala Ser Gln Lys Lys Ser Ile Gln Phe His Trp Lys Asn Ser Asn
20 25 30

Gln Ile Lys Ile Leu Gly Asn Gln Gly Ser Phe Leu Thr Lys Gly Pro
35 40 45

Ser Lys Leu Asn Asp Arg Ala Asp Ser Arg Arg Ser Leu Trp Asp Gln
50 55 60

Gly Asn Phe Pro Leu Ile Ile Lys Asn Leu Lys Ile Glu Asp Ser Asp
65 70 75 80

Thr Tyr Ile Cys Glu Val Glu Asp Gln Lys Glu Glu Val Gln Leu Leu
85 90 95

Val Phe Gly Leu Thr Ala Asn Ser Asp Thr His Leu Leu Gln Gly Gln
100 105 110

Ser Leu Thr Leu Thr Leu Glu Ser Pro Pro Gly Ser Ser Pro Ser Val
115 120 125

Gln Cys Arg Ser Pro Arg Gly Lys Asn Ile Gln Gly Gly Lys Thr Leu
130 135 140

Ser Val Ser Gln Leu Glu Leu Gln Asp Ser Gly Thr Trp Thr Cys Thr
145 150 155 160

Val Leu Gln Asn Gln Lys Lys Val Glu Phe Lys Ile Asp Ile Val Val
165 170 175

Leu Ala Phe Gln Lys Ala Ser Ser Ile Val Tyr Lys Lys Glu Gly Glu
180 185 190

Gln Val Glu Phe Ser Phe Pro Leu Ala Phe Thr Val Glu Lys Leu Thr
195 200 205

Gly Ser Gly Glu Leu Trp Trp Gln Ala Glu Arg Ala Ser Ser Ser Lys
210 215 220

Ser Trp Ile Thr Phe Asp Leu Lys Asn Lys Glu Val Ser Val Lys Arg
225 230 235 240

Val Thr Gln Asp Pro Lys Leu Gln Met Gly Lys Lys Leu Pro Leu His
245 250 255

Leu Thr Leu Pro Gln Ala Leu Pro Gln Tyr Ala Gly Ser Gly Asn Leu
260 265 270

Thr Leu Ala Leu Glu Ala Lys Thr Gly Lys Leu His Gln Glu Val Asn
275 280 285

Leu Val Val Met Arg Ala Thr Gln Leu Gln Lys Asn Leu Thr Cys Glu
290 295 300

Val Trp Gly Pro Thr Ser Pro Lys Leu Met Leu Ser Leu Lys Leu Glu
305 310 315 320

Asn Lys Glu Ala Lys Val Ser Lys Arg Glu Lys Ala Val Trp Val Leu
325 330 335

Asn Pro Glu Ala Gly Met Trp Gln Cys Leu Leu Ser Asp Ser Gly Gln
340 345 350

Val Leu Leu Glu Ser Asn Ile Lys Val Leu Pro Thr Trp Ser Thr Pro
355 360 365

Val His
370

## SEQUENCE LISTINGS (PART OF THE DESCRIPTION)

CCR5

```
ID   T90117 standard; cDNA; 1477 BP.
DE   cDNA for human CCR5.
KW   Human Cys-Cys chemokine receptor 5; CCR5; human immunodeficiency virus;
type 1; type 2; HIV-1; HIV-2; diagnosis; treatment; prevention; inflammatory
disease; rheumatoid arthritis; glomerulonephritis; asthma; idiopathic
pulmonary fibrosis; psoriasis; viral infection; cancer; atherosclerosis;
autoimmune disorder; ss.
OS   Homo sapiens.
FH   Key             Location/Qualifiers
FT   CDS             240..1298

SQ   Sequence 1477 BP; 374 A; 349 C; 320 G; 431 T; 3 other

     gaattccccc aacagagcca agctctccat ctagtggaca gggaagctag cagcaaacct
60
     tcccttcact acaaaacttc attgcttggc caaaaagaga gttaattcaa tgtagacatc
120
     tatgtaggca attaaaaacc tattgatgta taaaacagtt tgcattcatg gagggcaact
180
     aaatacattc taggacttta taaaagatca ctttttattt atgcacaggg tggaacaaga
240
     tggattatca agtgtcaagt ccaatctatg acatcaatta ttatacatcg gagccctgcc
300
     aaaaaatcaa tgtgaagcaa atcgcagccc gcctcctgcc tccgctctac tcactggtgt
360
     tcatctttgg ttttgtgggc aacatgctgg tcatcctcat cctgataaac tgcaaaaggc
420
     tgaagagcat gactgacatc tacctgctca acctggccat ctctgacctg tttttccttc
480
     ttactgtccc cttctgggct cactatgctg ccgcccagtg ggactttgga aatacaatgt
540
     gtcaactctt gacagggctc tattttatag gcttcttctc tggaatcttc ttcatcatcc
600
     tcctgacaat cgataggtac ctggctgtcg tccatgctgt gtttgcttta aaagccagga
660
     cggtcacctt tggggtggtg acaagtgtga tcacttgggt ggtggctgtg tttgcgtctc
720
     tcccaggaat catctttacc agatctcaaa aagaaggtct tcattacacc tgcagctctc
780
     attttccata cagtcagtat caattctgga agaatttcca gacattaaag atagtcatct
840
     tggggctggt cctgccgctg cttgtcatgg tcatctgcta ctcgggaatc ctaaaaactc
900
     tgcttcggtg tcgaaatgag aagaagaggc acagggctgt gaggcttatc ttcaccatca
960
     tgattgttta ttttctcttc tgggctccct acaacattgt ccttctcctg aacaccttcc
1020
     aggaattctt tggcctgaat aattgcagta gctctaacag gttggaccaa gctatgcagg
1080
     tgacagagac tcttgggatg acgcactgct gcatcaaccc catcatctat gcctttgtcg
1140
     gggagaagtt cagaaactac ctcttagtct tcttccaaaa gcacattgcc aaacgcttct
1200
     gcaaatgctg ttctattttc cagcaagagg ctcccgagcg agcaagctca gtttacaccc
1260
```

```
     gatccactgg ggagcaggaa atatctgtgg gcttgtgaca cggactcaag tgggctggtg
1320
     acccagtcag agttgtgcac atggcttagt tttcatacac agcctgggct gggggtnggt
1380
     tggnngaggt ctttttttaaa aggaagttac tgttatagag ggtctaagat tcatccattt
1440
     atttggcatc tgtttaaagt agattagatc cgaattc
1477
```

```
SQ   SEQUENCE   352 AA;  40524 MW;  7E61FEA5 CRC32;
     MDYQVSSPIY DINYYTSEPC QKINVKQIAA RLLPPLYSLV FIFGFVGNML VILILINCKR
     LKSMTDIYLL NLAISDLFFL LTVPFWAHYA AAQWDFGNTM CQLLTGLYFI GFFSGIFFII
     LLTIDRYLAV VHAVFALKAR TVTFGVVTSV ITWVVAVFAS LPGIIFTRSQ KEGLHYTCSS
     HFPYSQYQFW KNFQTLKIVI LGLVLPLLVM VICYSGILKT LLRCRNEKKR HRAVRLIFTI
     MIVYFLFWAP YNIVLLLNTF QEFFGLNNCS SSNRLDQAMQ VTETLGMTHC CINPIIYAFV
     GEKFRNYLLV FFQKHIAKRF CKCCSIFQQE APERASSVYT RSTGEQEISV GL
```

**gp120**

Gp120 Sequences, HIV-1 BaL **strain**

Nucleotide

```
atggatgcaatgaagagagggctctgctgtgtgctgctgctgtgtggagcagtcttcgtttcggctagcgtagaaaa
tttgtgggtcacagtttattatggggtacctgtgtggaaagaagcaaccaccactctattttgtgcatcagatgcta
aagcatatgatacagaggtacataatgtttgggccacacatgcctgtgtacccacagaccccaacccacaagaagta
gaattggaaaatgtgacagaaaattttaacatgtggaaaaataacatggtagaacagatgcatgaggatataatcag
tttatgggatcaaagcctaaagccatgtgtaaaattaactccactctgtgttactttaaattgcactgatttgagga
atgctactaatgggaatgacactaataccactagtagtagcagggaaatgatgggggaggagaaatgaaaaattgc
tctttcaaaatcaccacaaacataagaggtaaggtgcagaaagaatatgcactttttttatgaacttgatatagtacc
aatagataataatagtaataatagatataggttgataagttgtaacacctcagtcattacacaggcctgtccaaaga
tatcctttgagccaattcccatacattattgccccggctggttttgcgattctaaagtgtaaagataagaagttc
aatggaaaaggaccatgttcaaatgtcagcacagtacaatgtacacatgggattaggccagtagtatcaactcaact
gctgttaaatggcagtctagcagaagaagaggtagtaattagatccgaaaatttcgcggacaatgctaaaaccataa
tagtacagctgaatgaatctgtagaaattaattgtacaagacccaacaacaatacaagaaaaagtatacatatagga
ccaggcagagcattatatacaacaggagaaataataggagatataagacaagcacattgtaaccttagtagagcaaa
atggaatgacactttaaataagatagttataaaattaagagaacaatttgggaataaaacaatagtctttaagcatt
cctcaggaggggacccagaaattgtgacgcacagttttaattgtggaggggaatttttctactgtaattcaacacaa
ctgtttaatagtacttggaatgttactgaagagtcaaataacactgtagaaaataacacaatcacactcccatgcag
aataaaacaaattataaacatgtggcagaaagtaggaagagcaatgtatgcccctcccatcagaggacaaattagat
gttcatcaaatattacagggctgctattaacaagagatggtggtcccgaggccaacaagaccgaggtcttcagacct
ggaggaggagatatgagggacaattggagaagtgaattatataaatataaagtagtaaaaattgaaccattaggagt
agcacccaccaaggcaaagagaagagtggtgcagagagaaaaaagataa
```

Amino-acid

```
MDAMKRGLCCVLLLCGAVFVSASVENLWVTVYYGVPVWKEATTTLFCASDAKAYDTEVHNVWATHACVPTDPNPQEV
ELENVTENFNMWKNNMVEQMHEDIISLWDQSLKPCVKLTPLCVTLNCTDLRNATNGNDTNTTSSSREMMGGGEMKNC
SFKITTNIRGKVQKEYALFYELDIVPIDNNSNNRYRLISCNTSVITQACPKISFEPIPIHYCAPAGFAILKCKDKKF
NGKGPCSNVSTVQCTHGIRPVVSTQLLLNGSLAEEEVVIRSENFADNAKTIIVQLNESVEINCTRPNNNTRKSIHIG
PGRALYTTGEIIGDIRQAHCNLSRAKWNDTLNKIVIKLREQFGNKTIVFKHSSGGDPEIVTHSFNCGGEFFYCNSTQ
LFNSTWNVTEESNNTVENNTITLPCRIKQIINMWQKVGRAMYAPPIRGQIRCSSNITGLLLTRDGGPEANKTEVFRP
GGGDMRDNWRSELYKYKVVKIEPLGVAPTKAKRRVVQREKR*
```

CD4

```
ID   N90764 standard; cDNA; 1113 BP.
KW   Human soluble CD4 protein; T4 SEC1 cDNA; T4ex1; HIV gp120 envelope
protein; T-lymphocyte.
OS   Homo sapiens.
FH   Key              Location/Qualifiers
FT   CDS              1..1113
FT                    /*tag=  a
FT   misc_difference 385..387
FT                    /*tag=  b
FT   misc_difference 487..489
FT                    /*tag=  c
FT   misc_difference 547..549
FT                    /*tag=  d
FT   misc_difference 727..729
FT                    /*tag=  e
FT   misc_difference 1108..1110
FT                    /*tag=  f
Sequence 1113 BP; 302 A; 276 C; 314 G; 221 T; 0 other;

      aagaaagtgg tgctgggcaa aaaaggggat acagtggaac tgacctgtac agcttcccag
60
      aagaagagca tacaattcca ctggaaaaac tccaaccaga taaagattct gggaaatcag
120
      ggctccttct taactaaagg tccatccaag ctgaatgatc gcgctgactc aagaagaagc
180
      ctttgggacc aaggaaactt cccccctgatc atcaagaatc ttaagataga agactcagat
240
      acttacatct gtgaagtgga ggaccagaag gaggaggtgc aattgctagt gttcggattg
300
      actgccaact ctgacaccca cctgcttcag gggcagagcc tgaccctgac cttggagagc
360
      ccccctggta gtagcccctc agtgcaatgt aggagtccaa ggggtaaaaa catacagggg
420
      gggaagaccc tctccgtgtc tcagctggag ctccaggata gtggcacctg gacatgcact
480
      gtcttgcaga accagaagaa ggtggagttc aaaatagaca tcgtggtgct agctttccag
540
      aaggcctcca gcatagtcta taagaaagag ggggaacagg tggagttctc cttcccactc
600
      gcctttacag ttgaaaagct gacgggcagt ggcgagctgt ggtggcaggc ggagagggct
660
      tcctcctcca agtcttggat cacctttgac ctgaagaaca aggaagtgtc tgtaaaacgg
720
      gttacccagg accctaagct ccagatgggc aagaagctcc cgctccacct caccctgccc
780
      caggccttgc ctcagtatgc tggctctgga aacctcaccc tggcccttga agcgaaaaca
840
      ggaaagttgc atcaggaagt gaacctggtg gtgatgagag ccactcagct ccagaaaaat
900
      ttgacctgtg aggtgtgggg acccacctcc cctaagctga tgctgagctt gaaactggag
960
      aacaaggagg caaaggtctc gaagcgggag aaggcggtgt gggtgctgaa ccctgaggcg
1020
      gggatgtggc agtgtctgct gagtgactcg ggacaggtcc tgctggaatc caacatcaag
1080
      gttctgccca catggtccac cccggttcat taa
1113
```

```
ID   P93528 standard; protein; 370 AA.
FH   Key             Location/Qualifiers
FT   Misc-difference 129
FT   Misc-difference 183
FT   Misc-difference 163
FT   Misc-difference 243
FT   Misc-difference 370
SQ   Sequence   370 AA;
SQ   16 A;  9 R;  16 N;  13 D;  0 B;  6 C;  27 Q;  24 E;  0 Z;  23 G;  5 H;
SQ   13 I;  48 L;  39 K;  4 M;  10 F;  16 P;  35 S;  25 T;  10 W;  3 Y;  28
V;
SQ   0 Others;
     kkvvlgkkgd tveltctasq kksiqfhwkn snqikilgnq gsfltkgpsk lndradsrrs
     lwdqgnfpli iknlkiedsd tyicevedqk eevqllvfgl tansdthllq gqsltltles
     ppgsspsvqc rsprgkniqg gktlsvsqle lqdsgtwtct vlqnqkkvef kidivvlafq
     kassivykke geqvefsfpl aftvekltgs gelwwqaera sssksswitfd lknkevsvkr
     vtqdpklqmg kklplhltlp qalpqyagsg nltlaleakt gklhqevnlv vmratqlqkn
     ltcevwgpts pklmlslkle nkeakvskre kavwvlnpea gmwqcllsds gqvllesnik
     vlptwstpvh
```

## Claims

1. An assay method for determining whether an agent is capable of modulating the interaction of CCR5 with gp120; the method comprising:

   incubating the agent with CCR5 and gp120 to form a first reaction mixture; and
   determining whether said agent modulates the interaction of CCR5 with gp120;

      wherein said gp120 is associated with CD4; and
      wherein said interaction is a low affinity binding.

2. A method according to claim 1 wherein said method includes the step of adding a ligand to said first reaction mixture to form a second reaction mixture; wherein said ligand is capable of indicating whether said agent has modulated said interaction.

3. A method according to claim 2 wherein said ligand has a detectable label.

4. A method according to claim 3 wherein said detectable label is a fluorescent atom or a fluorescent group.

5. A method according to claim 5 wherein said radioactive atom is $Eu^{3+}$.

6. A method according to any one of claims 2 to 5 wherein said ligand comprises at least a first antibody.

7. A method according to claim 6 wherein said first antibody is capable of binding to gp120; and wherein said binding is high affinity binding, preferably wherein said first antibody is associated with a detectable label.

8. A method according to claim 6 or claim 7 wherein said ligand comprises at least a second antibody.

9. A method according to claim 7 wherein said second antibody is capable of binding to said first antibody.

10. A method according to claim 9 wherein said second antibody is an anti-IgG antibody.

11. A method according to any one of claims 8 to 10 when dependent on claim 3 wherein said detectable label is associated with said second antibody.

12. An agent identified by the method according to any one of claims 1 to 11, wherein said agent is capable of modulating the interaction of CCR5 with gp120.

**13.** A process comprising the steps of:

(a) performing the assay according to any one of claims 1 to 11;

(b) identifying one or more agents that are capable of modulating the interaction of CCR5 with gp120; and

(c) preparing a quantity of those one or more identified agents.

**14.** A process comprising the steps of:

(a) performing the assay according to any one of claims 1 to 11;

(b) identifying one or more agents that are capable of modulating the interaction of CCR5 with gp120; and

(c) admixing one or more of said agent(s) with a pharmaceutically acceptable carrier, diluent or excipient to form a pharmaceutical composition.

**15.** A process according to claim 14 wherein said process also includes the subsequent step of:
(d) administering said composition to a subject in need of same.

**16.** A method of affecting the *in vivo* interaction of CCR5 with gp120 with an agent;

wherein the agent is capable of modulating the interaction of CCR5 with gp120 in an *in vitro* assay method;

wherein the *in vitro* assay method is the assay method defined in any one of claims 1 to 11.

**17.** Use of an agent in the preparation of a pharmaceutical composition for the treatment of a disease or condition associated with the interaction of CCR5 with gp120, wherein the agent is the agent of claim 12 and/or wherein the agent is capable of modulating the interaction of CCR5 with gp120 when assayed *in vitro* by the assay method according to any one of claims 1 to 11.

**18.** A method of treating a subject with an agent, wherein the agent is the agent of claim 12 and/or wherein the agent is capable of modulating the interaction of CCR5 with gp120 when assayed *in vitro* by the assay method according to any one of claims 1 to 11.

**19.** A process for preparing a pharmaceutical for use in treating HIV; the process comprising forming a composition by admixing (a) the agent of claim 12 and/or an agent that is capable of modulating the interaction of CCR5 with gp120 when assayed *in vitro* by the assay method according to any one of claims 1 to 11 with (b) a pharmaceutically acceptable carrier, diluent or excipient.

**20.** A pack comprising at least two compartments; wherein first of said compartments houses the agent of claim 12 and/or an agent that is capable of modulating the interaction of CCR5 with gp120 when assayed *in vitro* by the assay method according to any one of claims 1 to 11; and wherein second of said compartments houses an additional pharmaceutically useful moiety.